(19) Europäisches Patentamt / European Patent Office / Office européen des brevets

(11) EP 4 660 189 A1

(12) **EUROPEAN PATENT APPLICATION**
published in accordance with Art. 153(4) EPC

(43) Date of publication:
**10.12.2025 Bulletin 2025/50**

(21) Application number: **24750253.7**

(22) Date of filing: **30.01.2024**

(51) International Patent Classification (IPC):
***C07D 211/46*** (2006.01) ***A61K 9/51*** (2006.01)
***A61K 9/127*** (2025.01) ***A61K 31/7088*** (2006.01)
***A61K 47/22*** (2006.01) ***A61K 47/44*** (2017.01)
***A61K 48/00*** (2006.01) ***A61P 21/00*** (2006.01)
***A61P 25/00*** (2006.01) ***A61P 43/00*** (2006.01)
***C07D 207/12*** (2006.01) ***C07D 211/34*** (2006.01)
***C07D 243/08*** (2006.01) ***C07D 453/00*** (2006.01)
***C07D 453/02*** (2006.01) ***C07D 471/10*** (2006.01)
***C12N 15/12*** (2006.01) ***C12N 15/88*** (2006.01)

(52) Cooperative Patent Classification (CPC):
**A61K 9/127; A61K 9/51; A61K 31/7088; A61K 47/22; A61K 47/44; A61K 48/00; A61P 21/00; A61P 25/00; A61P 43/00; C07D 207/12; C07D 211/34; C07D 211/46; C07D 243/08; C07D 453/00; C07D 453/02;** (Cont.)

(86) International application number:
**PCT/JP2024/002768**

(87) International publication number:
**WO 2024/162304 (08.08.2024 Gazette 2024/32)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **31.01.2023 JP 2023013548**
**29.06.2023 JP 2023107539**
**21.08.2023 LB 1288023**

(71) Applicant: **Astellas Pharma Inc.**
**Tokyo 103-8411 (JP)**

(72) Inventors:
- **NIGAWARA Takahiro**
  **Tokyo 103-8411 (JP)**
- **TOMINAGA Hiroaki**
  **Tokyo 103-8411 (JP)**
- **HAMAJIMA Toshihiro**
  **Tokyo 103-8411 (JP)**
- **KANAI Akira**
  **Tokyo 103-8411 (JP)**
- **WASHIO Takuya**
  **Tokyo 103-8411 (JP)**
- **OBA Yasunori**
  **Tokyo 103-8411 (JP)**
- **FUMIYAMA Hitoshi**
  **Tokyo 103-8411 (JP)**
- **IWASAKI Fumiyoshi**
  **Tokyo 103-8411 (JP)**
- **KATO Tetsuro**
  **Tokyo 103-8411 (JP)**
- **YAMADA Hiroyoshi**
  **Tokyo 103-8411 (JP)**
- **MATSUEDA Katsuki**
  **Tokyo 103-8411 (JP)**

(74) Representative: **Hoffmann Eitle**
**Patent- und Rechtsanwälte PartmbB**
**Arabellastraße 30**
**81925 München (DE)**

Remarks:
The complete document including Reference Table(s) and the Sequence Listing(s) can be downloaded from the EPO website

# (54) CARBAMOYL LIPID OR UREA LIPID EACH HAVING CYCLIC AMINE, LIPID NANOPARTICLES CONTAINING SAME, AND PHARMACEUTICAL COMPOSITION

(57) The present invention addresses the problem of creating a carbamoyl lipid or a urea lipid each having a

cyclic amino and developing lipid nanoparticles, and thereby providing a pharmaceutical composition for nucleic acid therapeutics and others. The present inventors have discovered a compound that is a carbamoyl lipid or a urea lipid each having a cyclic amino or a salt of the compound, and have studied on lipid nanoparticles that can be used in various pharmaceutical compositions. As a result, it was revealed that lipid nanoparticles can be formed using the compound of the present invention, which is a lipid, or a salt of the compound. By using the lipid nanoparticles containing the carbamoyl lipid or the urea lipid each having a cyclic amino according to the present invention, it is expected that a pharmaceutical composition containing the lipid nanoparticles each encapsulating a nucleic acid therein can be used as a prophylactic or therapeutic agent for astrocyte-related diseases.

(52) Cooperative Patent Classification (CPC): (Cont.)
**C07D 471/10; C07K 14/435; C12N 15/88**

## Description

### Technical Field

The present invention relates to a carbamoyl lipid or a urea lipid having cyclic amine which is useful as a component of lipid nanoparticles (also called a compound or a salt thereof below), lipid nanoparticles containing the same, a pharmaceutical composition containing lipid nanoparticles encapsulating a nucleic acid which is useful for treatment/-prevention therein (also called nucleic acid lipid nanoparticles below) and the like.

### Background Art

Nucleic acids are recently used or are about to be used for treating or preventing diseases. For example, because proteins are synthesized in the body based on messenger RNA (mRNA), mRNA has drawn attention as a pharmaceutical product which is used for the purpose of expressing a desired protein in target cells. It is, however, difficult to deliver a nucleic acid itself such as mRNA alone into cells, and thus lipid nanoparticles (LNPs) are used as a delivery technique for delivering a nucleic acid such as mRNA into target cells.

As LNPs, those having properties of being more easily introduced into cells as well as properties of protecting a nucleic acid such as mRNA from decomposition in the serum are desired. Moreover, as LNPs, those having properties of being easily introduced into appropriate cells through systemic or local administration are required. Accordingly, as part of optimization of LNPs which encapsulate a nucleic acid such as mRNA and which can be introduced into appropriate cells, cationic lipids which are one of the components thereof are studied.

As examples of application of nucleic acid lipid nanoparticles, COVID-19 vaccines and cancer vaccines have been put on the market or clinically developed. In the nucleic acid lipid nanoparticles, various lipids are used as a cationic lipid, which is one of the components (Patent Literatures 1 to 3).

Patent Literature 1 discloses that a cationic lipid is used for lipid nanoparticles. The lipid below is described in the claims.

[Chem. 1]

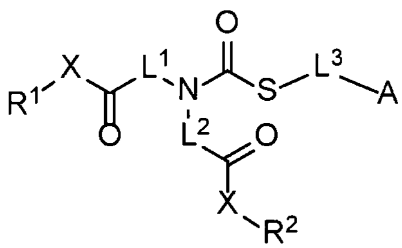

(see the literature for the symbols in the formula)

Patent Literature 2 discloses that the lipid is used for lipid nanoparticles.

Patent Literature 3 discloses that, for example, the lipid below is used for lipid nanoparticles.

Astrocytes are one type of cells which are the most abundant in the central nervous system, play an important role in the homeostasis of blood-brain barrier and synapse formation of neurons in normal environment and have an important role of supporting the normal brain function (Frontiers in Cellular Neuroscience, 2022, vol. 16, 850866 and Toxicologic Pathology, 2011, vol. 39(1), 115-123).

In the case of brain damage, however, astrocytes become activated, accumulate in the damaged site and form gliosis. Gliosis is believed to be a factor inhibiting neuronal regeneration, and a wide range of astrocyte-related diseases such as spinal cord injury, neurodegenerative diseases including Alzheimer's disease and epilepsy have been reported (Neuron, 2014, vol. 81, P229-248). Accordingly, when an effective drug which is introduced into astrocytes and acts can be developed, a new therapeutic method for a wide range of central nervous system diseases may be provided.

### Citation List

### Patent Literature

[Patent Literature 1] WO2022/150485A
[Patent Literature 2] WO2022/136641A
[Patent Literature 3] WO2020/246581A

Summary of Invention

Problems to be Solved by the Invention

An object of the invention is to provide a lipid compound or a salt thereof which is a component of lipid nanoparticles that can encapsulate a nucleic acid (an oligonucleotide, mRNA or the like) and that are introduced into target cells.

Another object of the invention is to provide nucleic acid lipid nanoparticles containing a nucleic acid which is useful for treating or preventing various diseases, such as mRNA, and a pharmaceutical composition containing the same.

Means for Solving the Problems

As a result of intensive examination, the present inventors have found the compound of the formula (I) of the invention or a salt thereof, which is a carbamoyl lipid or a urea lipid having cyclic amine such as piperidine and diazepane, and found that novel and useful lipid nanoparticles can be produced using the same as a cationic lipid. The invention can provide such a cationic lipid, lipid nanoparticles, nucleic acid lipid nanoparticles, a pharmaceutical composition containing nucleic acid lipid nanoparticles and the like.

That is, the invention relates to [1] to [26] below.

[1] A compound of a formula (I) or a salt thereof:

[Chem. 2]

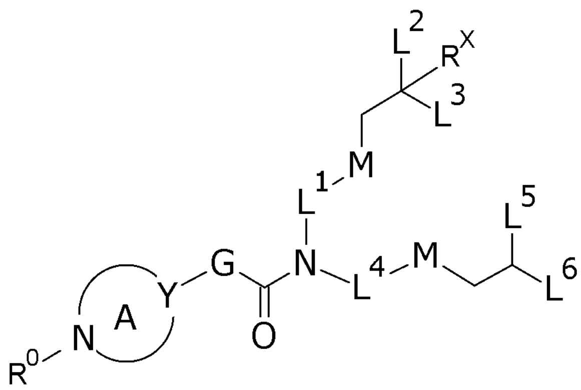

(I)

(in the formula,

$L^1$ is a $C_{5-10}$ alkylene, $CH_2$ or $-C_{3-6}$ cycloalkanediyl-$(CH_2)_p$-,
wherein p is 0 or 1,
$L^2$ is H or a $C_{5-15}$ alkyl,
wherein $L^2$ is H when $L^1$ is $CH_2$,
$L^3$ is a $C_{5-15}$ alkyl,
$R^X$ is H or adamantyl formed together with $L^2$, $L^3$ and the carbon atom to which they are bonded,
$L^4$ is a $C_{5-10}$ alkylene,
$L^5$ and $L^6$ are the same or different and are a $C_{5-15}$ alkyl,
M's are each independently C(=O)O or OC(=O),
the ring A is a group selected from the group consisting of

i) a 5- to 7-membered ring group composed of three to five carbon atoms, one N and one Y,

wherein one of the hydrocarbon chains between N and Y is $CH_2CH_2$, and the other is $(CH_2)_n$,

wherein n is an integer of 1, 2 or 3,

ii) a diazaspirononane ring group, and
iii) a quinuclidine ring group,

$R^0$ is ($C_{1-6}$ alkyl)$_2$N-$C_{1-6}$ alkylene-C(=O)-, (cyclic amino)-$C_{1-6}$ alkylene-C(=O)- or a $C_{1-6}$ alkyl,
wherein $R^0$ does not exist when the ring A is a quinuclidine ring group, and
the combination of G and Y (G, Y) is (O, CH), ($CH_2$, CH) or (a bond, N),
wherein Y is N when the ring A is a diazaspirononane ring group, and Y is CH when the ring A is a quinuclidine ring group.)

[2] The compound or the salt thereof described in [1], in which the combination of G and Y (G, Y) is (O, CH) or ($CH_2$, CH).
[3] The compound or the salt thereof described in [2], in which the combination of G and Y (G, Y) is (O, CH).
[4] The compound or the salt thereof described in [3], in which M is C(=O)O.
[5] The compound or the salt thereof described in [4], in which the ring A is a 6-membered ring composed of four carbon atoms, one N and one Y, one of the hydrocarbon chains between N and Y is $CH_2CH_2$, the other is $(CH_2)_n$, and n is 2.
[5a] The compound of a formula (I) or a salt thereof,

in which the ring A is a group selected from the group consisting of a formula (a), a formula (b) or a formula (c),

[Chem. 3]

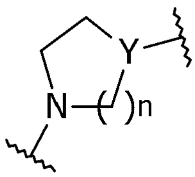

(a)

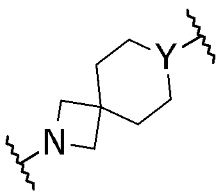

(b)

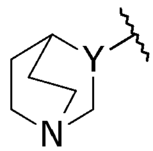

(c)

in which n is an integer of 1, 2 or 3, in which Y is N or CH in the formula (a), Y is N in the formula (b), and Y is CH in the formula (c).

[6] The compound or the salt thereof described in [5], in which $L^1$ is a $C_{6-9}$ alkylene, $L^2$ is a $C_{6-14}$ alkyl, $L^3$ is a $C_{6-14}$ alkyl, $L^4$ is a $C_{6-9}$ alkylene, $L^5$ is a $C_{6-14}$ alkyl, and $L^6$ is a $C_{6-14}$ alkyl.
[7] The compound or the salt thereof described in [6], in which $R^0$ is $(CH_3)_2N-CH_2-C(=O)-$.
[8] The compound or the salt thereof described in [1], in which the combination of G and Y (G, Y) is (a bond, N).
[9] The compound or the salt thereof described in [8], in which M is C(=O)O.
[10] The compound or the salt thereof described in [9], in which the ring A is a 7-membered ring composed of five carbon atoms, one N and one Y, one of the hydrocarbon chains between N and Y is $CH_2CH_2$, the other is $(CH_2)_n$, and n is 3.
[11] The compound or the salt thereof described in [10], in which $L^1$ is -$C_{3-6}$ cycloalkanediyl-$(CH_2)_p$-.
[12] The compound or the salt thereof described in [10], in which $L^1$ is a $C_{6-9}$ alkylene, $L^2$ is a $C_{6-14}$ alkyl, $L^3$ is a $C_{6-14}$ alkyl, $R^x$ is H, $L^4$ is a $C_{6-9}$ alkylene, $L^5$ is a $C_{6-14}$ alkyl, and $L^6$ is a $C_{6-14}$ alkyl.
[13] The compound or the salt thereof described in [11] or [12], in which $R^0$ is $CH_3$ or $C_2H_5$.
[14] The compound or the salt thereof described in [1], in which the compound is selected from the group consisting of

bis(2-nonylundecyl) 7,7'-[({[1-(N,N-dimethylglycyl)piperidin-4-yl]oxy}carbonyl)azanediyl]di(heptanoate),
bis(2-undecyltridecyl) 7,7-[({[1-(N,N-dimethylglycyl)piperidin-4-yl]oxy}carbonyl)azanediyl]di(heptanoate),
bis(2-decyltetradecyl) 7,7'-[({[1-(N,N-dimethylglycyl)piperidin-4-yl]oxy}carbonyl)azanediyl]di(heptanoate),
2-octyldecyl 8-[({[1-(N,N-dimethylglycyl)piperidin-4-yl]oxy} carbonyl) {7-oxo-7-[(2-undecyltridecyl)oxy]heptyl} amino]octanoate,
2-nonylundecyl 8-{[(1r,3r)-3-{2-[(2-decyldodecyl)oxy]-2-oxoethyl}cyclobutyl]({[1-(N,N-dimethylglycyl)piperidin-4-yl]oxy}carbonyl)amino}octanoate and
2-nonylundecyl 8-{({[1-(N,N-dimethylglycyl)piperidin-4-yl]oxy}carbonyl)[(1r,3r)-3-{2-oxo-2-[(2-undecyltridecyl)oxy]ethyl}cyclobutyl]amino}octanoate.

[15] The compound or the salt thereof described in [1], in which the compound is selected from the group consisting of

2-nonylundecyl 8-{(4-methyl-1,4-diazepane-1-carbonyl)[(1r,3r)-3-{2-[(2-octyldecyl)oxy]-2-oxoethyl}cyclobutyl]amino}octanoate,
bis(2-octyldodecyl) 9,9'-[(4-methyl-1,4-diazepane-1-carbonyl)azanediyl]di(nonanoate),
2-nonylundecyl 8-{(4-ethyl-1,4-diazepane-1-carbonyl)[(1r,3r)-3-{2-[(2-octyldecyl)oxy]-2-oxoethyl}cyclobutyl]amino}octanoate,
2-nonylundecyl 8-{(4-methyl-1,4-diazepane-1-carbonyl)[(1r,4r)-4-{2-oxo-2-[(2-undecyltridecyl)oxy]ethyl}cyclohexyl]amino}octanoate and
2-heptylnonyl (1r,4r)-4-[{8-[(2-heptylnonyl)oxy]-8-oxooctyl}(4-methyl-1,4-diazepane-1-carbonyl)amino]cyclohexane-1-carboxylate.

[16] Lipid nanoparticles containing the compound or the salt thereof described in any of [1] to [15].
[17] Lipid nanoparticles containing the compound or the salt thereof described in any of [1] to [15], a neutral lipid and a PEGylated lipid.
[18] The lipid nanoparticles described in [17], in which the neutral lipid is a phospholipid and a sterol, and the phospholipid is DSPC, DOPE, DOPC, DPPC, DHSM, SOPC or DoPhPE.
[19] The lipid nanoparticles described in [17] or [18], in which the neutral lipid is a phospholipid and a sterol, and the sterol is cholesterol or β-sitosterol.
[20] The lipid nanoparticles described in any of [17] to [19], in which the PEGylated lipid is DMG-PEG2000, C8 PEG2000 ceramide or polyethylene glycol monostearate.
[21] The lipid nanoparticles described in any of [17] to [19] which encapsulate a nucleic acid.
[22] The lipid nanoparticles described in [21], in which the nucleic acid is mRNA.
[23] The lipid nanoparticles described in [21] or [22] containing 40.0 to 60.0 mol% of the compound or the salt thereof according to claim 1, 37.5 to 59.0 mol% of the neutral lipid and 1.0 to 2.5 mol% of the PEGylated lipid based on the total amount of the lipid nanoparticles.
[24] The lipid nanoparticles described in [21] or [22] containing 40.0 to 60.0 mol% of the compound or the salt thereof according to claim 1, 38.0 to 59.0 mol% of the neutral lipid and 1.0 to 2.0 mol% of the PEGylated lipid based on the total amount of the lipid nanoparticles.
[25] The lipid nanoparticles described in [22] which can express a protein in an astrocyte.
[26] The lipid nanoparticles described in [21], in which the nucleic acid is a nucleic acid which is useful for preventing and/or treating an astrocyte-related disease.
[27] The lipid nanoparticles described in [22] which encapsulate a nucleic acid,

in which the nucleic acid is mRNA encoding NeuroD1 protein, and
the lipid nanoparticles contain the compound of the formula (I) or the salt thereof, neutral lipids and a PEGylated lipid, wherein the compound of the formula (I) or the salt thereof is bis(2-nonylundecyl) 7,7'-[({[1-(N,N-dimethylglycyl)piperidin-4-yl]oxy} carbonyl)azanediyl]di(heptanoate) or a salt thereof, the neutral lipids are DSPC and cholesterol, and the PEGylated lipid is DMG-PEG2000.

[28] The lipid nanoparticles described in [16], [21], [22] or [27], in which the nucleic acid is mRNA encoding NeuroD1 protein containing a base sequence encoding a protein having the amino acid sequence of SEQ ID NO: 2.
[29] A pharmaceutical composition containing the lipid nanoparticles described in [21] to [28] and one or more pharmaceutically acceptable pharmaceutical additives.
[30] The pharmaceutical composition described in [29] for preventing and/or treating an astrocyte-related disease.

The invention also relates to:
a pharmaceutical composition for preventing and/or treating an astrocyte-related disease containing the lipid nanoparticles described in [16] to [23].
The pharmaceutical composition includes an agent for preventing and/or treating an astrocyte-related disease containing the lipid nanoparticles described in [16] to [23].
The invention also relates to:

use of the lipid nanoparticles described in [16] to [28] for producing a pharmaceutical composition for preventing and/or treating an astrocyte-related disease;
the lipid nanoparticles described in [16] to [28] for use in preventing and/or treating an astrocyte-related disease;

use of the lipid nanoparticles described in [16] to [28] for preventing and/or treating an astrocyte-related disease; and a method for preventing and/or treating an astrocyte-related disease consisting of administering an effective amount of the lipid nanoparticles described in [16] to [28] to a subject.

The invention also relates to a method for delivering a nucleic acid which is useful for preventing and/or treating an astrocyte-related disease into cells of the body, in particular astrocytes, using nucleic acid lipid nanoparticles containing the compound of the formula (I) or a salt thereof.

The "astrocyte-related disease" is cerebral infarction, cerebral hemorrhage, traumatic brain injury, spinal cord injury, a neurodegenerative disease, epilepsy, amyotrophic lateral sclerosis, Duchenne muscular dystrophy, Alexander disease, multiple sclerosis or neuromyelitis optica. The astrocyte-related disease is, in an aspect, cerebral hemorrhage, traumatic brain injury, spinal cord injury, a neurodegenerative disease, epilepsy, amyotrophic lateral sclerosis, Duchenne muscular dystrophy, Alexander disease, multiple sclerosis or neuromyelitis optica.

Here, cerebral infarction is penetrating branch infarction or cerebral infarction having brain damage in a penetrating branch territory in an aspect, subacute-phase cerebral infarction in an aspect, subacute-phase to chronic-phase cerebral infarction in an aspect, chronic-phase cerebral infarction in an aspect, cerebral infarction having motor dysfunction with high severity in an aspect, cerebral infarction with modified Rankin Scale (mRS) of 2 or more, or cerebral infarction with mRS of 4 or more in an aspect, cerebral infarction as a combination of them in an aspect, and penetrating branch infarction, Cerebral infarction having brain damage in a penetrating branch territory and subacute-phase to chronic-phase cerebral infarction and cerebral infarction having motor dysfunction with high severity in an aspect. The "acute phase" is a period from 72 hours to less than 1 month after the onset of cerebral infarction with the possibility of exacerbation of the symptoms. The "subacute phase" is a period from 72 hours to less than 1 month after the onset of cerebral infarction. The "subacute phase to chronic phase" is a period subsequent to the lapse of 72 hours after the onset of cerebral infarction. The "chronic phase" is a period subsequent to the lapse of 1 month after the onset of cerebral infarction.

The invention is not limited to the above aspects, and includes any aspect as an appropriate combination of the contents of Description of Embodiments in the specification.

This specification includes the disclosed contents of Japanese Patent Application Nos. 2023-013548 and 2023-107539, to which the present application claims priority.

## Advantageous Effects of Invention

The compound of the formula (I) or a salt thereof can form lipid nanoparticles. The lipid nanoparticles can encapsulate a nucleic acid or the like. Lipid nanoparticles encapsulating a nucleic acid are introduced into target cells and can express a protein from the nucleic acid in the cells or the like into which the lipid nanoparticles have been introduced into. Such lipid nanoparticles express the protein in the target cells related to a disease, and thus a pharmaceutical composition which is useful for preventing and/or treating the disease can be provided.

The compound of the formula (I) or a salt thereof can form lipid nanoparticles, and the lipid nanoparticles can deliver a nucleic acid to target cells or astrocytes in an aspect. With lipid nanoparticles, a nucleic acid which is useful for treating and/or preventing a disease can be introduced into target cells to express a protein, and as a result, in an aspect, a pharmaceutical composition which is useful for preventing or treating an astrocyte-related disease can be provided. That is, the pharmaceutical composition containing the nucleic acid lipid nanoparticles of the invention and one or more pharmaceutically acceptable pharmaceutical additives can be used as an agent for preventing and/or treating an astrocyte-related disease.

## Brief Description of Drawings

[Figure 1] Figure 1 shows the results of evaluation of the changes in motor dysfunction with days after endothelin-1 treatment (induction of cerebral ischemia) of the ND1 nucleic acid lipid nanoparticle (ND1 mRNA) group of Test Example 4 and the control group (Control mRNA) using modified Rankin Scale (mRS). The horizontal axis shows the period after the endothelin-1 infusion (days), and the vertical axis shows the mRS values (maximum value of 6). The white circles show the averages of the mRS of the ND1 nucleic acid lipid nanoparticle group at the points of evaluation. Here, the mRS was evaluated every two weeks on and after day 28 after the endothelin-1 infusion. The black circles show the averages of the mRS of the control group at the points of evaluation. As the results on day 70, the averages of the results evaluated on day 69 or 70 are shown. The nucleic acid lipid nanoparticles were administered on day 21 after the endothelin-1 infusion.

## Mode for Carrying Out the Invention

The invention will be explained in detail below.

In this specification, the terms below have the meanings below unless otherwise specifically noted. The definitions below are for clarifying the defined terms but do not limit the terms. When the terms used here are not specifically defined, the terms are used with the meanings which are generally accepted by one skilled in the art. Unless otherwise specifically noted, when a symbol in a chemical formula in this specification is also used in another chemical formula, the same symbol has the same meaning.

The "alkyl" is a linear or branched saturated hydrocarbon group. For example, the $C_{5-15}$ alkyl means an alkyl having 5 to 15 carbon atoms. In an aspect, the alkyl is a $C_{5-10}$ alkyl. In an aspect, the alkyl is a $C_{6-14}$ alkyl. In an aspect, the alkyl is a $C_{6-12}$ alkyl. In an aspect, the alkyl is a $C_{8-14}$ alkyl. In an aspect, the alkyl is a $C_{7-13}$ alkyl. In an aspect, the alkyl is a $C_{8-9}$ alkyl. In an aspect, the alkyl is a $C_{8-12}$ alkyl. In an aspect, the alkyl is a $C_8$ alkyl. In an aspect, the alkyl is a $C_9$ alkyl. In an aspect, a $C_{1-6}$ alkyl.

The "alkylene" is a linear or branched divalent saturated hydrocarbon group. For example, the $C_{1-15}$ alkylene means an alkylene having 1 to 15 carbon atoms. In an aspect, the alkylene is a $C_{5-10}$ alkylene. In an aspect, the alkylene is a $C_{6-9}$ alkylene. In an aspect, the alkylene is a $C_{6-8}$ alkylene. In an aspect, the alkylene is a $C_{6-7}$ alkylene. In an aspect, the alkylene is a $C_{7-9}$ alkylene. In an aspect, the alkylene is a $C_6$ alkylene. In an aspect, the alkylene is a $C_7$ alkylene. In an aspect, the alkylene is a $CH_2$. In an aspect, a $C_{1-6}$ alkylene.

The "hydrocarbon chain" is a divalent group in which methylene or methylene groups are linked. In an aspect, the hydrocarbon chain is $CH_2CH_2$, and in an aspect, the hydrocarbon chain is $(CH_2)_n$ in which n is an integer of 1, 2 or 3.

The "halogen" is F, Cl, Br or I.

The "cycloalkane" is a saturated hydrocarbon ring. The "$C_{3-6}$ cycloalkane" is a cycloalkane having 3 to 6 carbon atoms, and examples thereof include cyclopropane, cyclobutane, cyclopentane and cyclohexane. The "$C_{3-6}$ cycloalkanediyl" is a divalent group of a $C_{3-6}$ cycloalkane. The $C_{3-6}$ cycloalkanediyl is, in an aspect, cyclopropanediyl, cyclobutanediyl, cyclopentanediyl or cyclohexanediyl. The $C_{3-6}$ cycloalkanediyl is, in an aspect, cyclopropane-1,2-diyl, cyclobutane-1,3-diyl, cyclopentane-1,3-diyl or cyclohexane-1,4-diyl. The $C_{3-6}$ cycloalkanediyl is, in an aspect, cyclobutane-1,3-diyl, cyclopentane-1,3-diyl or cyclohexane-1,4-diyl. The $C_{3-6}$ cycloalkanediyl is, in an aspect, cyclobutane-1,3-diyl or cyclohexane-1,4-diyl.

The "cyclic amino" is a saturated nitrogen-containing heterocyclic group composed of carbon atoms and one nitrogen atom or more and is a saturated ring group having a connector from the nitrogen atom. In an aspect, the cyclic amino is a 5- to 7-membered ring group composed of three to five carbon atoms, one N and one Y. In an aspect, the cyclic amino is diazepanyl, pyrrolidinyl or piperidyl each having a connector from the nitrogen atom. In an aspect, the cyclic amino is pyrrolidinyl or piperidyl each having a connector from the nitrogen atom. In an aspect, the cyclic amino is piperidyl having a connector from the nitrogen atom. In an aspect, the cyclic amino is pyrrolidinyl having a connector from the nitrogen atom. In an aspect, the cyclic amino is pyrrolidin-1-yl or piperidin-1-yl. In an aspect, the cyclic amino is pyrrolidin-1-yl, and in an aspect, the cyclic amino is piperidin-1-yl.

The "diazaspirononane" includes isomers such as diazaspiro[3.5]nonane and diazaspiro[4.4]nonane. In an aspect, the diazaspirononane is 2,7-diazaspiro[3.5]nonane. The "diazaspirononane ring group" in an aspect is diazaspiro[3.5]nonane-2,7-diyl. In an aspect, the group is 2,7-diazaspiro[3.5]nonane-2,7-diyl.

The "quinuclidine" includes isomers such as 1-azabicyclo[2.2.2]octane and 2-azabicyclo[2.2.2]octane (isoquinuclidine). The "quinuclidine" ring group in an aspect is quinuclidin-3-yl. In an aspect, the group is 1-azabicyclo[2.2.2]octan-3-yl.

The "lipid nanoparticles" are nanoparticles containing a lipid as a main component. In general, the lipid nanoparticles contain a cationic lipid, a neutral lipid and a PEGylated lipid. The "nucleic acid lipid nanoparticles" are lipid nanoparticles encapsulating a nucleic acid. The "nucleic acid lipid nanoparticles" in an aspect are lipid nanoparticles encapsulating a nucleic acid which is useful for preventing and/or treating an astrocyte-related disease or lipid nanoparticles encapsulating mRNA in an aspect.

The "particle size" is the particle size of lipid nanoparticles and is measured using a dispersion in still state with a particle size analyzer (Zetasizer Nano ZSP, manufactured by Malvern Panalytical) using the method of Dynamic Light Scattering (DLS) as a hydrodynamic diameter (Dh). The particle size is in accordance with ISO22412. The "particle size" of the lipid nanoparticles is calculated as a Z-average particle size. The particle size of the lipid nanoparticles is 10 nm to 1000 nm in an aspect, 30 nm to 500 nm in an aspect or 30 nm to 250 nm in an aspect. The particle size is 60 nm to 180 nm in an aspect. The particle size is 65 nm to 230 nm in an aspect.

The "cationic lipid" is a compound or a salt thereof which can carry a cation (positive electric charge) in the molecule in response to the pH and which has a linear or branched fatty chain. In an aspect, the cationic lipid is the compound of the formula (I) or a salt thereof.

"Neutral lipids" include "phospholipids" and/or "sterols". In an aspect, the neutral lipid is a phospholipid and a sterol. In an aspect, the neutral lipid is a phospholipid, and in an aspect, the neutral lipid is a sterol.

The "phospholipid" is a lipid having a phosphoric acid ester group. Examples thereof include 1,2-distearoyl-sn-glycero-3-phosphocholine (DSPC), 1,2-dilinoleoyl-sn-glycero-3-phosphocholine, 1,2-dimyristoyl-sn-glycero-phosphocholine (DMPC), 1,2-dioleoyl-sn-glycero-3-phosphocholine (DOPC), 1,2-dierucoyl-sn-glycero-3-phosphocholine

(DEPC), 1,2-dipalmitoyl-sn-glycero-3-phosphocholine (DPPC), 1,2-diundecanoyl-sn-glycero-phosphocholine (DUPC), 1-palmitoyl-2-oleoyl-sn-glycero-3-phosphocholine (POPC), 1-stearoyl-2-oleoyl-sn-glycero-3-phosphocholine (SOPC), 1,2-dioleoyl-sn-glycero-3-phospho-rac-(1-glycerol) sodium salt (DOPG), 1,2-dimyristoyl-sn-glycero-3-phospho-rac-(1-glycerol) sodium salt (DMPG), 1,2-dipalmitoyl-sn-glycero-3-phospho-rac-(1-glycerol) sodium salt (DPPG), 1,2-distearoyl-sn-glycero-3-phospho-rac-(1-glycerol) sodium salt (DSPG), 1,2-dioleoyl-sn-glycero-3-phosphoethanolamine (DOPE), 1,2-dimyristoyl-sn-glycero-3-phosphoethanolamine (DMPE), 1,2-dipalmitoyl-sn-glycero-3-phosphoethanolamine (DPPE), 1,2-distearoyl-sn-glycero-3-phosphoethanolamine (DSPE), 1-palmitoyl-2-oleoyl-sn-glycero-3-phosphoethanolamine (POPE), 1,2-dierucoyl-sn-glycero-3-phosphoethanolamine (DEPE), 1,2-dioleoyl-sn-glycero-3-phospho-L-serine (DOPS), 1,2-di-O-phytanyl-sn-glycero-3-phosphoethanolamine (DoPhPE), dihydrosphingomyelin (DHSM), sphingomyelin and the like. In an aspect, the phospholipid is DSPC, DOPE, DOPC, DPPC, DHSM, SOPC or DoPhPE. In an aspect, the phospholipid is DSPC. In an aspect, the phospholipid is DOPE.

The "sterol" is a steroid alcohol and is a compound having a hydroxy group on the A ring of a steroid skeleton. In an aspect, the sterol is cholesterol and a corticosteroid. In an aspect, the sterol is fecosterol, sitosterol, ergosterol, campesterol, stigmasterol, brassicasterol, tomatidine, tomatin, ursolic acid, $\alpha$-tocopherol, $\beta$-sitosterol, a mixture thereof or the like. Moreover, the sterol is a combination of two or more kinds. In an aspect, the sterol is cholesterol or $\beta$-sitosterol. In an aspect, the sterol is cholesterol.

The "PEGylated lipid" refers to a lipid having polyethylene glycol (PEG). The PEGylated lipid is a lipid modified with polyethylene glycol. The PEGylated lipid is PEG-modified phosphatidylethanolamine, PEG-modified phosphatidic acid, PEG-modified ceramide, PEG-modified dialkylamine, PEG-modified diacylglycerol, PEG-modified dialkylglycerol, PEG-modified monostearate, a mixture thereof or the like. For example, the PEGylated lipid is 1,2-dimyristoyl-rac-glycero-3-methoxy PEG (DMG-PEG2000, also referred to as DMG-PEG), 1,2-dipalmitoyl-rac-glycero-3-PEG (DPG-PEG), 1,2-distearoyl-rac-glycero-3-PEG (DSG-PEG), 1,2-dilauroyl-sn-glycero-3-phosphoethanolamine-PEG (DLPE-PEG), 1,2-dimyristoyl-sn-glycero-3-phosphoethanolamine-PEG (DMPE-PEG), 1,2-dipalmitoyl-sn-glycero-3-phosphocholine-PEG (DPPC-PEG), N-octanoyl-sphingosine-1-{succinyl[methoxy(polyethylene glycol)2000]} (= C8 PEG2000 ceramide), polyethylene glycol monostearate or 1,2-distearoyl-sn-glycero-3-phosphoethanolamine-PEG (DSPE-PEG). In an aspect, the PEGylated lipid is DMG-PEG2000. In an aspect, the PEGylated lipid is DMG-PEG2000, C8 PEG2000 ceramide or polyethylene glycol monostearate.

The "encapsulation efficiency" refers to the amount of the nucleic acid introduced into the lipid nanoparticles based on the total amount of the nucleic acid in the lipid nanoparticle dispersion. For example, when 98 mg of the nucleic acid of a total amount of 100 mg of the nucleic acid is introduced into the lipid nanoparticles, the encapsulation efficiency can be indicated as 98%. The "encapsulation", when used in this specification, means being completely or substantially contained in the inside or being contained. In nucleic acid lipid nanoparticles of mRNA or the like, the encapsulation efficiency of the nucleic acid is measured by the method described in detail below. The value of the encapsulation efficiency of the nucleic acid is 70% or more in an aspect, 80% or more in an aspect, 90% or more in an aspect or 93% or 95% or more in an aspect, and 96% or more in an aspect.

The "N/P ratio" is a value obtained by dividing the number of moles of the amino group of the cationic lipid in the nucleic acid lipid nanoparticles (N) by the number of moles of the phosphoric acid of RNA (P). In an aspect, the N/P ratio is 1 to 12, in an aspect, the N/P ratio is 3 to 9, and in an aspect, the N/P ratio is 6 to 12. In an aspect, the N/P ratio is 6 or 12, and in an aspect, the N/P ratio is 6.

"Nucleic acids" are deoxyribonucleic acid (DNA) and ribonucleic acid (RNA), and examples of the nucleic acid include messenger RNA (mRNA), microRNA (miRNA), small interfering RNA (siRNA), small hairpin RNA (shRNA), ribozymes and antisense oligonucleotides. In an aspect, the nucleic acid is a nucleic acid which is useful for treating and/or preventing an astrocyte-related disease. In an aspect, the nucleic acid is mRNA. In an aspect, the nucleic acid is mRNA which is useful for treating and/or preventing an astrocyte-related disease. In an aspect, the nucleic acid is siRNA. In an aspect, the mRNA is mRNA of Ascl1 (mRNA encoding Ascl1 protein is also called Ascl mRNA, and mRNA encoding xxx protein is also called xxx mRNA below), Dlx2 mRNA, NeuroD1 mRNA (also called mRNA encoding NeuroD1 protein below), Ngn2 mRNA, BDNF mRNA, NGF mRNA, NT-3 mRNA or the like. In an aspect, the nucleic acid is NeuroD1 mRNA. In an aspect, the mRNA is Ascl1 mRNA. In an aspect, the nucleic acid is NeuroD1 mRNA containing a base sequence encoding a protein having the amino acid sequence of SEQ ID NO: 2. In an aspect, the nucleic acid is NeuroD1 mRNA containing the base sequence of SEQ ID NO: 1. In an aspect, the nucleic acid is NeuroD1 mRNA containing the base sequence of SEQ ID NO: 3. In an aspect, the nucleic acid is NeuroD1 mRNA containing the base sequence of SEQ ID NO: 4. In an aspect, the nucleic acid is NeuroD1 mRNA containing the base sequence of SEQ ID NO: 5. In an aspect, the nucleic acid is mRNA encoding NeuroD1 protein containing a base sequence for cap addition. In an aspect, the nucleic acid is mRNA encoding NeuroD1 protein in which the 5' terminal sequence is a 5' terminal sequence suitable for the capping method using CleanCap (registered trademark) Reagent AG (TriLink BioTechnologies). In an aspect, the nucleic acid is mRNA encoding NeuroD1 protein in which the 5' terminal sequence is AGG. In an aspect, the nucleic acid is mRNA encoding NeuroD1 protein in which the 5' terminal sequence is position 1 to position 3 of SEQ ID NO: 4. In an aspect, two or more kinds of nucleic acid are contained in the lipid nanoparticles, and in an aspect, one kind of nucleic acid is contained in

the lipid nanoparticles. The lipid nanoparticles encapsulating the nucleic acid contain, based on the total weight of the lipid nanoparticles, the nucleic acid in an amount of 0.001 to 60 weight% in an aspect, 0.1 to 40 weight% in an aspect, 1.0 to 25 weight% in an aspect or contains 3.0 to 10 weight% of the nucleic acid in an aspect. The mRNA may be a natural nucleic acid or an artificial nucleic acid (for example, a nucleic acid containing natural nucleic acid bases and/or artificial nucleic acid bases).

The nucleic acid in this specification is sometimes described as a base sequence containing "T" as a typical DNA sequence, but for example, when a base sequence specified by a specific SEQ ID NO: represents a DNA sequence in RNA (for example, mRNA) containing the base sequence specified by the specific SEQ ID NO:, the base sequence is understood as an RNA sequence in which the "T" in the DNA sequence is replaced with "U". The bases "adenine (A)", "thymine (T)", "guanine (G)", "cytosine (C)" and "uracil (U)" composing a base sequence (for example, the base sequences of SEQ ID NOs: 1 and 3) and nucleosides and nucleotides containing the bases may be of a natural type or a modified type for each base, each nucleoside and each nucleotide and/or have another modification (methylation or the like), unless otherwise specified in the sequence listing.

The "mRNA" is a messenger ribonucleic acid, is translated into a desired protein and refers to RNA containing a base sequence encoding (at least one) polypeptide (for example, a polypeptide which exists in the nature, a polypeptide having insertion, deletion, substitution and/or addition of an amino acid to a polypeptide which exists in the nature or a polypeptide which does not exist in the nature) and a sequence which enables or promotes translation of mRNA. In an aspect, the mRNA is mRNA which is translated *in vivo* and can produce the encoded polypeptide. The mRNA can contain a 5' cap, a 5' untranslated region (an untranslated region is also called UTR below), a coding region (CDS), a 3' UTR and a poly(A) chain in the structure.

The "5' cap" is a modified structure found at the 5' terminal and is involved in stability of mature mRNA, initiation of translation and the like. A structure in which 7-methylguanosine (also called $m^7G$) and mRNA are linked at 5' and 5' through triphosphoric acid (also called ppp) is called Cap-0. A structure in which position 2' of the ribose in the first nucleoside of mRNA is methylated in addition to the Cap-0 structure is called Cap-1, and a structure in which position 2' of the riboses of the first and second nucleosides are methylated is called Cap-2. Cap-0, Cap-1 and Cap-2 are sometimes called $m^7$GpppNp, $m^7$GpppN$_1$mp and $m^7$GpppN$_1$mpN$_2$mp, respectively ($N_1$ and $N_2$ each represent a nucleoside, and m represents a 2'-O methyl group) (Nature Reviews Molecular Cell Biology 2014, vol.15(5), p313-326). In an aspect, the 5' cap is Cap-0, Cap-1 or Cap-2. In an aspect, the 5' cap is Cap-0. In an aspect, the 5' cap is Cap-1. In an aspect, the 5' cap is Cap-2.

The "UTRs" are untranslated regions and include a 5' UTR and a 3' UTR. The 5' UTR and the 3' UTR may be derived from a gene to be expressed or derived from a heterologous gene. The UTRs may be of a natural type or a modified type having a sequence of a natural-type UTR which has been changed by insertion, deletion, substitution and/or addition of one or some (for example, 2, 3, 4, 5 or 6 nucleotides) nucleotides. The "UTR derived from a gene" in this specification includes not only a natural-type UTR but also such a modified-type UTR. In a UTR, more than one UTR may be linked directly or through a spacer sequence. The start codon and the 5' UTR may contain a part of the Kozak sequence (for example, a sequence in the 5' side from the start codon in the Kozak sequence). In an aspect, the UTRs are globin gene-derived UTRs. In an aspect, the UTRs are $\alpha$-globin gene-derived UTRs. In an aspect, the UTRs are human $\alpha$-globin gene-derived UTRs. In an aspect, the UTRs are $\beta$-globin gene-derived UTRs. In an aspect, the UTRs are human $\beta$-globin gene-derived UTRs. In an aspect, the 5' UTR and the 3' UTR are human $\alpha$-globin gene-derived 5' UTR and 3' UTR.

The "CDS" is a coding sequence and means a DNA sequence region translated into a protein, and the codons may be optimized. The stop codon at the 3' terminal of the CDS may be any stop codon of TAA, TGA or TAG, and more than one stop codon may be used in succession (for example, TAATGATAG).

A "modified nucleotide" is a modified nucleotide. For example, the modified nucleotide is a nucleotide modified by methylation, atom exchange, saturation of a double bond, deamination, replacement of an oxygen atom or the like with a sulfur atom or the like. In an aspect, the modified nucleotide is a nucleotide in which the nucleic acid base is modified. In an aspect, the modified nucleotide is a nucleotide in which the ribose is modified. In an aspect, the modified nucleotide is a nucleotide in which the phosphate group is modified. In an aspect, the modified nucleotide is a nucleotide containing 1-methyladenosine, pseudouridine, N1-methylpseudouridine (also called 1-methylpseudouridine), dihydrouridine, 5-methoxycytidine, 5-methylcytidine, 7-methylguanosine, N6-methyladenosine, inosine or thiouridine. In an aspect, the modified nucleotide is a nucleotide containing 1-methyladenosine. In an aspect, the modified nucleotide is a nucleotide containing modified uridine. In an aspect, the modified nucleotide is a nucleotide containing pseudouridine or N1-methylpseudouridine. In an aspect, the modified nucleotide is a nucleotide containing pseudouridine. In an aspect, the modified nucleotide is a nucleotide containing N1-methylpseudouridine. In an aspect, the modified nucleotide is a nucleotide containing dihydrouridine. In an aspect, the modified nucleotide is a nucleotide containing inosine. In an aspect, the modified nucleotide is a nucleotide containing 4-thiouridine. Here, in this specification, the modified nucleotides with "a part or all" can include those in which some of the predetermined modified nucleotides are not substituted in the entire modified nucleotide sequence. The percentage of the residues substituted to modified nucleotides from the specific nucleotides in the entire sequence may be 1 to 100%, 1 to 90%, 1 to 80%, 1 to 70%, 1 to 60%, 1 to 50%, 1 to 40%, 1 to 30%, 1 to 20%, 1 to

10%, 1 to 5%, 75 to 99%, 50 to 75%, 25 to 50% or 10 to 25% but is not limited thereto. In an aspect, "a part" as the percentage of the residues substituted to modified nucleotides of all the residues may be, for example, 60 to 99%, 70 to 99%, 80 to 99%, 90 to 99%, 95 to 99%, 60 to 99.9%, 70 to 99.9%, 80 to 99.9%, 90 to 99.9% or 95 to 99.9% but is not limited thereto.

The "poly(A) chain" is a polyadenylic acid chain having the function of stabilization of mRNA, nuclear export, translation or the like. The poly(A) chain is also called a poly(A) sequence. In an aspect, the poly(A) chain length is 20 to 1000 bases. In an aspect, the poly(A) chain length is 30 to 500 bases, 50 to 200 bases, 60 to 150 bases, 70 to 130 bases, 70 to 120 bases, 70 to 80 bases, 120 bases or 79 bases. In an aspect, the poly(A) chain length is 30 to 500 bases (in an aspect, 40 to 200, 50 to 200, 50 to 150, 50 to 100, 50 to 90, 60 to 150, 60 to 100, 60 to 90, 70 to 130, 70 to 120, 70 to 100, 70 to 90, 70 to 85, 70 to 80, 75 to 130, 75 to 120, 75 to 100 or 75 to 90, in an aspect, 74 to 84, 75 to 85, 75 to 83 or 76 to 82 bases). In an aspect, the poly(A) chain length is 120 bases. In an aspect, the poly(A) chain length is 79 bases. In an aspect, the poly(A) chain length is 74 to 84 bases. In an aspect, the poly(A) chain length is 75 to 85 bases.

The "expression" means that a polypeptide or a protein is synthesized based on the nucleotide sequence and refers to, for example, translation of mRNA into a polypeptide or a protein or synthesis of a desired protein by posttranslational modification of a polypeptide/protein.

"NeuroD1" is a neurogenic differentiation protein (neuronal differentiation 1) and is a basic helix-loop-helix (bHLH) transcription factor belonging to the NeuroD family. NeuroD1 protein activates transcription of a gene containing a specific DNA sequence known as E-box (also called transcription factor activity). NeuroD1 has a central function of inducing differentiation from neural stem cells into neurons. Glial cells are classified into four cell types of astrocytes, oligodendrocytes, ependymal cells and microglia. Ectopic expression of NeuroD1 in glial cells such as NG2 cells and astrocytes is known to be able to convert glial cells into the nerve (WO2014/015261A). In an aspect, NeuroD1 is a protein consisting of an amino acid sequence having a sequence identity of 90% or more (for example, 95% or more, 98% or more, 99% or more, 99.4% or more or 99.5% or more) to the amino acid sequence of SEQ ID NO: 2 and having transcription factor activity. In an aspect, NeuroD1 is a protein consisting of an amino acid sequence having insertion, deletion, substitution and/or addition of 1 to 50 (for example, one or two, one to three, one to five, one to seven, 1 to 10 or 1 to 30) amino acids in the amino acid sequence of SEQ ID NO: 2 and having transcription factor activity. In an aspect, NeuroD1 is a protein having the amino acid sequence of SEQ ID NO: 2.

The "transcription factor activity" means the ability of a protein to regulate transcription of a related gene (namely, the ability of regulating transcription). Whether a protein has transcription factor activity or not can be determined specifically, for example, by the change (increase or decrease) in the expression level of the related gene by a recombinant vector compared to that in cells without introduction of the vector. The recombinant vector can be produced by inserting a nucleic acid encoding the protein into a vector having any promotor, and the protein can be expressed by introducing the recombinant vector into cells. For example, when the expression level of the related gene whose expression is known to be facilitated by NeuroD1 is increased in cells into which a vector including a nucleic acid encoding NeuroD1 has been introduced, it can be determined that NeuroD1 protein has transcription factor activity.

The "sequence identity" means the percentage (%) of the matching residues between sequences when a reference biological sequence (a base sequence, an amino acid sequence or the like) and a target biological sequence are aligned (generally, the percentage % of the matching residues based on the entire length of the target sequence). The "sequence identity" can be calculated, for example, as an identity value obtained using EMBOSS Needle (Nucleic Acids Res., 2015; Vol.43: pW580-W584) using the parameters provided as default. The parameters are as follows (Gap Open Penalty = 10, Gap Extend Penalty = 0.5, Matrix = EBLOSUM62, End Gap Penalty = false).

"Cerebral infarction" is also called ischemic stroke. A disease with brain damage caused by blockage or breakage of a brain vessel is stroke. Of these, a disease caused by blockage of a vessel supplying the brain is cerebral infarction.

"Penetrating branch infarction" is a disease in which infarction is caused in the thalamus, the caudate nucleus, the putamen, the globus pallidus and/or the internal capsule, which are controlled by the perforating branch, and in which the function at the infarction site is damaged. The "penetrating branch" are fine arteries branched from the middle cerebral artery constituting the major cerebral artery and supply the basal ganglia/thalamus area and the internal capsule. Here, that a brain area is "territory supplied" by a vessel means that blood is supplied to the brain area by the vessel, and the brain area is in the vascular territory of the vessel.

The "penetrating branch territory" means the area supplied by penetrating branch and refers to the basal ganglia/thalamus area and the internal capsule.

"Cerebral infarction having brain damage in a penetrating branch territory" means cerebral infarction in which brain damage caused by infarction is mainly caused in the penetrating branch territory. Cerebral infarction having brain damage in a penetrating branch territory includes not only cerebral infarction in which brain damage is caused in the penetrating branch territory due to infarction caused in the penetrating branch territory but also cerebral infarction in which brain damage is secondarily caused in the penetrating branch territory, for example, as in the case in which cell necrosis caused by infarction caused around the penetrating branch territory spreads to the penetrating branch territory to damage the function of the site. The brain damage in this specification means functional damage in the brain due to cell necrosis

caused by infarction. The brain damage generally has cell necrosis in the area having the brain damage. "Cerebral infarction having brain damage in a penetrating branch territory" may be cerebral infarction having cell necrosis in the penetrating branch territory.

Aspects of the compound of the formula (I), which is the compound of the invention, or a salt thereof are shown below. In this regard, all the aspects can be freely combined. Even when the combination is not specifically described, one aspect or two or more aspects can be combined with another aspect.

1. Cationic Lipid

Aspects of the compound of the formula (I), which is the compound of the invention, or a salt thereof are shown below.

(Aspects of Compound of Invention)

(1) The compound of the formula (I) or a salt thereof in which the combination of G and Y (G, Y) is (O, CH) or ($CH_2$, CH). In an aspect, the compound of the formula (I) or a salt thereof in which the combination of G and Y (G, Y) is (O, CH). The compound of the formula (I) or a salt thereof in which the combination of G and Y (G, Y) is ($CH_2$, CH). The compound of the formula (I) or a salt thereof in which the combination of G and Y (G, Y) is (a bond, N).

(2) The compound of the formula (I) or a salt thereof in which $L^1$ is a $C_{6-10}$ alkylene. The compound of the formula (I) or a salt thereof in which $L^1$ is a $C_{6-9}$ alkylene. The compound of the formula (I) or a salt thereof in which $L^1$ is a $C_{6-8}$ alkylene. The compound of the formula (I) or a salt thereof in which $L^1$ is a $C_{7-10}$ alkylene. The compound of the formula (I) or a salt thereof in which $L^1$ is a $C_{7-9}$ alkylene. The compound of the formula (I) or a salt thereof in which $L^1$ is a $C_6$ alkylene. The compound of the formula (I) or a salt thereof in which $L^1$ is $CH_2$. The compound of the formula (I) or a salt thereof in which $L^1$ is -$C_{3-6}$ cycloalkanediyl-$(CH_2)_p$-. The compound of the formula (I) or a salt thereof in which $L^1$ is -cyclobutanediyl-$(CH_2)_p$-. The compound of the formula (I) or a salt thereof in which $L^1$ is -$C_{3-6}$ cycloalkanediyl-$(CH_2)_p$-, where the $C_{3-6}$ cycloalkanediyl is cyclobutane-1,3-diyl, cyclopentane-1,3-diyl or cyclohexane-1,4-diyl. The compound of the formula (I) or a salt thereof in which $L^1$ is -$C_{3-6}$ cycloalkanediyl-$(CH_2)_p$-, where the $C_{3-6}$ cycloalkanediyl is cyclobutane-1,3-diyl or cyclohexane-1,4-diyl. The compound of the formula (I) or a salt thereof in which $L^1$ is -cyclobutane-1,3-diyl-$(CH_2)_p$-. The compound of the formula (I) or a salt thereof in which $L^1$ is -cyclohexane-1,4-diyl-$(CH_2)_p$-.

(3) The compound of the formula (I) or a salt thereof in which p is 0. The compound of the formula (I) or a salt thereof in which p is 1.

(4) The compound of the formula (I) or a salt thereof in which $L^2$ is H. The compound of the formula (I) or a salt thereof in which $L^2$ is a $C_{5-15}$ alkyl. The compound of the formula (I) or a salt thereof in which $L^2$ is a $C_{6-14}$ alkyl. The compound of the formula (I) or a salt thereof in which $L^2$ is a $C_{6-12}$ alkyl. The compound of the formula (I) or a salt thereof in which $L^2$ is a $C_{7-13}$ alkyl. The compound of the formula (I) or a salt thereof in which $L^2$ is a $C_{8-14}$ alkyl. The compound of the formula (I) or a salt thereof in which $L^2$ is a $C_{8-12}$ alkyl. The compound of the formula (I) or a salt thereof in which $L^2$ is a $C_{8-9}$ alkyl. The compound of the formula (I) or a salt thereof in which $L^2$ is a $C_8$ alkyl. The compound of the formula (I) or a salt thereof in which $L^2$ is a $C_9$ alkyl.

(5) The compound of the formula (I) or a salt thereof in which $L^3$ is a $C_{6-14}$ alkyl. The compound of the formula (I) or a salt thereof in which $L^3$ is a $C_{7-13}$ alkyl. The compound of the formula (I) or a salt thereof in which $L^3$ is a $C_{6-12}$ alkyl. The compound of the formula (I) or a salt thereof in which $L^3$ is a $C_{8-9}$ alkyl. The compound of the formula (I) or a salt thereof in which $L^3$ is a $C_8$ alkyl. The compound of the formula (I) or a salt thereof in which $L^3$ is a $C_9$ alkyl.

(6) The compound of the formula (I) or a salt thereof in which $R^X$ is H. The compound of the formula (I) or a salt thereof in which $R^X$ is adamantyl formed together with $L^2$, $L^3$ and the carbon atom to which they are bonded.

(7) The compound of the formula (I) or a salt thereof in which $L^4$ is a $C_{6-10}$ alkylene. The compound of the formula (I) or a salt thereof in which $L^4$ is a $C_{6-9}$ alkylene. The compound of the formula (I) or a salt thereof in which $L^4$ is a $C_{6-8}$ alkylene. The compound of the formula (I) or a salt thereof in which $L^4$ is a $C_{7-10}$ alkylene. The compound of the formula (I) or a salt thereof in which $L^4$ is a $C_{7-9}$ alkylene. The compound of the formula (I) or a salt thereof in which $L^4$ is a $C_{6-7}$ alkylene. The compound of the formula (I) or a salt thereof in which $L^4$ is a $C_7$ alkylene. The compound of the formula (I) or a salt thereof in which $L^4$ is a $C_6$ alkylene.

(8) The compound of the formula (I) or a salt thereof in which $L^5$ is a $C_{6-14}$ alkyl. The compound of the formula (I) or a salt thereof in which $L^5$ is a $C_{6-12}$ alkyl. The compound of the formula (I) or a salt thereof in which $L^5$ is a $C_{7-13}$ alkyl. The compound of the formula (I) or a salt thereof in which $L^5$ is a $C_{8-14}$ alkyl. The compound of the formula (I) or a salt thereof in which $L^5$ is a $C_{8-12}$ alkyl. The compound of the formula (I) or a salt thereof in which $L^5$ is a $C_9$ alkyl.

(9) The compound of the formula (I) or a salt thereof in which $L^6$ is a $C_{6-14}$ alkyl. The compound of the formula (I) or a salt

thereof in which $L^6$ is a $C_{6-12}$ alkyl. The compound of the formula (I) or a salt thereof in which $L^6$ is a $C_{7-13}$ alkyl. The compound of the formula (I) or a salt thereof in which $L^6$ is a $C_9$ alkyl.

(10) The compound of the formula (I) or a salt thereof in which M is C(=O)O. The compound of the formula (I) or a salt thereof in which M is OC(=O). The compound of the formula (I) or a salt thereof in which M is *C(=O)O or *OC(=O), and $L^1$ or $L^4$ is bonded to *. The compound of the formula (I) or a salt thereof in which M is *C(=O)O, and $L^1$ or $L^4$ is bonded to *. The compound of the formula (I) or a salt thereof in which one M of the two M's is *C(=O)O, the other is *OC(=O), and $L^1$ or $L^4$ is bonded to *. The compound of the formula (I) or a salt thereof in which M is *OC(=O), and $L^1$ or $L^4$ is bonded to *.

(11) The compound of the formula (I) or a salt thereof in which the ring A is a 5- to 7-membered ring composed of three to five carbon atoms, one N and one Y, where one of the hydrocarbon chains between N and Y is $CH_2CH_2$, the other is $(CH_2)_n$, and n is an integer of 1, 2 or 3. The compound of the formula (I) or a salt thereof in which the ring A is a 5- or 6-membered ring composed of three to four carbon atoms, one N and one Y, where one of the hydrocarbon chains between N and Y is $CH_2CH_2$, the other is $(CH_2)_n$, and n is an integer of 1 or 2. The compound of the formula (I) or a salt thereof in which the ring A is a 6-membered ring composed of four carbon atoms, one N and one Y, where one of the hydrocarbon chains between N and Y is $CH_2CH_2$, the other is $(CH_2)_n$, and n is 2. The compound of the formula (I) or a salt thereof in which the ring A is a 7-membered ring composed of five carbon atoms, one N and one Y, where one of the hydrocarbon chains between N and Y is $CH_2CH_2$, the other is $(CH_2)_n$, and n is 3. The compound of the formula (I) or a salt thereof in which the ring A is a diazaspirononane ring group. The compound of the formula (I) or a salt thereof in which the ring A is a diazaspiro[3.5]nonane ring group. The compound of the formula (I) or a salt thereof in which the ring A is a 2,7-diazaspiro[3.5]nonane ring group. The compound of the formula (I) or a salt thereof in which the ring A is a quinuclidine ring group, and $R^0$ does not exist. The compound of the formula (I) or a salt thereof in which the ring A is a group selected from the group consisting of a formula (a), a formula (b) and a formula (c),

[Chem. 4]

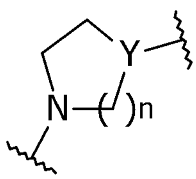

(a)

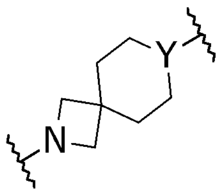

(b)

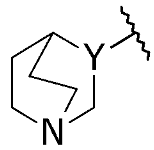

(c)

(the wavy line indicates that the right side is bonded to G and that the left side is bonded to $R^0$) in which n is an integer of 1, 2 or 3, where Y is N or CH in the formula (a), Y is N in the formula (b), and Y is CH in the formula (c). The compound of the formula (I) or a salt thereof in which the ring A is the group of the formula (a), n is an integer of 1, 2 or 3, and Y is N or CH. The compound of the formula (I) or a salt thereof in which the ring A is the group of the formula (a), n is 2, and Y is N or CH. The compound of the formula (I) or a salt thereof in which the ring A is the group of the formula (a), n is 3, and Y is N or CH. The compound of the formula (I) or a salt thereof in which the ring A is the group of the formula (b), and Y is N. The compound of the formula (I) or a salt thereof in which the ring A is the group of the formula (c), and Y is CH.

(12) The compound of the formula (I) or a salt thereof in which $R^0$ is $(C_{1-6}\ alkyl)_2N\text{-}C_{1-6}$ alkylene-C(=O)-. The compound of the formula (I) or a salt thereof in which $R^0$ is (cyclic amino)-$C_{1-6}$ alkyl-C(=O)-. The compound of the formula (I) or a salt thereof in which $R^0$ is $(CH_3)_2N\text{-}CH_2\text{-}C(=O)\text{-}$. The compound of the formula (I) or a salt thereof in which $R^0$ is piperidino-$CH_2$-C(=O)-. The compound of the formula (I) or a salt thereof in which $R^0$ is $CH_3$ or $C_2H_5$. The compound of the formula (I) or a salt thereof in which $R^0$ is $CH_3$.

(13) The compound of the formula (I) or a salt thereof which is a combination of compatible two or more of the groups described in (1) to (12) above.

The combination in (13) includes the aspects below.

(14) The compound of the formula (I) or a salt thereof in which the combination of G and Y (G, Y) is (O, CH), $R^X$ is H, M is C(=O)O, the ring A is a 6-membered ring composed of four carbon atoms, one N and one Y, one of the hydrocarbon chains between N and Y is $CH_2CH_2$, the other is $(CH_2)_n$, n is 2, and $R^0$ is $(C_{1-6}\ alkyl)_2N\text{-}C_{1-6}$ alkylene-C(=O)-.

(15) The compound of the formula (I) or a salt thereof in which the combination of G and Y (G, Y) is (a bond, N), $R^X$ is H, M is C(=O)O, the ring A is a 7-membered ring composed of five carbon atoms, one N and one Y, one of the hydrocarbon chains between N and Y is $CH_2CH_2$, the other is $(CH_2)_n$, and n is 3.

(16) The compound of the formula (I) or a salt thereof in which the combination of G and Y (G, Y) is (O, CH), $L^1$ is a $C_{6-9}$ alkylene, $L^2$ is a $C_{6-14}$ alkyl, $L^3$ is a $C_{6-14}$ alkyl, $R^X$ is H, $L^4$ is a $C_{6-9}$ alkylene, $L^5$ and $L^6$ are a $C_{6-14}$ alkyl, M is C(=O)O, the ring A is a 6-membered ring composed of four carbon atoms, one N and one Y, one of the hydrocarbon chains between N and Y is $CH_2CH_2$, the other is $(CH_2)_n$, n is 2, and $R^0$ is $(C_{1-6}$ alkyl$)_2$N-$C_{1-6}$ alkylene-C(=O)-.

(17) The compound of the formula (I) or a salt thereof in which the combination of G and Y (G, Y) is (a bond, N), $L^1$ is -$C_{3-6}$ cyclobutanediyl-$(CH_2)_p$-, p is 1, $L^2$ is a $C_{6-14}$ alkyl, $L^3$ is a $C_{6-14}$ alkyl, $R^X$ is H, $L^4$ is a $C_{6-9}$ alkylene, $L^5$ and $L^6$ are a $C_{6-14}$ alkyl, M is C(=O)O, the ring A is a 7-membered ring composed of five carbon atoms, one N and one Y, one of the hydrocarbon chains between N and Y is $CH_2CH_2$, the other is $(CH_2)_n$, n is 3, and $R^0$ is $CH_3$ or $C_2H_5$.

(18) Examples of specific compounds or salts thereof included in the invention are as follows.

Bis(2-nonylundecyl) 7,7'-[({[1-(N,N-dimethylglycyl)piperidin-4-yl]oxy}carbonyl)azanediyl]di(heptanoate) or a salt thereof,
bis(2-undecyltridecyl) 7,7-[({[1-(N,N-dimethylglycyl)piperidin-4-yl]oxy}carbonyl)azanediyl]di(heptanoate) or a salt thereof,
bis(2-decyltetradecyl) 7,7'-[({[1-(N,N-dimethylglycyl)piperidin-4-yl]oxy}carbonyl)azanediyl]di(heptanoate) or a salt thereof,
2-octyldecyl 8-[({[1-(N,N-dimethylglycyl)piperidin-4-yl]oxy} carbonyl) {7-oxo-7-[(2-undecyltridecyl)oxy]heptyl} amino]octanoate or a salt thereof,
2-nonylundecyl 8-{[(1r,3r)-3-{2-[(2-decyldodecyl)oxy]-2-oxoethyl}cyclobutyl]({[1-(N,N-dimethylglycyl)piperidin-4-yl]oxy}carbonyl)amino}octanoate or a salt thereof,
2-nonylundecyl 8-{({[1-(N,N-dimethylglycyl)piperidin-4-yl]oxy}carbonyl)[(1r,3r)-3-{2-oxo-2-[(2-undecyltridecyl) oxy]ethyl}cyclobutyl]amino}octanoate or a salt thereof,
2-nonylundecyl 8-{(4-methyl-1,4-diazepane-1-carbonyl)[(1r,3r)-3-{2-[(2-octyldecyl)oxy]-2-oxoethyl}cyclobutyl] amino}octanoate or a salt thereof,
bis(2-octyldodecyl) 9,9'-[(4-methyl-1,4-diazepane-1-carbonyl)azanediyl]di(nonanoate) or a salt thereof,
2-nonylundecyl 8-{(4-ethyl-1,4-diazepane-1-carbonyl)[(1r,3r)-3-{2-[(2-octyldecyl)oxy]-2-oxoethyl}cyclobutyl] amino}octanoate or a salt thereof,
2-nonylundecyl 8-{(4-methyl-1,4-diazepane-1-carbonyl)[(1r,4r)-4-{2-oxo-2-[(2-undecyltridecyl)oxy]ethyl}cyclohexyl]amino}octanoate or a salt thereof,
2-heptylnonyl (1r,4r)-4-[{8-[(2-heptylnonyl)oxy]-8-oxooctyl}(4-methyl-1,4-diazepane-1-carbonyl)amino]cyclohexane-1-carboxylate or a salt thereof or
2-nonylundecyl 8-{[4-(N,N-diethylglycyl)-1,4-diazepane-1-carbonyl][(1r,3r)-3-{2-[(2-octyldecyl)oxy]-2-oxoethyl} cyclobutyl]amino}octanoate or a salt thereof.

In this specification, the compound of the formula (I) or a salt thereof may be described only as one of isomers, but the invention includes isomers other than that and also includes separated isomers or mixtures thereof.

In addition, the compound of the formula (I) or a salt thereof may have an asymmetric center or an axial chirality and may have enantiomers (optical isomers) based thereon. The compound of the formula (I) or a salt thereof includes both isolated enantiomers such as (R)-form and (S)-form and mixtures thereof (including a racemic mixture or a non-racemic mixture). In an aspect, the enantiomer is "stereochemically pure". The "stereochemically pure" means the purity to a degree that one skilled in the art can recognize substantially stereochemically pure. In an aspect, the enantiomer is, for example, a compound having a stereochemical purity of 90% ee (enantiomeric excess) or more, 95% ee or more, 98% ee or more or 99% ee or more.

The salt of the compound of the formula (I) is a pharmaceutically acceptable salt and may form an acid addition salt depending on the type of the substituent. Specific examples include an acid addition salt with an inorganic acid, such as hydrochloric acid, hydrobromic acid, hydroiodic acid, sulfuric acid, nitric acid and phosphoric acid, and with an organic acid, such as formic acid, acetic acid, propionic acid, oxalic acid, malonic acid, succinic acid, fumaric acid, maleic acid, lactic acid, malic acid, mandelic acid, tartaric acid, dibenzoyltartaric acid, ditoluoyltartaric acid, citric acid, methanesulfonic acid, ethanesulfonic acid, benzenesulfonic acid, p-toluenesulfonic acid, aspartic acid and glutamic acid and the like.

Furthermore, the invention also includes various hydrates, solvates and crystal polymorphism substances of the compound of the formula (I) or a salt thereof.

The invention also includes all the compounds of the formula (I) or salts thereof which are pharmaceutically acceptable and labeled with one or more radioactive or nonradioactive isotopes. Examples of preferable isotopes used for isotope labeling of the compound of the invention include isotopes of hydrogen ($^2H$, $^3H$ and the like), carbon ($^{11}C$, $^{13}C$, $^{14}C$ and the like), nitrogen ($^{13}N$, $^{15}N$ and the like), oxygen ($^{15}O$, $^{17}O$, $^{18}O$ and the like), fluorine ($^{18}F$ and the like), chlorine ($^{36}Cl$ and the like), iodine ($^{123}I$, $^{125}I$ and the like), phosphorus ($^{32}P$ and the like) and sulfur ($^{35}S$ and the like). The isotopically labeled compound of the invention of the present application can be used for studies such as histological distribution study of drugs and/or substrates and the like. For example, a radioisotope such as tritium ($^3H$) and carbon-14 ($^{14}C$) can be used

for the purpose due to easiness of labeling and convenience of detection. Replacement with a heavier isotope, for example replacement of hydrogen with deuterium ($^2H$), is sometimes therapeutically advantageous because metabolic stability improves (for example, increased *in vivo* half-life, decreased required dose and declined drug interaction). Replacement with a positron-emitting isotope ($^{11}C$, $^{18}F$, $^{15}O$, $^{13}N$ or the like) can be used for positron emission tomography (PET) for testing the substrate acceptor occupancy. The isotopically labeled compound of the invention can be generally produced by a conventional method known to one skilled in the art or by a production method similar to those in Examples or Production Examples or the like using an appropriate isotopically labeled reagent instead of an unlabeled reagent.

2. Lipid Nanoparticles

Aspects of lipid nanoparticles containing the compound of the formula (I), which is the compound of the invention, or a salt thereof are shown below.

(Aspects of Lipid Nanoparticles)

(1) Lipid nanoparticles containing the compound of the formula (I) or a salt thereof which contain a neutral lipid and a PEGylated lipid.

(1a) Lipid nanoparticles containing the compound of the formula (I) or a salt thereof in which the neutral lipid is a phospholipid and a sterol. Lipid nanoparticles containing the compound of the formula (I) or a salt thereof in which the neutral lipid is DSPC and cholesterol.

(1b) The lipid nanoparticles described in (1) in which the phospholipid is DSPC. The lipid nanoparticles described in (1) in which the phospholipid is DSPC, DOPE, DOPC, DPPC, DHSM, SOPC or DoPhPE.

(1c) The lipid nanoparticles described in (1a) in which the sterol is cholesterol. The lipid nanoparticles described in (1a) in which the sterol is cholesterol or β-sitosterol.

(1d) The lipid nanoparticles described in (1) in which the PEGylated lipid is DMG-PEG2000. The lipid nanoparticles described in (1) in which the PEGylated lipid is DMG-PEG2000, C8 PEG2000 ceramide or polyethylene glycol monostearate.

(1e) The lipid nanoparticles described in (1) in which the composition ratio of the compound of the formula (I) or the salt thereof is 20.0 to 80.0 mol% based on the total amount of the lipid nanoparticles. The lipid nanoparticles described in (1) in which the composition ratio of the compound of the formula (I) or the salt thereof is 20.0 to 70.0 mol% based on the total amount of the lipid nanoparticles. Lipid nanoparticles containing the compound of the formula (I) or a salt thereof in which the composition ratio of the compound of the formula (I) or the salt thereof is 30.0 to 70.0 mol% based on the total amount of the lipid nanoparticles. The lipid nanoparticles described in (1) in which the composition ratio of the compound of the formula (I) or the salt thereof is 40.0 to 55.0 mol% based on the total amount of the lipid nanoparticles. The lipid nanoparticles described in (1) in which the composition ratio of the compound of the formula (I) or the salt thereof is 40.0 to 60.0 mol% based on the total amount of the lipid nanoparticles. The lipid nanoparticles described in (1) in which the composition ratio of the compound of the formula (I) or the salt thereof is 35.0 to 50.0 mol% based on the total amount of the lipid nanoparticles.

(1f) The lipid nanoparticles described in (1) in which the composition ratio of the neutral lipid is 10.0 to 79.9 mol% based on the total amount of the lipid nanoparticles. The lipid nanoparticles described in (1) in which the composition ratio of the neutral lipid is 27.0 to 79.5 mol% based on the total amount of the lipid nanoparticles. The lipid nanoparticles described in (1) in which the composition ratio of the neutral lipid is 27.5 to 69.0 mol% based on the total amount of the lipid nanoparticles. The lipid nanoparticles described in (1) in which the composition ratio of the neutral lipid is 37.5 to 59.0 mol% based on the total amount of the lipid nanoparticles. The lipid nanoparticles described in (1) in which the composition ratio of the neutral lipid is 38.0 to 59.0 mol% based on the total amount of the lipid nanoparticles. The lipid nanoparticles described in (1) in which the composition ratio of the neutral lipid is 47.5 to 64.0 mol% based on the total amount of the lipid nanoparticles. The lipid nanoparticles described in (1) in which the composition ratio of the neutral lipid is 42.0 to 59.0 mol% based on the total amount of the lipid nanoparticles.

(1g) The lipid nanoparticles described in (1a) in which the composition ratio of the phospholipid is 0 to 18.0 mol% based on the total amount of the lipid nanoparticles. The lipid nanoparticles described in (1a) in which the composition ratio of the phospholipid is 0 to 16.0 mol% based on the total amount of the lipid nanoparticles. The lipid nanoparticles described in (1a) in which the composition ratio of the phospholipid is 0 to 14.0 mol% based on the total amount of the lipid nanoparticles. The lipid nanoparticles described in (1a) in which the composition ratio of the phospholipid is 0 to 13.0 mol% based on the total amount of the lipid nanoparticles.

(1h) The lipid nanoparticles described in (1a) in which the composition ratio of the sterol is 22.5 to 62.5 mol% based on the total amount of the lipid nanoparticles. The lipid nanoparticles described in (1a) in which the composition ratio of the sterol is 22.5 to 58.5 mol% based on the total amount of the lipid nanoparticles. The lipid nanoparticles described in (1a) in which the composition ratio of the sterol is 38.5 to 58.5 mol% based on the total amount of the lipid nanoparticles. The lipid nanoparticles described in (1a) in which the composition ratio of the sterol is 26.5 to 52.5 mol% based on the total amount of the lipid nanoparticles.

(1i) The lipid nanoparticles described in (1) in which the composition ratio of the PEGylated lipid is 0.1 to 10 mol% based on the total amount of the lipid nanoparticles. The lipid nanoparticles described in (1) in which the composition ratio of the PEGylated lipid is 1.0 to 3.0 mol% based on the total amount of the lipid nanoparticles. The lipid nanoparticles described in (1) in which the composition ratio of the PEGylated lipid is 0.5 to 3.0 mol% based on the total amount of the lipid nanoparticles. The lipid nanoparticles described in (1) in which the composition ratio of the PEGylated lipid is 1.0 to 2.5 mol% based on the total amount of the lipid nanoparticles. The lipid nanoparticles described in (1) in which the composition ratio of the PEGylated lipid is 1.0 to 2.0 mol% based on the total amount of the lipid nanoparticles.

(2) Lipid nanoparticles containing the compound of the formula (I) or a salt thereof which encapsulate a nucleic acid. Lipid nanoparticles containing the compound of the formula (I) or a salt thereof which encapsulate a nucleic acid or particularly a nucleic acid which is useful for preventing and/or treating an astrocyte-related disease.

(3) The lipid nanoparticles described in (2) which contain a neutral lipid and a PEGylated lipid.

(3a) The lipid nanoparticles described in (3) in which the nucleic acid is mRNA. The lipid nanoparticles described in (3) in which the nucleic acid is a nucleic acid which is useful for preventing and/or treating an astrocyte-related disease. The lipid nanoparticles described in (3) in which the nucleic acid is mRNA which is useful for preventing and/or treating an astrocyte-related disease. The lipid nanoparticles described in (3) in which the nucleic acid is Ascl1 mRNA, Dlx2 mRNA, NeuroD1 mRNA, Ngn2 mRNA, BDNF mRNA, NGF mRNA or NT-3 mRNA. The lipid nanoparticles described in (3) in which the nucleic acid is BDNF mRNA or NeuroD1 mRNA. The lipid nanoparticles described in (3) in which the nucleic acid is NeuroD1 mRNA.

(3b) The lipid nanoparticles described in (3) in which the nucleic acid is NeuroD1 mRNA containing a base sequence encoding a protein having a sequence identity of 90% or more (for example, 95% or more, 98% or more, 99% or more, 99.4% or more or 99.5% or more) to a protein having the amino acid sequence of SEQ ID NO: 2. The lipid nanoparticles described in (3) in which the nucleic acid is NeuroD1 mRNA containing a base sequence having a sequence identity of 70% or more (for example, 80% or more, 90% or more or 95% or more) to the base sequence of SEQ ID NO: 1 or 3 and encodes the amino acid sequence of SEQ ID NO: 2. The lipid nanoparticles described in (3) in which the nucleic acid is NeuroD1 mRNA containing a base sequence having a sequence identity of 70% or more (for example, 80% or more, 90% or more or 95% or more) to the base sequence of SEQ ID NO: 1 and encodes the amino acid sequence of SEQ ID NO: 2. The lipid nanoparticles described in (3) in which the nucleic acid is NeuroD1 mRNA containing a base sequence having a sequence identity of 70% or more (for example, 80% or more, 90% or more or 95% or more) to a base sequence shown as positions 44 to 1114 of SEQ ID NO: 4 and encodes the amino acid sequence of SEQ ID NO: 2. The lipid nanoparticles described in (3) in which the nucleic acid is NeuroD1 mRNA containing a base sequence having a sequence identity of 70% or more (for example, 80% or more, 90% or more or 95% or more) to a base sequence shown as positions 44 to 1114 of SEQ ID NO: 5 and encodes the amino acid sequence of SEQ ID NO: 2. The lipid nanoparticles described in (3) in which the nucleic acid is NeuroD1 mRNA containing a base sequence having a sequence identity of 70% or more (for example, 80% or more, 90% or more or 95% or more) to a base sequence shown as positions 44 to 1114 of SEQ ID NO: 5 and encodes the amino acid sequence of SEQ ID NO: 2. The lipid nanoparticles described in (3) in which the nucleic acid is NeuroD1 mRNA containing a base sequence having a sequence identity of 70% or more (for example, 80% or more, 90% or more or 95% or more) to a base sequence shown as positions 44 to 1114 of SEQ ID NO: 5 and encodes the amino acid sequence of SEQ ID NO: 2.

(3c) The lipid nanoparticles described in (3) in which the nucleic acid is NeuroD1 mRNA containing a base sequence encoding a protein having the amino acid sequence of SEQ ID NO: 2. The lipid nanoparticles described in (3) in which the nucleic acid is NeuroD1 mRNA containing the base sequence of SEQ ID NO: 1. The lipid nanoparticles described in (3) in which the nucleic acid is NeuroD1 mRNA containing the base sequence of SEQ ID NO: 3. The lipid nanoparticles described in (3) in which the nucleic acid is NeuroD1 mRNA containing the base sequence of SEQ ID NO: 4. The lipid nanoparticles described in (3) in which the nucleic acid is NeuroD1 mRNA containing the base sequence of SEQ ID NO: 5.

(3d) The lipid nanoparticles described in (3) in which the nucleic acid is NeuroD1 mRNA having a 5' cap that is Cap-0, Cap-1 or Cap-2. The lipid nanoparticles described in (3) in which the nucleic acid is NeuroD1 mRNA having a 5' cap that is Cap-1.

(3e) The lipid nanoparticles described in (3) in which the nucleic acid is NeuroD1 mRNA having a start codon and a 5' UTR containing the Kozak sequence. The lipid nanoparticles described in (3) in which the nucleic acid is NeuroD1 mRNA having a 5' UTR or a 3' UTR that is derived from α-globin gene. The lipid nanoparticles described in (3) in which the nucleic acid is NeuroD1 mRNA having a 5' UTR or a 3' UTR that is derived from human α-globin gene. The lipid nanoparticles described in (3) in which the nucleic acid is NeuroD1 mRNA having a 5' UTR or a 3' UTR that is derived from β-globin gene. The lipid nanoparticles described in (3) in which the nucleic acid is NeuroD1 mRNA having a 5' UTR or a 3' UTR that is derived from human β-globin gene. The lipid nanoparticles described in (3) in which the nucleic acid is NeuroD1 mRNA having a start codon and a 5' UTR containing the Kozak sequence. The lipid nanoparticles described in (3) in which the nucleic acid is NeuroD1 mRNA having a 5' UTR that is derived from α-globin gene. The lipid nanoparticles described in (3) in which the nucleic acid is NeuroD1 mRNA having a 5' UTR that is derived from human α-globin gene. The lipid nanoparticles described in (3) in which the nucleic acid is NeuroD1 mRNA having a 5' UTR that is derived from β-globin gene. The lipid nanoparticles described in (3) in which the nucleic acid is NeuroD1 mRNA having a 5' UTR that is derived from human β-globin gene. The lipid nanoparticles described in (3) in which the nucleic acid is NeuroD1 mRNA having a 3' UTR that is derived from α-globin gene. The lipid nanoparticles described in (3) in which the nucleic acid is NeuroD1 mRNA having a 3' UTR that is derived from β-globin gene. The lipid nanoparticles described in (3) in which the nucleic acid is NeuroD1 mRNA having a 3' UTR that is derived from human α-globin gene. The lipid nanoparticles described in (3) in which the nucleic acid is NeuroD1 mRNA having a 3' UTR that is derived from human β-globin gene.

(3f) The lipid nanoparticles described in (3) in which the nucleic acid is NeuroD1 mRNA containing a modified nucleotide. The lipid nanoparticles described in (3) in which the nucleic acid is NeuroD1 mRNA containing an N1-methylpseudouridine. The lipid nanoparticles described in (3) in which the nucleic acid is NeuroD1 mRNA in which a part or all of the uridines are N1-methylpseudouridines. The lipid nanoparticles described in (3) in which the nucleic acid is NeuroD1 mRNA in which a part of the uridines have been substituted with N1-methylpseudouridines. The lipid nanoparticles described in (3) in which the nucleic acid is NeuroD1 mRNA in which all the uridines have been substituted with N1-methylpseudouridines.

(3g) The lipid nanoparticles described in (3) in which the nucleic acid is NeuroD1 mRNA having a poly(A) chain of 20 to 1000 bases. The lipid nanoparticles described in (3) in which the nucleic acid is NeuroD1 mRNA having a poly(A) chain of 30 to 500 bases, 50 to 200 bases, 60 to 150 bases, 70 to 130 bases, 70 to 120 bases, 70 to 80 bases, 120 bases or 79 bases. The lipid nanoparticles described in (3) in which the nucleic acid is NeuroD1 mRNA having a poly(A) chain of 120 bases. The lipid nanoparticles described in (3) in which the nucleic acid is NeuroD1 mRNA having a poly(A) chain of 79 bases.

(4) Lipid nanoparticles encapsulating a nucleic acid and containing a neutral lipid, a PEGylated lipid and the compound of the formula (I) or a salt thereof which are of a combination of compatible two or more of the aspects (1) to (18) of the cationic lipid and the aspect (1) of the lipid nanoparticles of the aspects described in (2) and (3) above. Lipid nanoparticles encapsulating a nucleic acid and containing a neutral lipid, a PEGylated lipid and the compound of the formula (I) or a salt thereof which are of a combination of compatible two or more of the aspects (1) to (18) of the cationic lipid and the aspects (2) and (3) of the lipid nanoparticles encapsulating a nucleic acid of the above aspects.

Specific examples of the combinations of (4) above are the aspects below.

(4a) Lipid nanoparticles which encapsulate a nucleic acid,
in which the nucleic acid is mRNA encoding NeuroD1 protein, and
the lipid nanoparticles contain the compound of the formula (I) or the salt thereof, neutral lipids and a PEGylated lipid, wherein the compound of the formula (I) or the salt thereof is bis(2-nonylundecyl) 7,7'-[({[1-(N,N-dimethylglycyl) piperidin-4-yl]oxy}carbonyl)azanediyl]di(heptanoate) or a salt thereof, the neutral lipids that are DSPC and cholesterol and the PEGylated lipid that is DMG-PEG2000.

(4b) The lipid nanoparticles described in (4a) in which the nucleic acid is mRNA encoding NeuroD1 protein containing a base sequence encoding a protein having the amino acid sequence of SEQ ID NO: 2.

(4c) The lipid nanoparticles described in (4a) in which the nucleic acid is mRNA encoding NeuroD1 protein containing the base sequence of SEQ ID NO: 1.

(4d) The lipid nanoparticles described in (4a) in which the nucleic acid is mRNA encoding NeuroD1 protein containing the base sequence of SEQ ID NO: 3.

(4e) The lipid nanoparticles described in (4a) in which the nucleic acid is mRNA encoding NeuroD1 protein containing the base sequence of SEQ ID NO: 4.

(4f) The lipid nanoparticles described in (4a) in which the nucleic acid is mRNA encoding NeuroD1 protein containing the base sequence of SEQ ID NO: 5.

(4g) Lipid nanoparticles containing 20.0 to 80.0 mol% of bis(2-nonylundecyl) 7,7'-[({[1-(N,N-dimethylglycyl)piperidin-4-yl]oxy}carbonyl)azanediyl]di(heptanoate) or a salt thereof, 10.0 to 79.9 mol% of DSPC and cholesterol and 0.1 to 10 mol% of DMG-PEG2000 based on the total amount of the lipid nanoparticles in (4a) to (4f).
(4h) Lipid nanoparticles containing 40.0 to 55.0 mol% of bis(2-nonylundecyl) 7,7'-[({[1-(N,N-dimethylglycyl)piperidin-4-yl]oxy}carbonyl)azanediyl]di(heptanoate) or a salt thereof, 42.0 to 59.0 mol% of DSPC and cholesterol and 1.0 to 3.0 mol% of DMG-PEG2000 based on the total amount of the lipid nanoparticles in (4a) to (4f).
(4i) Lipid nanoparticles which encapsulate a nucleic acid and contain a neutral lipid, a PEGylated lipid and the compound of the formula (I) or a salt thereof,
in which the nucleic acid is mRNA encoding NeuroD1 protein in which in the base sequence, the nucleotides at positions 1 to 3 are a 5' terminal sequence suitable for the capping method using CleanCap (registered trademark) Reagent AG (TriLink BioTechnologies), positions 4 to 43 are a 5' UTR, positions 44 to 1114 are CDS of human NeuroD1 gene (SEQ ID NO: 3), positions 1115 to 1120 are two successive stop codons, positions 1121 to 1231 are a 3' UTR, and positions 1232 to 1310 correspond to a poly(A) chain.

Aspects of the specific lipid nanoparticles included in the invention are as follows.

(5a) Lipid nanoparticles which encapsulate a nucleic acid,

in which the nucleic acid is mRNA encoding human ND1 having a base sequence (SEQ ID NO: 5) in which all the uridines are N1-methylpseudouridines in a base sequence (SEQ ID NO: 4) having a 5' cap structure that is Cap-1, a 5' UTR that is derived from human $\alpha$-globin gene, a CDS that is CDS of human NeuroD1 gene (SEQ ID NO: 3), a 3' UTR that is derived from human $\alpha$-globin gene and a poly(A) chain containing 79 bases, where in the base sequence of SEQ ID NO: 4 or SEQ ID NO: 5, the nucleotides at positions 1 to 3 correspond to AGG, positions 4 to 43 correspond to the 5' UTR, positions 44 to 1114 correspond to CDS of human NeuroD1 gene (SEQ ID NO: 3), positions 1115 to 1120 correspond to two successive stop codons, positions 1121 to 1231 correspond to the 3' UTR, and positions 1232 to 1310 correspond to the poly(A) chain, and
the lipid nanoparticles contain the compound of the formula (I) or the salt thereof, neutral lipids and a PEGylated lipid, wherein the compound of the formula (I) or the salt thereof is bis(2-nonylundecyl) 7,7'-[({[1-(N,N-dimethylglycyl)piperidin-4-yl]oxy}carbonyl)azanediyl]di(heptanoate) or a salt thereof, the neutral lipids that are DSPC and cholesterol and the PEGylated lipid that is DMG-PEG2000.

(5b) The lipid nanoparticles described in (5a) which encapsulate a nucleic acid and which contain 50 mol% of bis(2-nonylundecyl) 7,7'-[({[1-(N,N-dimethylglycyl)piperidin-4-yl]oxy}carbonyl)azanediyl]di(heptanoate) or a salt thereof, 48.5 mol% of DSPC and cholesterol and 1.5 mol% of DMG-PEG2000 based on the total amount of the lipid nanoparticles.

3. Pharmaceutical Composition

Aspects of the pharmaceutical composition containing lipid nanoparticles containing the compound of the formula (I), which is the compound of the invention, or a salt thereof and one or more pharmaceutically acceptable pharmaceutical additives are shown below.

(Aspects of Pharmaceutical Composition)

(6a) The pharmaceutical composition containing lipid nanoparticles described in any of the aspects (2), (3) to (3g), (4) to (4i) and (5a) to (5b) of the lipid nanoparticles and one or more pharmaceutically acceptable pharmaceutical additives.
(6b) The pharmaceutical composition described in (6a) for preventing or treating an astrocyte-related disease.
(6c) A pharmaceutical composition containing the lipid nanoparticles described in (5a) and one or more pharmaceutically acceptable pharmaceutical additives.
(6d) A pharmaceutical composition containing the lipid nanoparticles described in (5b) and one or more pharmaceutically acceptable pharmaceutical additives.

Aspects of the specific pharmaceutical compositions included in the invention are as follows.

(6e) A pharmaceutical composition which encapsulate a nucleic acid and contain neutral lipids, a PEGylated lipid and

the compound of the formula (I) or a salt thereof,

in which the nucleic acid is mRNA encoding NeuroD1 protein, and
the compound of the formula (I) or the salt thereof is bis(2-nonylundecyl) 7,7'-[({[1-(N,N-dimethylglycyl)piperidin-4-yl]oxy}carbonyl)azanediyl]di(heptanoate) or a salt thereof,
the neutral lipids are DSPC and cholesterol, and
the PEGylated lipid is DMG-PEG2000.

(6f) A pharmaceutical composition which encapsulate a nucleic acid and contain neutral lipids, a PEGylated lipid and the compound of the formula (I) or a salt thereof,

in which the nucleic acid is mRNA encoding NeuroD1 protein containing a base sequence encoding a protein having the amino acid sequence of SEQ ID NO: 2, and
the lipid nanoparticles contain the compound of the formula (I) or the salt thereof is bis(2-nonylundecyl) 7,7'-[({[1-(N,N-dimethylglycyl)piperidin-4-yl]oxy}carbonyl)azanediyl]di(heptanoate) or a salt thereof,
the neutral lipids are DSPC and cholesterol, and
the PEGylated lipid is DMG-PEG2000.

(6g) A pharmaceutical composition containing lipid nanoparticles which encapsulate a nucleic acid and contain neutral lipids, a PEGylated lipid and the compound of the formula (I) or a salt thereof and

in which the nucleic acid is mRNA encoding human ND1 having a base sequence (SEQ ID NO: 5) in which all the uridines are N1-methylpseudouridines in a base sequence (SEQ ID NO: 4) having a 5' cap structure that is Cap-1, a 5' UTR that is derived from human $\alpha$-globin gene, a CDS that is CDS of human NeuroD1 gene (SEQ ID NO: 3), a 3' UTR that is derived from human $\alpha$-globin gene and a poly(A) chain containing 79 bases, where in the base sequence of SEQ ID NO: 4 or SEQ ID NO: 5, the nucleotides at positions 1 to 3 correspond to AGG, positions 4 to 43 correspond to the 5' UTR, positions 44 to 1114 correspond to CDS of human NeuroD1 gene (SEQ ID NO: 3), positions 1115 to 1120 correspond to two successive stop codons, positions 1121 to 1231 correspond to the 3' UTR, and positions 1232 to 1310 correspond to the poly(A) chain, and
the lipid nanoparticles contain the compound of the formula (I) or the salt thereof, neutral lipids and a PEGylated lipid, wherein the compound of the formula (I) or the salt thereof is bis(2-nonylundecyl) 7,7'-[({[1-(N,N-dimethylglycyl)piperidin-4-yl]oxy} carbonyl)azanediyl]di(heptanoate) or a salt thereof, the neutral lipids are DSPC and cholesterol, and the PEGylated lipid is DMG-PEG2000.

Although the administration form of the pharmaceutical composition is not limited, examples thereof include parenteral administration by intracerebral, intraarticular, intravenous, intramuscular, subcutaneous or other injection, a transmucosal liquid agent, an inhalant or the like. In general, through intracerebral administration, the pharmaceutical composition is delivered to the central nervous system. In an aspect, the administration is administration into the brain parenchyma. In an aspect, the administration is intraventricular administration. In an aspect, the administration is intraspinal administration. In an aspect, the administration is intrathecal administration. In an aspect, the administration is administration into the cord parenchyma.

The injection may contain a sterile aqueous or nonaqueous solution, suspension or emulsion. Examples of the aqueous solvent include distilled water for injection or physiological saline. An example of the nonaqueous solvent is an alcohol such as ethanol. Such a composition may further contain an isotonizing agent, an antiseptic, a wetting agent, an emulsifier, a dispersant, a stabilizer or a dissolution aid. These are sterilized, for example, by filtration through a bacteria keeping filter, incorporation of a microbicide or irradiation. In addition, such a composition can be produced as a sterile solid composition which is dissolved or suspended in sterile water or a sterile solvent for injection before use.

The transmucosal agent such as an inhalant or a transnasal agent is used in a liquid form and can be produced according to a conventionally known method. For example, known pharmaceutical additives and in addition, a pH modifier, an antiseptic, a surfactant, a lubricant, a stabilizer, a thickener or the like may be appropriately added. The administration can be performed using an appropriate device for inhalation or insufflation. For example, the agent can be administered using a known device such as a metering and administering inhalation device or an atomizer, as a compound alone or a powder of a formulated mixture, or as a solution or a suspension in combination with a pharmaceutically acceptable carrier. A dry powder inhaler or the like may be for a single administration or multiple administrations. The agent may be used in a form of a pressurized aerosol spray or the like using an appropriate ejection agent, for example, a suitable gas, such as a chlorofluoroalkane or carbon dioxide.

In general, in the case of intracerebral administration, the administration is at about 0.001 to 10 mg per 1 kg of the brain in an aspect, at about 0.001 to 50 mg per 1 kg of the brain in an aspect or at about 0.001 to 100 mg per 1 kg of the brain

in an aspect, once a day or in several divided portions. The dose is appropriately decided depending on the individual case considering the disease, the symptom, the age, the gender and the like.

In general, in the case of intramuscular administration, the daily dose of the pharmaceutical composition of the invention is appropriately about 0.00001 to 10 mg/kg per body weight, and the administration is once a day or in several divided portions. The dose is appropriately decided depending on the individual case considering the disease, the symptom, the age, the gender and the like.

Although it depends on the route of administration, the dosage form, the site of administration and the types of the pharmaceutical additives, the pharmaceutical composition of the invention contains, based on the total weight of the pharmaceutical composition, 5 to 50 weight% of the lipid nanoparticles in an aspect, and the amount of the lipid nanoparticles or the like is 3 to 70 weight% in an aspect or 10 to 50 weight% in an aspect.

The pharmaceutical composition of the invention can be used in combination with various therapeutic agents or preventive agents for a disease to which the pharmaceutical composition is considered to have an effectiveness. The combination use may be simultaneous administration or separate administration which is sequential or at a desired interval. A simultaneous administration preparation may be a formulated agent or may be separately formulated.

4. Production Method of Cationic Lipid

The compound of the formula (I) or a salt thereof can be produced by applying various known synthetic methods using characteristics based on the basic structure or the type of substituent thereof. Here, depending on the type of the functional group, it is sometimes effective as a production technique to substitute the functional group with an appropriate protective group (a group that can be easily converted to the functional group) in the process from a raw material to an intermediate. Examples of the protective group include protective groups described in Wuts (P. G. M. Wuts) and Greene (T. W. Greene), "Greene's Protective Groups in Organic Synthesis (4th edition, 2006)" and the like, and a group may be appropriately selected and used depending on the reaction conditions. In such a method, a desired compound can be produced by introducing the protective group and then removing the protective group, when required.

The compound of the formula (I), which is the compound of the invention, or a salt thereof can be produced by the following production methods.

Typical methods for producing the compound of the formula (I) or a salt thereof are explained below. The production methods can also be carried out referring to the references cited. In the following description, a compound or a salt thereof is referred to as a compound. In this regard, the production method of the invention is not limited to the examples shown below.

(First Production Method)

[Chem. 5]

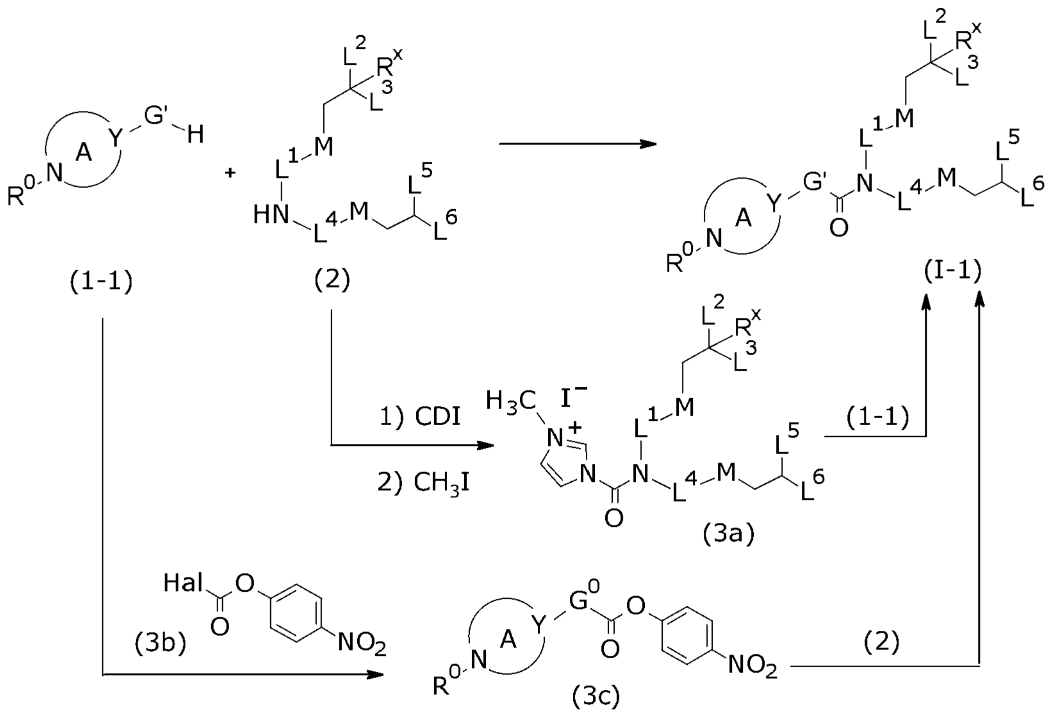


(In the formulae, the combination (G', Y) is (O, CH) or (a bond, N). The combination ($G^0$, Y) is (O, CH), and (G', Y) in a compound (1-1), which is a raw material for producing a compound (3c), and a target product (I-1) is ($G^0$, Y), that is, (O, CH). Hal represents a halogen.)

Regarding a compound (I-1) of the invention, a compound (1-1) and a compound (2) can be reacted with an equal amount or an excess amount of a carbonylation reagent in a solvent inert to the reaction, in the presence of a base, from under cooling to under heating, at -20°C to 100°C in an aspect, generally for 0.1 hours to around a day. Examples of the solvent include a halogenated hydrocarbon such as DCM and 1,2-dichloroethane, an aromatic hydrocarbon such as toluene, an ether such as THF, diethyl ether, 1,4-dioxane and CPME, DMF, DMSO, MeCN, ethyl acetate and a mixture thereof. Examples of the carbonylation reagent include triphosgene, diphosgene, 4-nitrophenyl chloroformate, phenyl chloroformate, bis(pentafluorophenyl) carbonate, CDI and the like. Examples of the base include $K_2CO_3$, KOtBu, DIPEA or the like. The compound (I-1) of the invention can be produced by reacting the compound (1-1) and the compound (2) and then using an acid such as TFA from at room temperature to under heating, at 40°C to 145°C in an aspect.

In this regard, the compound (I-1) can also be produced through isolating the compound (1-1) or the compound (2), each of which is a raw material, once as an active derivative. The following shows exemplary production methods through such an active derivative. The respective methods are referred to as the compound (3a) route and as the compound (3c) route.

(First Production Method - Compound (3a) Route)

If the compound (I-1) is produced through the compound (3a), which is an active derivative of the compound (2), a two-step process of the following steps is used: producing the compound (3a) from the compound (2); and then producing the compound (I-1) from the compound (3a).

The step of producing the compound (3a) from the compound (2) will be described. The compound (3a) can be produced by inducing the compound (2) to a carbamoylimidazol compound and then methylating the compound. In the first step, CDI is added to the compound (2), and the reaction is conducted in the presence or the absence of a base. As the base, $Et_3N$ or pyridine can be used, and as the solvent, THF, DCM, CPME, DMF or the like can be used. The temperature may be 0°C to reflux. In the subsequent methylation step, the reaction can be conducted with a methylating agent such as $CH_3I$ in a solvent such as MeCN, chloroform or DCM at room temperature to 90°C for 12 hours to 36 hours.

The step of producing the compound (I-1) of the invention from the compound (3a) will be described.

The compound (1-1) and the compound (3a) are used in an equal amount or with either thereof in an excess amount, and a mixture thereof is stirred in a solvent inert to the reaction such as DCM from under cooling to under heating, at 70°C to 160°C in an aspect, generally for 0.1 hours to 5 days. NaH or DIPEA can be used as a base, and DMF, THF, DCM

or the like can be used as the solvent.

(First Production Method - Compound (3c) Route)

If the compound (I-1) is produced through the compound (3c), which is an active derivative of the compound (1-1), a two-step process of the following steps is used: producing the compound (3c) from the compound (1-1); and then producing the compound (I-1) from the compound (3c).

The step of producing the compound (3c) from the compound (1-1) will be described. The compound (1-1) and the compound (3b) are used in an equal amount or with either thereof in an excess amount, and a mixture thereof is stirred in a solvent inert to the reaction such as THF from under cooling to under heating, at 70°C to 160°C in an aspect, generally for 0.1 hours to 5 days.

The step of producing the compound (I-1) of the invention from the compound (3c) will be described. The compound (3c) and the compound (2) are used in an equal amount or with either thereof in an excess amount, and a mixture thereof is stirred in the presence of a base, in a solvent inert to the reaction from under cooling to under heating, at 70°C to 160°C in an aspect, generally for 0.1 hours to 5 days. Examples of the solvent include a halogenated hydrocarbon such as chloroform, an ether such as THF, DMF, DMSO, ethyl acetate, acetonitrile or water and a mixed solvent thereof. Examples of the base include an organic base such as triethylamine, DIPEA or N-methylmorpholine.

In the First Production Method, alternatively, the (I-1) can be produced by conducting the reaction with a compound which is a compound (1-1) with $R^0$ therein being a protective group as a starting material, then conducting deprotection for the protective group, and adding $R^0$ through an appropriate reaction (the same is applied to production methods below). Examples of the protective group in the First Production Method include a tert-butoxycarbonyl group and a benzyloxycarbonyl group.

[Reference] Tetrahedron 61 (2005) 7153-7175

(Second Production Method)

[Chem. 6]

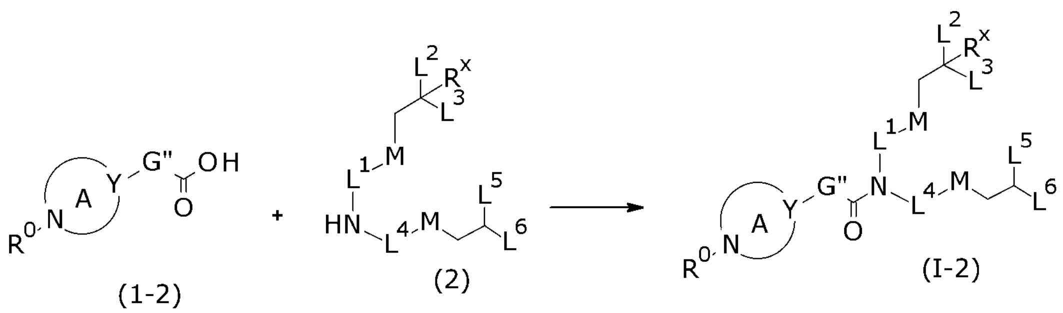

(In the formulae, the combination (G", Y) is ($CH_2$, CH).)

A compound (I-2) of the invention can be produced from a compound (1-2) and the compound (2).

In this reaction, the compound (1-2) and the compound (2) are used in an equal amount or with either thereof in an excess amount, and a mixture thereof is stirred in the presence of a condensing agent, in a solvent inert to the reaction, from under cooling to under heating, at -20°C to 60°C in an aspect, generally for 0.1 hours to five days. Examples of the solvent include a halogenated hydrocarbon such as DCM, an ether such as THF, DMF, DMSO, ethyl acetate, acetonitrile or water and a mixture thereof. Examples of the condensing agent include HATU, EDCI·HCl, CDI, dicyclohexylcarbodiimide, diphenylphosphoryl azide and phosphorus oxychloride. An additive (for example, 1-hydroxybenzotriazole, DMAP) sometimes progresses the reaction more smoothly. The reaction sometimes progresses smoothly when an organic base such as triethylamine, DIPEA and N-methylmorpholine or an inorganic base such as potassium carbonate, sodium carbonate and potassium hydroxide is added.

A method in which the carboxylic acid (1-2) is converted to a reactive derivative and then reacted with the compound (2) can also be used. Examples of the reactive derivative of the carboxylic acid include an acid halogenation product obtained by a reaction with a halogenating agent such as phosphorus oxychloride and thionyl chloride, a mixed acid anhydride obtained by a reaction with isobutyl chloroformate or the like, an active ester and the like obtained by condensation with 1-hydroxybenzotriazole or the like. The reaction of such a reactive derivative and the compound (2) can

be performed in a solvent inert to the reaction such as a halogenated hydrocarbon, an aromatic hydrocarbon and an ether, from under cooling to under heating or at -20°C to 60°C in an aspect.

(Third Production Method)

[Chem. 7]

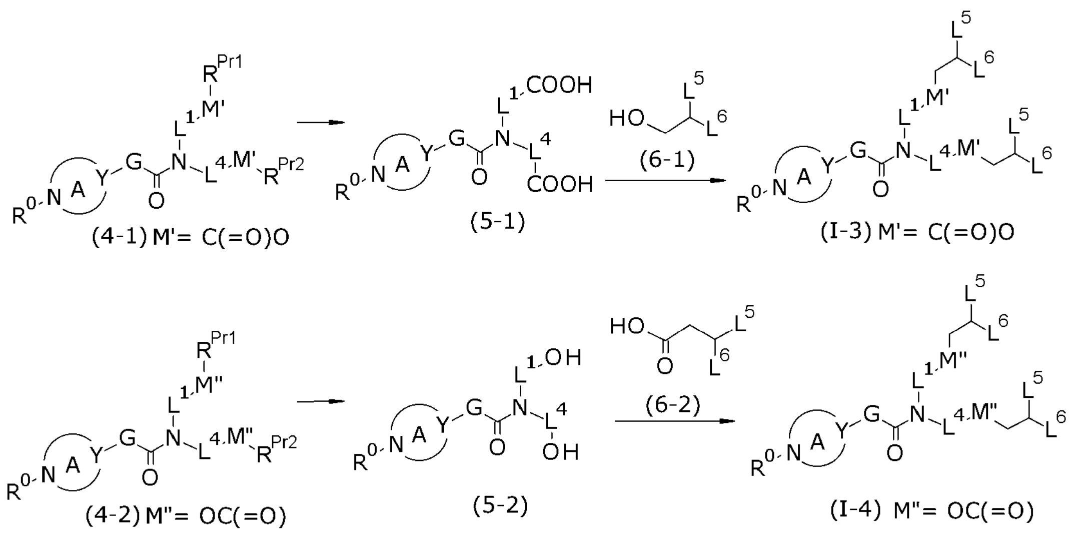


(4-1) M'= C(=O)O (5-1) (I-3) M'= C(=O)O

(4-2) M"= OC(=O) (5-2) (I-4) M"= OC(=O)

(In the formulae, $R^{Pr1}$ and $R^{Pr2}$ are the same or different and represent a $C_{1-6}$ alkyl or benzyl.)

A compound (I-3) of the invention, in which $L^2$ and $L^3$ are $L^5$ and $L^6$, respectively, $R^x$ is H and M is C(=O)O in the compound of the invention, can be produced from a compound (4-1) as a raw material by hydrolysis and esterification.

A compound (5-1) can be produced under conditions for deprotection of the compound (4-1) such as hydrolysis reaction and catalytic reduction thereof. Here, it can also be referred to Greene and Wuts, "Protective Groups in Organic Synthesis", 4th edition, John Wiley & Sons Inc, 1999.

The compound (I-3) of the invention can be produced by esterification of the compound (5-1) and the compound (6-1).

In this reaction, the compound (5-1) and a compound (6-1) are used in an equal amount or with either thereof in an excess amount, and a mixture thereof is stirred in the presence of a condensing agent, in a solvent inert to the reaction, from under cooling to under heating, at -20°C to 60°C in an aspect, generally for 0.1 hours to five days. Examples of the solvent include an aromatic hydrocarbon such as toluene, a halogenated hydrocarbon such as DCM, an ether such as diethyl ether and DMF. Examples of the condensing agent include CDI, HATU, 1-(3-dimethylaminopropyl)-3-ethylcarbodiimide, dicyclohexylcarbodiimide, diphenylphosphoryl azide and phosphorus oxychloride. DMAP or the like as an additive and DIPEA or the like as a base sometimes progress the reaction smoothly.

Similarly, a compound (I-4) of the invention, in which M is OC(=O) in the compound of the invention, can be produced from a compound (4-2) as a raw material by hydrolysis and esterification through a compound (5-2).

(Fourth Production Method)

[Chem. 8]

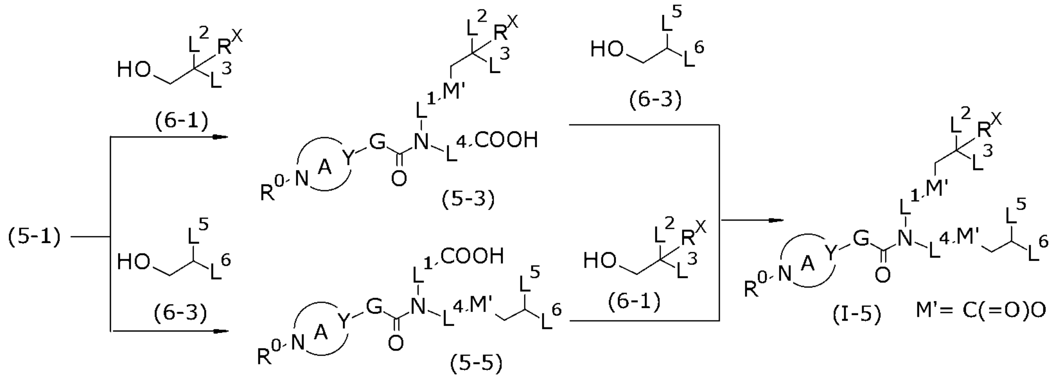

(5-1) (6-1) (6-3) (5-3) (5-5) (6-3) (6-1) (I-5) M'= C(=O)O

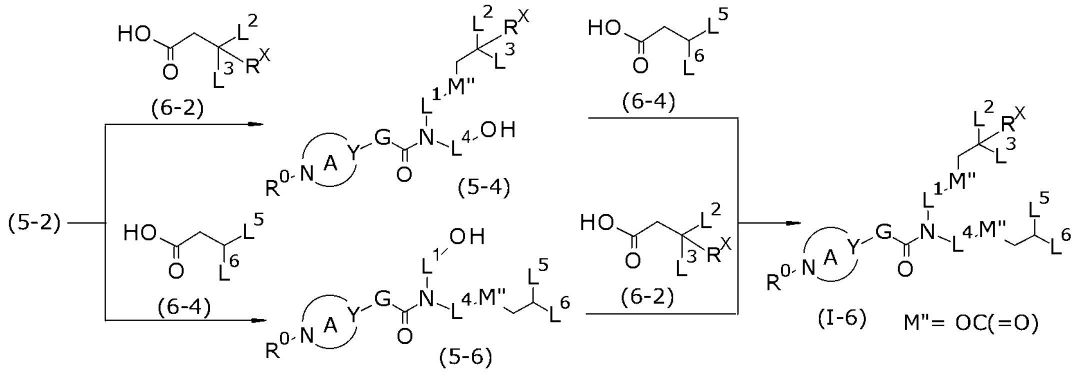

(5-2) (6-2) (6-4) (5-4) (5-6) (6-4) (6-2) (I-6) M"= OC(=O)

Moreover, a method in which a half ester in which only one is esterified is isolated in the esterification in the last step to synthesize the final product is also used. The compound (5-1) can be esterified with the compound (6-1) or a compound (6-3) to obtain a compound (5-3) or a compound (5-5) as a half ester, and by reacting the compound (6-3) or the compound (6-1), a compound (I-5) of the invention can be produced.

Similarly, the compound (5-2) can be esterified with a compound (6-2) or a compound (6-4) to obtain a compound (5-4) or a compound (5-6) as a half ester, and by reacting the compound (6-4) or the compound (6-2), a compound (I-6) of the invention can be produced.

(Raw Material Production Method 1)

[Chem. 9]

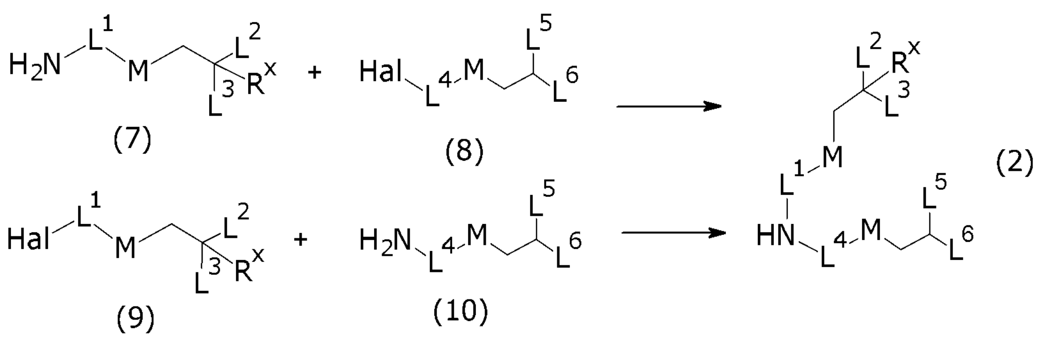

(7) (8) (2) (9) (10)

(In the formulae, Hal represents a halogen.)

The compound (2) can be produced by a reaction of a compound (7) and a compound (8). Here, a methanesulfonyloxy group or the like can be used as a leaving group instead of the halogen.

In this reaction, the compound (7) and the compound (8) are used in an equal amount or with either thereof in an excess amount, and a mixture thereof is stirred in a solvent inert to the reaction or without a solvent, from under cooling to

under reflux with heat, preferably at 0°C to 80°C, generally for 0.1 hours to five days. Examples of the solvent include an aromatic hydrocarbon such as toluene and xylene, an ether such as CPME, diethyl ether, THF and 1,4-dioxane, a halogenated hydrocarbon such as DCM and 1,2-dichloroethane, DMAc, NMP, DMF, DMSO, MeCN, ethyl acetate and a mixture thereof. The reaction sometimes further progresses in the presence of an organic base such as $Et_3N$, DIPEA and N-methylmorpholine or an inorganic base such as KI, NaI, TBAI, $K_2CO_3$, $Na_2CO_3$ and KOH.

In this regard, the compound (2) can be produced similarly also by reacting a compound (9) and a compound (10).

[Reference] S. R. Sandler and W. Karo, "Organic Functional Group Preparations", 2nd edition, Vol. 1, Academic Press Inc., 1991 and "Jikken Kagaku Koza (Courses in Experimental Chemistry) (5th edition)" edited by The Chemical Society of Japan, Vol. 14 (2005) (Maruzen)

(Raw Material Production Method 2)

[Chem. 10]

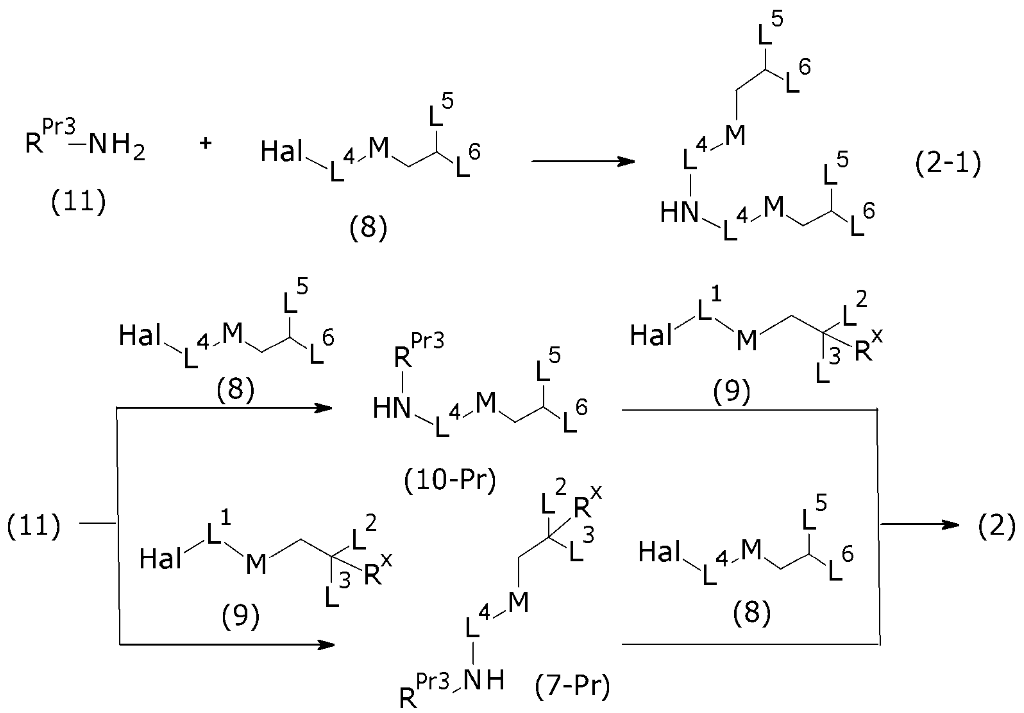

(In the formula, $R^{Pr3}$ is a protective group of amine.)

A compound (2-1) is a compound such that in the partial structure of the compound of the invention in the compound (2), $L^2$ and $L^3$ are $L^5$ and $L^6$, respectively, $R^X$ is H and the two side chain moieties are both $-L^4-M-CH_2CH(L^5)L^6$. The compound (2-1) can be produced by a similar method to the Raw Material Production Method 1. The compound (2-1) can be produced by reacting a compound (8) with a compound (11), and then deprotecting.

The compound (2) can be produced by monoalkylating a compound (11) as a raw material with a compound (8) or a compound (9) and then further reacting with the compound (9) or the compound (8), and then deprotecting.

(Raw Material Production Method 3)

[Chem. 11]

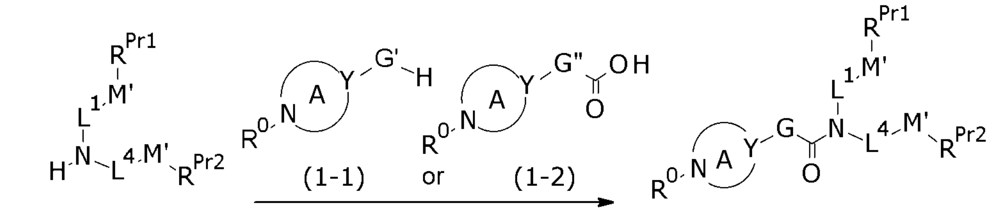


(13-1) M'= C(=O)O (4-1) M'= C(=O)O

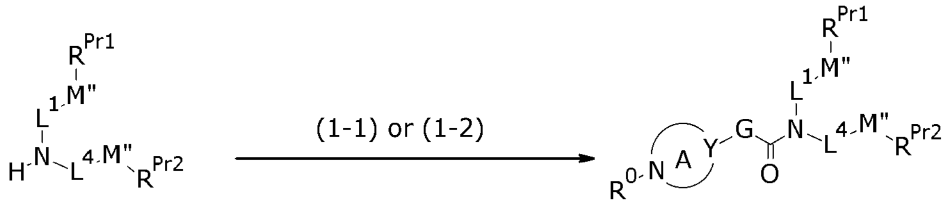


(13-2) M"= OC(=O) (4-2) M"= OC(=O)

A compound (4-1) and a compound (4-2) can be produced from a compound (13-1) and a compound (13-2), respectively, as a raw material. A compound (4-1) in which G is G' can be produced with the compound (1-1). A compound (4-1) in which G is G" can be produced with the compound (1-2). For the reaction conditions, those in the First Production Method or those in the Second Production Method can be used.

The compound (13-1) and the compound (13-2) can be produced with any of the methods described in the Raw Material Production Methods 1 and 2.

(Raw Material Production Method 4)

[Chem. 12]

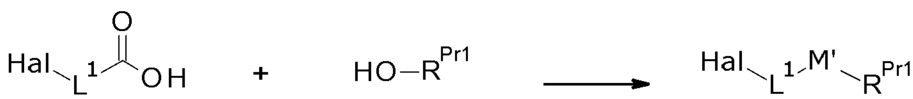

(14-1) (15-1) (8-1)

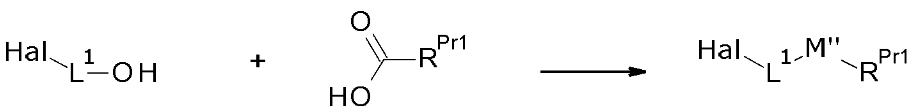

(14-2) (15-2) (8-2)

A raw material compounds (8-1) and (8-2) can be respectively produced with a compound (14-1) and a compound (14-2) as a raw material by esterification of a compound (15-1) and a compound (15-2).

Similarly, a compound (12-1) and a compound (12-2) in each of which $R^{Pr1}$ is $R^{Pr2}$ and $L^1$ is $L^4$ can be produced by using a compound in which $R^{Pr1}$ is $R^{Pr2}$ and $L^1$ is $L^4$ in the above reaction formulae. The compound (8) and the compound (9) can also be produced in a similar manner. The compound (7) and the compound (10) can also be produced in a similar manner by using a compound with a protective group added to $NH_2$ as a raw material.

The compound of the formula (I) or a salt thereof is isolated and purified as a free compound or a salt, a hydrate, a solvate or a crystal polymorphous substance thereof. The compound of the formula (I) or a salt thereof can also be produced by subjecting to a salt formation reaction which is an ordinary method. The isolation and purification are performed by applying a general operation, such as extraction, fractional crystallization and various types of fraction chromatography (silica gel chromatography (also called silica gel column chromatography below) and the like).

Various isomers can be produced by selecting an appropriate raw material compound or can be separated using a difference in physiochemical properties between the isomers. For example, an optical isomer can be obtained by a general optical resolution method of a racemate (for example, fractional crystallization for inducing to a diastereomer salt with an optically active base or acid, chromatography and the like using a chiral column or the like) and can also be

produced from an appropriate optically active raw material compound.

5. Production Method of Lipid Nanoparticles

The lipid nanoparticles can be produced by adding components such as the compound of the formula (I) or a salt thereof obtained by the production method described above, a neutral lipid, a PEGylated lipid and a nucleic acid and dispersing in a medium. For example, the compound of the formula (I) or a salt thereof, DSPC, a sterol, a PEGylated lipid and the like are dissolved in a solvent to obtain an oil phase, and the oil phase is mixed with or suspended in an aqueous phase such as a buffer solution containing a nucleic acid. Then, the solvent in the mixture solution is removed by a method such as dialysis and ultrafiltration, and lipid nanoparticles can be thus obtained. Moreover, a pharmaceutical composition can be produced by adding a pharmaceutical additive such as the excipient described above to the lipid nanoparticles and then stirring.

6. Production Method of Pharmaceutical Composition

The pharmaceutical composition can be prepared by a generally used method using a pharmaceutical additive (an excipient or the like) generally used in the field, namely, a pharmaceutical additive for a drug or the like. Regarding the pharmaceutical additive, although the components contained in the pharmaceutical composition are not limited, a pharmaceutical additive, such as a preservative, a stabilizer, an antioxidant and an antiseptic, may be contained in addition to the excipient. Another pharmaceutical additive may be added to the pharmaceutical composition.

Moreover, a pharmaceutically acceptable medium may be added to the pharmaceutical composition.

For example, the pharmaceutical composition is a solution which is refrigerated or frozen for storage and/or transport (for example, refrigerated for storage and/or transport for storing at a temperature of 4°C or lower, such as a temperature of about -150°C to about 0°C or about -80°C to about -20°C). In an aspect, the solution is PBS.

The injection preferably contains a sterile aqueous or nonaqueous solution, suspension or emulsion. Examples of the aqueous solvent include distilled water for injection or physiological saline. An example of the nonaqueous solvent is an alcohol such as ethanol. Such a composition may further contain an isotonizing agent, an antiseptic, a wetting agent, an emulsifier, a dispersant, a stabilizer or a dissolution aid. These are sterilized, for example, by filtration through a bacteria keeping filter, incorporation of a microbicide or irradiation. In addition, such a composition can be produced as a sterile solid composition which is dissolved or suspended in sterile water or a sterile solvent for injection before use.

The transmucosal agent such as an inhalant or a transnasal agent is used in a liquid form and can be produced according to a conventionally known method. For example, a known pharmaceutical additive and in addition, a pH modifier, an antiseptic, a surfactant, a lubricant, a stabilizer, a thickener or the like may be appropriately added. The administration can be performed using an appropriate device for inhalation or insufflation. For example, the agent can be administered using a known device such as a metering and administering inhalation device or an atomizer, as a compound alone or a powder of a formulated mixture, or as a solution or a suspension in combination with a pharmaceutically acceptable pharmaceutical additive. A dry powder inhaler or the like may be for a single administration or multiple administrations. The agent may be used in a form of a pressurized aerosol spray or the like using an appropriate ejection agent, for example, a suitable gas, such as a chlorofluoroalkane or carbon dioxide.

7. Production Method of Nucleic Acid

The nucleic acid, such as mRNA for example, can be produced using a known technique in the field. Examples are shown below, but the production is not limited to the examples. mRNA can be produced using a linear plasmid as a DNA template by *in vitro* transcription (IVT). As the production methods, (i) the post-transcriptional capping method (in the process, plasmid production, *in vitro* transcription and 5' capping are conducted in this order), (ii) the co-transcriptional capping method (in the process, plasmid production and then *in vitro* transcription together with 5' capping are conducted) and the like are known. In the co-transcriptional capping method, Cap-0 is added to the 5' terminal of the RNA when the anti-reverse cap analog (ARCA) technique is used, and Cap-1 can be added to the 5' terminal of the RNA when the CleanCap (registered trademark, TriLink) capping technique is used.

(*in vitro* Transcription)

In IVT, a transcription buffer solution, nucleoside triphosphates (NTPs), an RNase inhibitor and a polymerase (example: T7 RNA polymerase) can be generally used for the reaction. The NTPs may be of natural type or unnatural type (modified type).

(Capping)

The capping can be conducted according to a method using vaccinia capping enzyme, 2'O-methyltransferase, CleanCap or the like, or the likes. When the sequence transcribed by IVT does not contain any poly(A) chain, poly(A) addition reaction can also be conducted. Here, regarding the sequence, when the capping method using CleanCap (registered trademark) Reagent AG (TriLink BioTechnologies) is used, the 5' terminal sequence suitable for the capping method using CleanCap (registered trademark) Reagent AG (TriLink BioTechnologies) is, for example, AGG (position 1 to position 3 of SEQ ID NO: 4).

(Plasmid Production)

The linear plasmid can be produced by the following processes. A plasmid obtained by inserting any UTR and any base sequence (example: a base sequence encoding NeuroD1 protein) into a plasmid (example: a high-copy plasmid for *Escherichia coli* (pUC18, pCU18 or the like)) can be produced and can be amplified using *Escherichia coli* or the like. After purification, the linear plasmid can be produced from the plasmid using a restriction enzyme and a buffer solution. After purification, the linear plasmid can be used directly as a DNA template. Alternatively, the linear plasmid can be used as a DNA template after subjecting to polymerase chain reaction (PCR).

(Purification)

The mRNA and the intermediates in the production processes can be purified by a known method in the field. Examples are shown below, but the purification is not limited to the examples. The DNA template can be removed with deoxyribonuclease I (DNase I). The transcribed RNA can be purified using a silica gel column or the like. The RNA containing a poly(A) chain can be purified with an Oligo dT column. The linear plasmid can be purified using PureLink (registered trademark) (Thermo Fisher Scientific) or the like.

Examples

The pharmacological activities of lipid nanoparticles containing the compounds of the formula (I) or salts thereof as a component were examined by the following tests. In Test Examples below in this specification, the abbreviations below are sometimes used.

(Abbreviations)

B27: B27 serum-free supplement, BDNF: brain-derived neurotrophic factor, DMEM: Dulbecco's modified Eagle medium, DSPC: 1,2-distearoyl-sn-glycero-3-phosphocholine, FBS: fetal bovine serum, FLuc: luciferase (firefly), FLuc nucleic acid lipid nanoparticles: lipid nanoparticles encapsulating FLuc mRNA, LNP: lipid nanoparticles, mRNA: messenger ribonucleic acid, ND1: :NeuroD1, ND1 nucleic acid lipid nanoparticles: lipid nanoparticles encapsulating NeuroD1 mRNA, PBS: phosphate-buffered saline, RLA: relative luciferase activity, and RLU: relative light unit.

(Evaluation of Introduction of Lipid Nanoparticles Using Mouse Primary Astrocytes)

Regarding the compounds of the formula (I) or salts thereof composing lipid nanoparticles, the efficiencies of introduction of a nucleic acid to mouse primary astrocytes by lipid nanoparticles were evaluated. As the mRNA, FLuc mRNA (TriLink BioTechnologies) was used, and nucleic acid lipid nanoparticles were produced using NanoAssesmblr (Precision NanoSystems). The efficiencies of introduction of the test drugs (nucleic acid lipid nanoparticles) were calculated by evaluating the activity of luciferase encoded by the mRNA.

(Acquisition of Mouse Primary Astrocytes)

Mouse astrocytes were acquired in accordance with Lynette C. Foo. (2013). Purification of Rat and Mouse Astrocytes by Immunopanning. Cold Spring Harbor Protocols, 5, 421-432. Here, the cells were recovered in 5% $CO_2$. As the mouse, newborn P1-P10 of C57BL/6J (Charles River Laboratories Japan, Inc.) was used.

Test Example 1: *in vitro* Luciferase Assay

(Test Method)

The mouse astrocytes acquired by the above method were seeded at 2000 cells/well in a polyD-Lysine coated 96-well plate (IWAKI) and cultured in 5% $CO_2$ at 37°C overnight. As the medium, 100 μL/well of DMEM/F12 (Gibco) containing 2% B-27 (registered trademark) supplement (Thermo Fisher Scientific), 10% FBS (Hyclone) and 1% Penicillin-Streptomycin (PS) (Thermo Fisher Scientific) was used. On the next day, 100 μL of PBS and the test drugs (FLuc nucleic acid lipid nanoparticles) diluted with the medium were added to be a final concentration of 0.8 μg/mL, and the cells were cultured at 200 μL/well overnight. After removing all the medium on the next day, ONE-Glo Luciferase assay system (Promega) was diluted two-fold with PBS, and 100 μL thereof was added to each well. After mixing for a minute, 80 μL thereof were transferred to a 96-well white plate (Nunc), and the luminescence thereof was detected with Envision (Perkin Elmer). RLU (Relative Light Unit) was used as the indicator of activity.

(Evaluation of Expression)

As a result of the above test, luciferase activity was observed in the nucleic acid lipid nanoparticles containing typical Example compounds of the invention as a component. This suggests that the lipid nanoparticles encapsulating FLuc mRNA were introduced into the cells and translated, and thus luciferase protein was expressed. The results of the nucleic acid lipid nanoparticles containing the Example compounds as a component are shown in the table below. Here, NUM indicates lipid nanoparticles containing an Example compound of the compounds of the formula (I) or salts thereof as a component. ExA-LB (A and B are numbers) indicates lipid nanoparticles containing the Example compound ExA of the compounds of the formula (I) or salts thereof and having the composition LB. L0 indicates lipid nanoparticles containing Ex1, DSPC, cholesterol and DMG-PEG2000 (mole ratio of 50/10/38.5/1.5) as components. For example, Ex1-L0 to Ex5-L0 indicate the lipid nanoparticles containing the Example compounds Ex1, Ex2, Ex3, Ex4 and Ex5, respectively, of the compounds of the formula (I) or salts thereof, as a component and having the lipid composition L0. The average of RLU for expression of nucleic acid lipid nanoparticles was calculated for each group, and relative values as the value for Ex1-L1 was assumed as 1 are shown as RLA (Relative Luciferase Activity) in Table xx below. For Ex1-L5, Ex1-L6, Ex1-L7, Ex1-L9, Ex1-L11, Ex1-L13, Ex1-L14, Ex1-L15, Ex1-L22, Ex1-L23, Ex1-L24, Ex1-L25, Ex1-L32, Ex1-L33, Ex1-L34, Ex1-L35 and Ex1-L36 of Ex1-L1 to Ex1-L36, the relative values as the value for Ex1-L1* was assumed as 1 are shown as RLA. Ex1-L1 to Ex1-L36 shown in NUM in the table below indicate Ex1-L1-FLuc mRNA to Ex1-L36-FLuc mRNA. This means that Ex1-L1 to Ex1-L36 are each lipid nanoparticles encapsulating FLuc mRNA, having a lipid composition shown below and containing the Example compound Ex1.

[Table 1]

| NUM | RLU |
|---|---|
| Ex1-L0 | 5470267 |
| Ex2-L0 | 7063200 |
| Ex3-L0 | 13879867 |
| Ex4-L0 | 6695600 |
| Ex5-L0 | 16398000 |
| Ex6-L0 | 12484533 |
| Ex7-L0 | 12250267 |
| Ex8-L0 | 12144800 |
| Ex9-L0 | 7345200 |
| Ex10-L0 | 11898400 |
| Ex11-L0 | 7790133 |
| Ex12-L0 | 5474133 |
| Ex13-L0 | 17351467 |
| Ex14-L0 | 746933 |
| Ex15-L0 | 3035067 |
| Ex16-L0 | 3404667 |
| Ex17-L0 | 2128933 |

(continued)

| NUM | RLU |
|---|---|
| Ex18-L0 | 1086267 |
| Ex19-L0 | 2606800 |
| Ex20-L0 | 1030267 |
| Ex21-L0 | 635600 |
| Ex22-L0 | 2087200 |
| Ex23-L0 | 1781867 |
| Ex24-L0 | 1956133 |
| Ex25-L0 | 4246800 |
| Ex26-L0 | 2745733 |
| Ex27-L0 | 9528000 |
| Ex28-L0 | 3058400 |
| Ex29-L0 | 4522400 |
| Ex30-L0 | 2129600 |
| Ex31-L0 | 4159467 |
| Ex32-L0 | 7754533 |
| Ex33-L0 | 3068533 |
| Ex34-L0 | 1601733 |
| Ex35-L0 | 3531333 |
| Ex36-L0 | 7583600 |
| Ex37-L0 | 7424000 |
| Ex38-L0 | 5977600 |
| Ex39-L0 | 1086267 |
| Ex40-L0 | 5026267 |
| Ex41-L0 | 1576667 |
| Ex42-L0 | 1549867 |
| Ex43-L0 | 5709467 |
| Ex44-L0 | 3650800 |
| Ex45-L0 | 5221200 |
| Ex46-L0 | 2424000 |
| Ex47-L0 | 3274933 |
| Ex48-L0 | 4776400 |
| Ex51-L0 | 2556933 |
| Ex52-L0 | 5584000 |
| Ex56-L0 | 1811067 |
| Ex57-L0 | 2395600 |
| Ex58-L0 | 2083867 |
| Ex59-L0 | 8100533 |
| Ex60-L0 | 12113467 |
| Ex63-L0 | 7663733 |

(continued)

| NUM | RLU |
|---|---|
| Ex64-L0 | 11945733 |
| Ex68-L0 | 2069867 |
| Ex70-L0 | 1326000 |
| Ex71-L0 | 990800 |
| Ex72-L0 | 2870400 |
| Ex73-L0 | 1108133 |
| Ex74-L0 | 1476267 |
| Ex75-L0 | 2713067 |
| Ex76-L0 | 2386933 |
| Ex78-L0 | 24995200 |
| Ex79-L0 | 8101733 |
| Ex80-L0 | 7523200 |
| Ex81-L0 | 9238533 |
| Ex82-L0 | 33014667 |
| Ex83-L0 | 3457200 |
| Ex84-L0 | 4166000 |
| Ex85-L0 | 16961600 |
| Ex86-L0 | 4478267 |
| Ex87-L0 | 3752933 |
| Ex88-L0 | 6170667 |
| Ex89-L0 | 13646400 |
| Ex90-L0 | 11357067 |
| Ex91-L0 | 6592267 |
| Ex92-L0 | 7308133 |
| Ex93-L0 | 12747600 |
| Ex94-L0 | 5726267 |
| Ex95-L0 | 4854667 |
| Ex96-1L0 | 5326267 |
| Ex97-L0 | 3856933 |

[Table 2]

| NUM | RLA |
|---|---|
| Ex1-L1 | 1.00 |
| Ex1-L1* | 1.00 |
| Ex1-L2 | 0.45 |
| Ex1-L3 | 0.24 |
| Ex1-L4 | 0.11 |
| Ex1-L5 | 0.30 |
| Ex1-L6 | 0.14 |

(continued)

| NUM | RLA |
|---|---|
| Ex1-L7 | 0.21 |
| Ex1-L8 | 0.47 |
| Ex1-L9 | 0.47 |
| Ex1-L10 | 0.61 |
| Ex1-L11 | 0.35 |
| Ex1-L12 | 0.33 |
| Ex1-L13 | 0.24 |
| Ex1-L14 | 0.48 |
| Ex1-L15 | 0.27 |
| Ex1-L16 | 0.14 |
| Ex1-L17 | 0.60 |
| Ex1-L18 | 0.51 |
| Ex1-L19 | 0.48 |
| Ex1-L20 | 0.68 |
| Ex1-L21 | 0.64 |
| Ex1-L22 | 0.80 |
| Ex1-L23 | 0.11 |
| Ex1-L24 | 0.05 |
| Ex1-L25 | 0.03 |
| Ex1-L26 | 0.45 |
| Ex1-L27 | 0.52 |
| Ex1-L28 | 0.38 |
| Ex1-L29 | 0.06 |
| Ex1-L30 | 0.25 |
| Ex1-L31 | 0.60 |
| Ex1-L32 | 0.57 |
| Ex1-L33 | 0.25 |
| Ex1-L34 | 0.27 |
| Ex1-L35 | 0.13 |
| Ex1-L36 | 0.17 |

Test Example 2 *in vivo* Luciferase Assay

(Test Method)

The efficiencies of introduction of nucleic acid lipid nanoparticles in the brain were evaluated using IVIS spectrum (PerkinElmer). Nucleic acid lipid nanoparticles were administered into the brain parenchyma of 6-14-week-old BALB/c mice (Charles River Laboratories Japan, Inc., n=3) under isoflurane (isoflurane inhalation anesthetic solution "VTRS"; Mylan N.V., the same applies below) anesthesia. For the administration into the brain parenchyma, the coordinates for administration were determined at 0.0 mm anteroposterior from the bregma, 2.0 mm to the left and 2.5 mm deep based on the mouse brain atlas (Academic press). Under isoflurane anesthesia, after shaving with clippers, the skull was fixed with a brain stereotaxic apparatus (KOPF), and a hole with a diameter of around 1 mm was made with a drill (Foredom). An Ito syringe (Ito Corporation) was filled with a test drug (FLuc nucleic acid lipid nanoparticles) of 0.3 mg/mL, and 1 μL thereof

was administered to the set coordinates using an injection pump (Narishige Group) at a flow rate of 0.2 μL/min. After leaving still for a minute after the administration, the needle was pulled out slowly, and the scalp was stitched together. After a day, 250 μL/animal of 15 mg/mL luciferin (Promega) was intraperitoneally administered under isoflurane anesthesia, and the luminescence was observed after 20 minutes using IVIS spectrum. The obtained luminescence values were converted to digits with photons/sec unit with Living image software and used for the analysis.

(Evaluation of Expression)

As a result of the above test, luciferase activity was observed in the nucleic acid lipid nanoparticles containing typical Example compounds of the invention as a component (Ex1-L0, Ex56-L0, Ex72-L0-Fluc mRNA). This suggests that the lipid nanoparticles encapsulating FLuc mRNA were introduced into the cells in the brain and translated, and thus luciferase protein was expressed. The data of expression of the nucleic acid lipid nanoparticles are shown in the table below. In the table, the average of the three individuals of Ex1-L0-Fluc mRNA evaluated is shown. Ex1-L0 shown in NUM in the table below indicates Ex1-L0-FLuc mRNA. This means that Ex1-L0 is lipid nanoparticles encapsulating FLuc mRNA, having the same lipid composition as that of Ex1-L0 and containing the Example compound Ex1.

[Table 3]

| NUM | Bioluminescence imaging (photons/sec) |
|---|---|
| Ex1-L0 | 142233000 |
| Ex56-L0 | 119667000 |
| Ex72-L0 | 86243300 |

Test Example 3: *in vitro* Conversion of Rat Primary Astrocytes into Neurons

(Addition of Nucleic Acid Lipid Nanoparticles to Rat Primary Astrocytes)

Rat primary astrocytes were acquired in accordance with Lynette C.Foo. (2013). Purification of Rat and Mouse Astrocytes by Immunopanning. Cold Spring Harbor Protocols, 5, 421-432 and used. Here, the cells were recovered in 5% $CO_2$. As the rats, newborn Wistar rats (CLEA Japan, Inc., P1-P10) were used. The acquired rat primary astrocytes were seeded at 40,000 cells/well in a poly-D-lysine coated 96-well plate (Corning, 356640) and cultured in 5% $CO_2$ at 37°C overnight. For the culture, 200 μL/well of DMEM/F-12 medium (Thermo Fisher Scientific, 11320-033) containing 2% B-27 (registered trademark) supplement (Thermo Fisher Scientific, A1895601), 10% fetal bovine serum (FBS, Cytiva, SH30070.03) and 1% penicillin-streptomycin (PS) (Thermo Fisher Scientific, 15070063) was used. The DMEM/F12 medium containing B-27 (registered trademark) supplement, FBS and PS was used as the culture medium also below. On the next day, PBS and ND1 nucleic acid lipid nanoparticles (Ex1-L0-ND1 mRNA) diluted with the culture medium were added to be a final concentration of 0.5 μg/mL, and the cells were cultured for 24 hours. The culture medium was added to the control.

The culture medium was removed 24 hours after the addition of the ND1 nucleic acid lipid nanoparticles and changed to a transdifferentiation medium, and the medium was changed every two to three days. The transdifferentiation medium was DMEM/F12 medium containing 2% B-27 (registered trademark) supplement, 1% GlutaMAX (trademark) supplement (Thermo Fisher Scientific, 35050-061), 1% PS and 0.02% BDNF solution, and 200 μL/well of the medium was added. The BDNF solution was prepared by dissolving BDNF powder (PeproTech, 450-02) in pure water to be 100 μg/mL.

(Observation of Expression of NeuroD1 Protein and DCX Protein by Immunoblotting Method)

The culture medium was removed before the addition of the nucleic acid lipid nanoparticles (day 0) and eight hours and on day 1, day 2, day 4 and day 6 after the addition, and the cells were washed with PBS and lysed using RIPA Buffer (SIGMA-Aldrich, R0278-50ML). Before changing the medium 24 hours after the addition of the nucleic acid lipid nanoparticles, cell lysis on day 1 after the addition of the nucleic acid lipid nanoparticles was conducted. After protein quantification by the Lowry method, 5 μg of the protein was analyzed by electrophoresis, and the expression of NeuroD1 protein was detected by the immunoblotting method using an anti-NeuroD1 antibody (Santa Cruz Biotechnology, sc46684). Moreover, the expression of DCX protein was detected by the immunoblotting method using an anti-DCX antibody (Cell Signaling Technology, 4604S).

As a result, it was found that the expression level of NeuroD1 protein became the maximum eight hours after the addition of the nucleic acid lipid nanoparticles and that the expression decreased on day 1 after the addition. The results show that the nucleic acid lipid nanoparticles were introduced into the rat primary astrocytes and that NeuroD1 protein was

expressed transiently in the rat primary astrocytes. It was also found that the expression of DCX protein increased from day 1 after the addition of the nucleic acid lipid nanoparticles and that the expression increased also on day 6 after the addition of the nucleic acid lipid nanoparticles. The results show that NeuroD1 mRNA could be delivered to the rat primary astrocytes using LNPs containing Ex1-L0 and that the rat primary astrocytes were converted to neurons by the NeuroD1 mRNA because NeuroD1 protein was expressed from the NeuroD1 mRNA in the rat primary astrocytes and exhibited the functions.

Test Example 4: Action of ND1 Nucleic Acid Lipid Nanoparticles on Cynomolgus Monkey Cerebral Infarction Model

(Production of Cynomolgus Monkey Cerebral Infarction Model)

Four individuals of male cynomolgus monkey (three years old or older, Shin Nippon Biomedical Laboratories, Ltd.) were subjected to induction anesthesia by intramuscular injection (i.m.) using ketamine hydrochloride (Ketalar for muscular injection 500 mg; Daiichi Sankyo Propharma Co., Ltd.) in an amount of about 10 mg/kg body weight. The cynomolgus monkeys under anesthesia were intratracheally intubated and subjected to maintenance anesthesia by inhalation of isoflurane (isoflurane inhalation anesthetic solution "VTRS"; Mylan N.V.) through the tracheal tube under mechanical ventilation or with spontaneous breathing. After the hair on the head of each cynomolgus monkey under anesthesia was shaved with clippers, the cynomolgus monkey was fixed with a brain stereotaxic apparatus (Narishige Group). Then, with consideration for bleeding, the skin in the head of the cynomolgus monkey was cut open, and the bregma, which is the intersection of the sagittal suture and the coronal suture in the front of the skull surface, was observed. The infusion sites are as shown below based on the bregma (B: 0 mm, ML coordinate: 0 mm (median)).

B: the distance in the anterior-posterior axial direction based on the bregma (a plus value indicates the distance in the front)
L: the distance to the left based on the ML coordinate
D: the depth in the brain from the dura mater in the vertical downward direction

An endothelin-1 (Peptide Institute, Inc. code 4198-v) solution was infused to the four individuals of male cynomolgus monkey. Specifically, in the skull of each cynomolgus monkey, holes with a diameter of around 1 mm were made with a drill at locations (i) B: 2.0 mm, L: 8.0 mm, (ii) B: 5.0 mm, L: 8.0 mm, (iii) B: 9.0 mm, L: 5.0 mm and (iv) B: 9.0 mm, L: 12.0 mm (four locations in total) based on the bregma for the infusion sites. A micro syringe (HAMILTON) connected to a microinjector (Narishige Group) was inserted from each hole and placed in such a manner that the needle tip was at the depth (D) 19.0 mm ((i) and (ii) above), the depth (D) 13.0 mm ((iii) above) or the depth (D) 15.0 mm ((iv) above) from the dura mater. Endothelin-1 was infused to the four sites in total corresponding to the locations for each individual using the micro syringes. Endothelin-1 was infused at a concentration of 2.0 μg/μL (diluted with 1% acetic acid (prepared by diluting acetic acid (Wako, 017-00256) with Milli-Q (registered trademark) water)) in an amount of 30 μL/site at an infusion rate of 1.5 μL/min. After the completion of the infusion, the syringes were left still for about 20 minutes to prevent any liquid leakage. The infusion sites (i) to (iv) above target (i) the ventral lateral thalamic nucleus and the ventral posterior thalamic nucleus, (ii) the globus pallidus, (iii) the caudate nucleus and (iv) the putamen, respectively. By infusing endothelin to the infusion sites as described above, damage was caused to the internal capsule in addition to the targeted sites. In this manner, a cerebral infarction model having basal ganglia disorder, thalamus disorder and internal capsule disorder was produced. This model can evaluate penetrating branch infarction, cerebral infarction having brain damage in a penetrating branch territory, subacute-phase to chronic-phase cerebral infarction and cerebral infarction having motor dysfunction with high severity.

(Administration of ND1 Nucleic Acid Lipid Nanoparticles to Model)

On day 21 after the endothelin-1 infusion treatment (chronic phase), the four individuals of the cerebral infarction model produced by the above method were divided into two groups (a control group with n=2 and an ND1 nucleic acid lipid nanoparticle group with n=2) in such a manner that there was no difference between the groups regarding the mRS for monkeys (see Table 3 below). Here, Ex1-L0-eGFP mRNA was administered to the control group (two individuals), and Ex1-L0-ND1 mRNA was administered to the ND1 nucleic acid lipid nanoparticle group (two individuals).

For medical examination of human stroke, the mRS (for human clinical uses) described in Table 3 is widely used as an indicator of disability. The mRS (for monkeys) is an indicator in which the mRS (for human clinical uses) is modified for monkeys. As an indicator used for scoring monkeys' motor dysfunction, the NonHuman Primate Stroke Scale (NHPSS), which is an evaluation scale obtained by adopting the National Institutes of Health Stroke Scale (NIHSS), which is an evaluation scale for stroke neurological severity of humans, to nonhuman primate models, is known. The mRS (for monkeys) can evaluate the durability of motor dysfunction like the NHPSS, and the mRS can be used for evaluating the

durability of motor dysfunction of this model.

[Table 4]

| Score | mRS (for human clinical practice) | mRS (for monkeys) |
|---|---|---|
| 0 | No symptoms at all | No symptoms at all |
| 1 | No significant disability: despite symptoms, able to carry out all usual duties and activities | Mild paralysis[1] of a forelimb or hindlimb on impaired side |
| 2 | Slight disability: unable to perform all previous activities but able to look after own affairs without assistance | Slight decline in locomotor activity[3], or mild paralysis of a forelimb and hindlimb on impaired side |
| 3 | Moderate disability: requiring some help but able to walk without assistance | (i) Decline in locomotor activity[4], or (ii) seated posture (including incomplete standing-up motion), or (iii) paralysis[2] of a forelimb and hindlimb on impaired side. No decreased level of consciousness[5] |
| 4 | Moderately severe disability: unable to walk without assistance and unable to attend to own bodily needs without assistance | (i) Decline in locomotor activity and (ii) seated posture/recumbent posture[6] (including incomplete standing-up motion), and (iii) paralysis of a forelimb and hindlimb on impaired side or a slightly decreased level of consciousness[7] |
| 5 | Severe disability: bedridden, incontinent and requiring constant nursing care and attention | Recumbent posture (including incomplete standing-up motion), and paralysis of a forelimb and hindlimb on impaired side, and a decreased level of consciousness[8] |
| 6 | Death | Death |

[1]Mild paralysis: no significant paralysis is found, but a slight sign of paralysis is found.
[2]Paralysis: significant paralysis is found.
[3]Slight decline in locomotor activity: no significant decline in locomotor activity is found, but a slight sign of decline in locomotor activity is found.
[4]Decline in locomotor activity: significant decline in locomotor activity is found.
[5]Level of consciousness (monkeys): determined on the level of response to human.
[6]Seated posture/recumbent posture: at least one of seated posture and recumbent posture.
[7]Slightly decreased level of consciousness: no significantly decreased level of consciousness is found, but a slight sign of a decreased level of consciousness is found.
[8]Decreased level of consciousness: a significantly decreased level of consciousness is found.

The cerebral infarction model individuals of the groups were subjected to induction anesthesia through i.m. using ketamine hydrochloride in an amount of about 10 mg/kg body weight and maintenance anesthesia through isoflurane inhalation. Under continuous anesthesia, the hair on the head was shaved with clippers, and then each model individual was fixed with a brain stereotaxic apparatus. Next, the operation part was disinfected, and the skin of the head was cut open to expose the bregma with consideration for bleeding.

For each of the cerebral infarction model individuals of the groups, a micro syringe was placed in each of the four holes in total through which endothelin-1 was infused when the cerebral infarction model was produced in such a manner that the needle tip was at the same depth (D) as that for the infusion of endothelin-1. Ex1-L0-ND1 mRNA was administered to two individuals, and Ex1-L0-eGFP mRNA was administered to two individuals. The nucleic acid lipid nanoparticles were prepared at 0.3 mg/mL with PBS and administered at 30 μL/site at an infusion rate of 1.5 μL/min. After the completion of the administration, the syringes were left still for about 20 minutes.

(Observation of Motor Function)

The individuals of the ND1 nucleic acid lipid nanoparticle group and the control group in (Administration of ND1 Nucleic Acid Lipid Nanoparticles to Model) above were observed before the endothelin-1 infusion treatment (day 0) and on days 1, 7, 14, 21, 22, 28, 42, 56, 70 and 84 after the endothelin-1 infusion treatment based on the mRS (Table 3). Here, the

behaviors were evaluated every two weeks on and after day 28 after the endothelin-1 infusion treatment, and as the evaluation of the behaviors of the control group on day 70 after the endothelin-1 infusion (day 49 after the administration of the nucleic acid lipid nanoparticles), the individuals of the control group were evaluated on day 69 or 70 after the endothelin-1 infusion (day 48 or 49 after the administration of the nucleic acid lipid nanoparticles). The results were used in this Example as the results on day 70 after the endothelin-1 infusion (day 49 after the administration of the nucleic acid lipid nanoparticles) of the control group.

As a result, in both of the ND1 nucleic acid lipid nanoparticle group and the control group, there was no difference in the motor function on day 1 after the administration, and similar motor dysfunction to that before the administration of the nucleic acid lipid nanoparticles was observed. In the control group, however, the motor function was not improved even on day 84 after the endothelin-1 infusion (day 63 after the administration of the nucleic acid lipid nanoparticles), while in the ND1 nucleic acid lipid nanoparticle group, the motor function was recovered on and after day 56 after the endothelin-1 infusion (day 35 after the administration of the nucleic acid lipid nanoparticles), and significant recovery of the motor function was observed on day 84 after the endothelin-1 infusion (day 63 after the administration of the nucleic acid lipid nanoparticles) (Figure 1). This shows that administration of the ND1 nucleic acid lipid nanoparticles can recover motor dysfunction following brain damage.

From the above results, administration of ND1 nucleic acid lipid nanoparticles can treat cerebral infarction (especially, penetrating branch infarction, Cerebral infarction having brain damage in a penetrating branch territory, subacute-phase to chronic-phase cerebral infarction and cerebral infarction having motor dysfunction with high severity).

From the results of the above Test Examples, using the compound of the formula (I) or a salt thereof as a cationic lipid, lipid nanoparticles which are suitable for a pharmaceutical composition for preventing and/or treating an astrocyte-related disease can be produced.

The production method of the compound of the formula (I) will be explained in further detail below based on Examples. In this regard, the invention is not limited to the compounds described in the following Examples. The production methods of raw material compounds are also shown in Production Examples. The production method of the compound of the formula (I) is not limited only to the production methods of specific Examples described below, and the compound of the formula (I) can also be produced by a combination of the production methods or a method that is obvious to one skilled in the art.

The abbreviations below are sometimes used in this specification, Examples, Production Examples and the Tables.

DCM: dichloromethane, DMAP: 4-(dimethylamino)pyridine, DIPEA: N,N-diisopropylethylamine, DMF: N,N-dimethylformamide, DMAc: N,N-dimethylacetamide, DMSO: dimethyl sulfoxide, $Et_3N$: triethylamine, THF: tetrahydrofuran, EtOH: ethanol, CPME: cyclopentyl methyl ether, KI: potassium iodide, CDI: 1,1'-carbonyldiimidazole, NaH: sodium hydride, HATU: 1-[bis(dimethylamino)methylene]-1H-1,2,3-triazolo[4,5-b]pyridinium 3-oxide hexafluorophosphate, EDCI·HCl: 1-ethyl-3-(3-dimethylaminopropyl)carbodiimide hydrochloride, $Pd(OH)_2/C$: 20% palladium hydroxide on carbon (50% water content), KOtBu: potassium t-butoxide, TBAI: tetrabutylammonium iodide, TFA: trifluoroacetic acid, MeCN: acetonitrile, NMP: 1-methyl-2-pyrrolidinone, NUM: Example number or Production Example number (/I indicates that Example or Production Example is an iodide. /Na indicates that Example or Production Example is a sodium salt. /HCl indicates that Example or Production Example is a hydrochloride.), REF: Production Example number or Example number used as reference for the production method, PEx: Production Example number, Ex: Example number, STR: chemical structural formula, DAT: physiochemical data, and NMR: indicating the chemical shift $\delta$ value ppm of 500 MHz $^1$H-NMR (Parentheses in NMR indicate the measurement solvent, and for example, ($CD_3OD$) is the measurement value in deuterated methanol. NMR signal shows a typical signal.) s: singlet, d: doublet, t: triplet, q: quartet, quin: quintet, br: broad, dd: double doublet, m: multiplet, ESI+: m/z value in ESI-MS+, CI+: m/z value in CI-MS+, in this specification, a compound is sometimes named using naming software, such as ACD/Name (registered trademark, Advanced Chemistry Development, Inc.). For the purpose of convenience, the concentration mol/L is shown as M. For example, 1 M aqueous sodium hydroxide solution means an aqueous sodium hydroxide solution of 1 mol/L.

Examples and Production Examples

Production Example 1

To a mixture of diethyl 7,7'-azanediyldi(heptanoate) (10.7 g) and DCM (107 mL), CDI (7.35 g) was added at room temperature, and the mixture was stirred at room temperature for 16 hours. The reaction solution was concentrated under reduced pressure, and the obtained residue was purified by silica gel column chromatography (hexane / ethyl acetate) to obtain diethyl 7,7'-[(1H-imidazole-1-carbonyl)azanediyl]di(heptanoate) (13.5 g) as an oil.

Production Example 2

To a mixture of diethyl 7,7'-[(1H-imidazole-1-carbonyl)azanediyl]di(heptanoate) (3 g) and MeCN (30 mL), methyl iodide (2.2 mL) was added at room temperature, and the mixture was stirred at room temperature for two hours. Methyl iodide (2.2 mL) was added to the reaction mixture at room temperature, and the mixture was stirred at room temperature for 16 hours. The reaction mixture was concentrated under reduced pressure to obtain 1-[bis(7-ethoxy-7-oxoheptyl)carbamoyl]-3-methyl-1H-imidazol-3-ium iodide (4.2 g) as an oil.

Production Example 3

To a mixture of 1-[bis(7-ethoxy-7-oxoheptyl)carbamoyl]-3-methyl-1H-imidazol-3-ium iodide (4.2 g) and DMF (10 mL), a mixture of 2-(dimethylamino)-1-(4-hydroxypiperidin-1-yl)ethan-1-one (1.45 g) and THF (10 mL) was added at room temperature, followed by addition of NaH (60% in oil, 310 mg) at room temperature. The mixture was stirred at room temperature for three hours. To the reaction mixture, 1 M hydrochloric acid was added to adjust the pH to approximately 7. After diluting with ethyl acetate, water was added, and the organic layer was separated. The aqueous layer was subjected to extraction with ethyl acetate. The combined organic layer was washed with saturated brine / water (1/1), dried over anhydrous sodium sulfate and then concentrated under reduced pressure. The obtained residue was purified by amino silica gel column chromatography (hexane / ethyl acetate) to obtain diethyl 7,7'-[({[1-(N,N-dimethylglycyl)piperidin-4-yl]oxy}carbonyl)azanediyl]di(heptanoate) (1.93 g) as an oil.

Production Example 4

To a mixture of diethyl 7,7'-[({[1-(N,N-dimethylglycyl)piperidin-4-yl]oxy}carbonyl)azanediyl]di(heptanoate) (1.13 g), THF (17 mL) and EtOH (17 mL), 1 M aqueous sodium hydroxide solution (4.17 mL) was added at room temperature, and the mixture was stirred at room temperature for 72 hours. The reaction mixture was concentrated under reduced pressure and concentrated after adding EtOH (100 mL) to obtain sodium 7,7'-[({[1-(N,N-dimethylglycyl)piperidin-4-yl]oxy}carbonyl)azanediyl]di(heptanoate) (1.21 g) as a solid.

Example 1

To a mixture of sodium 7,7'-[({[1-(N,N-dimethylglycyl)piperidin-4-yl]oxy}carbonyl)azanediyl]di(heptanoate) (200 mg) and DCM (3 mL), 2-nonylundecan-1-ol (270 mg), HATU (430 mg), DIPEA (0.4 mL) and DMAP (10 mg) were added at room temperature, and the mixture was stirred at room temperature for 16 hours. Saturated sodium hydrogen carbonate aqueous solution was added to the reaction mixture, and the mixture was stirred at room temperature for 10 minutes. After diluting with DCM, water was added, and extraction was conducted using a phase separator. The organic layer was dried over anhydrous sodium sulfate and then concentrated under reduced pressure. The obtained residue was purified by silica gel column chromatography (chloroform / methanol), and then the crudely purified product was purified by amino silica gel column chromatography (hexane / ethyl acetate) to obtain bis(2-nonylundecyl) 7,7'-[({[1-(N,N-dimethylglycyl)piperidin-4-yl]oxy}carbonyl)azanediyl]di(heptanoate) (197 mg) as an oil.

Production Example 5

To a mixture of diethyl 7,7'-[({[1-(N,N-dimethylglycyl)piperidin-4-yl]oxy}carbonyl)azanediyl]di(heptanoate) (0.8 g), THF (12 mL) and EtOH (12 mL), 1 M aqueous sodium hydroxide solution (6 mL) was added at room temperature, and the mixture was stirred at room temperature for 16 hours. To the reaction mixture, 1 M hydrochloric acid (6 mL) was added, and the mixture was stirred at room temperature for 10 minutes. The reaction mixture was concentrated under reduced pressure, and chloroform / methanol (4/1) was added to the residue to obtain a suspension. Then, the suspension was filtered through celite (registered trademark). The filtrate was concentrated under reduced pressure to obtain 7,7'-[({[1-(N,N-dimethylglycyl)piperidin-4-yl]oxy}carbonyl)azanediyl]di(heptanoic acid) (700 mg) as a solid.

Example 2

To a mixture of 7,7'-[({[1-(N,N-dimethylglycyl)piperidin-4-yl]oxy}carbonyl)azanediyl]di(heptanoic acid) (100 mg) and DMF (1.5 mL), a mixture of 2-undecyltridecan-1-ol (175 mg) and DCM (1.5 mL) was added at room temperature, followed by addition of HATU (235 mg), DIPEA (0.21 mL) and DMAP (5 mg) at room temperature. The mixture was stirred in an oil bath at 50°C for an hour and at room temperature for 72 hours. Saturated sodium hydrogen carbonate aqueous solution was added to the reaction mixture, and the mixture was stirred at room temperature for 10 minutes. After diluting with chloroform, extraction was conducted using a phase separator, and the organic layer was dried over anhydrous

sodium sulfate and then concentrated under reduced pressure. The obtained residue was purified by silica gel column chromatography (chloroform / methanol), and then the crudely purified product was purified by amino silica gel column chromatography (hexane / ethyl acetate) to obtain bis(2-undecyltridecyl) 7,7'-[({[1-(N,N-dimethylglycyl)piperidin-4-yl]oxy}carbonyl)azanediyl]di(heptanoate) (98 mg) as an oil.

Production Example 6

After DMAP (17.5 g) and EDCI·HCl (27.5 g) were added to a mixture of 7-bromoheptanoic acid (20 g) and 2-decyltetradecan-1-ol (33.9 g) in DMF (200 mL) under ice cooling, the mixture was stirred at room temperature for 20 hours. After the reaction mixture was added to ice water, ethyl acetate was added. After the organic layer was separated, the organic layer was washed with saturated brine. After the aqueous layer was subjected extraction with ethyl acetate, the organic layers were combined, dried over anhydrous magnesium sulfate and then concentrated under reduced pressure. The obtained residue was purified by silica gel column chromatography (hexane / ethyl acetate) to obtain 2-decyltetradecyl 7-chloroheptanoate (32.0 g) as an oil.

Production Example 7

A mixture of 4-methoxybenzylamine (200 mg), 2-decyltetradecyl 7-chloroheptanoate (1.8 g), potassium carbonate (705 mg) and KI (48 mg) in DMAc (4 mL) was stirred in an oil bath at 125°C for 16 hours and then allowed to cool to room temperature. After ethyl acetate and water were added to the reaction mixture, the organic layer was separated. The organic layer was washed with water, dried over anhydrous sodium sulfate and then concentrated under reduced pressure. The obtained residue was purified by silica gel column chromatography (chloroform / methanol). To the mixture of the obtained crudely purified product and acetic acid (2.5 mL), $Pd(OH)_2/C$ (100 mg) was added under nitrogen atmosphere at room temperature. After the inside of the system was substituted with hydrogen gas, the mixture was stirred in the state in which a balloon filled with hydrogen was attached in an oil bath at 35°C for 19 hours. After stirring in an oil bath at 55°C for four hours, the inside of the system was substituted with nitrogen, and then $Pd(OH)_2/C$ (100 mg) was added at the same temperature. After the inside of the system was substituted with hydrogen gas, the mixture was stirred in the state in which a balloon filled with hydrogen was attached in an oil bath at 55°C for two hours. After substitution with nitrogen, the mixture was cooled to room temperature, and the precipitate of the reaction mixture was removed by filtration. After the filtrate was added to a mixture of ethyl acetate and water, potassium carbonate was added to adjust the pH to 10 or more. The organic layer was separated, and the organic layer was washed with water, dried over anhydrous sodium sulfate and then concentrated under reduced pressure. The obtained residue was purified by silica gel column chromatography (chloroform / methanol) to obtain bis(2-decyltetradecyl) 7,7'-azanediyldi(heptanoate) (270 mg) as an oil.

Example 3

A mixture of bis(2-decyltetradecyl) 7,7'-azanediyldi(heptanoate) (200 mg), CDI (103 mg) and THF (2 mL) was stirred at room temperature for an hour. In a separate container, KOtBu (190 mg) was added to a mixture of 2-(dimethylamino)-1-(4-hydroxypiperidin-1-yl)ethan-1-one (315 mg) and DMF (1.5 mL) at room temperature, and the mixture was stirred at room temperature for 10 minutes. The reaction mixture of the latter was added to the reaction mixture of the former at room temperature, and the mixture was stirred in an oil bath at 75°C for 15 hours. To the reaction mixture, a mixture separately obtained by stirring 2-(dimethylamino)-1-(4-hydroxypiperidin-1-yl)ethan-1-one (78.9 mg), KOtBu (47.4 mg) and DMF (0.5 mL) at room temperature for 10 minutes was added in an oil bath at 75°C, and the mixture was stirred at the same temperature for three hours and then allowed to cool to room temperature. After the reaction mixture was added to a mixture of ethyl acetate and water at room temperature, the organic layer was separated. The organic layer was washed with water, dried over anhydrous sodium sulfate and then concentrated under reduced pressure. The obtained residue was purified by amino silica gel column chromatography (heptane / ethyl acetate) to obtain bis(2-decyltetradecyl) 7,7'-[({[1-(N,N-dimethylglycyl)piperidin-4-yl]oxy}carbonyl)azanediyl]di(heptanoate) (29.3 mg) as an oil.

Production Example 8

To a mixture of 2-nonylundecan-1-ol (3.25 g) and DCM (40 mL), 8-bromooctanoic acid (2.97 g), DIPEA (4.7 mL), HATU (5.4 g) and DMAP (135 mg) were added in a water bath, and then the mixture was stirred at room temperature for 6.5 hours. After chloroform and water were added to the reaction mixture, the organic layer was separated, and the aqueous layer was subjected to extraction with chloroform. The combined organic layer was washed with saturated brine, dried over anhydrous magnesium sulfate and then concentrated under reduced pressure. The obtained residue was purified by silica gel column chromatography (hexane / ethyl acetate) to obtain 2-nonylundecyl 8-bromooctanoate (5.27 g) as an oil.

Production Example 9

To a mixture of 2-octyldecan-1-ol (500 mg) in DCM (5 mL), {(1r,3r)-3-[(tert-butoxycarbonyl)amino]cyclobutyl} acetic acid (511 mg), DMAP (24 mg), DIPEA (798 μL) and HATU (918 mg) were added at room temperature, and the mixture was stirred at room temperature for 6 hours. Chloroform and water were added to the reaction mixture, and the organic layer was separated. The aqueous layer was subjected to extraction with chloroform. The combined organic layer was washed with saturated brine, dried over anhydrous magnesium sulfate and then concentrated under reduced pressure. The obtained residue was purified by silica gel column chromatography (hexane / ethyl acetate) to obtain 2-octyldecyl {(1r,3r)-3-[(tert-butoxycarbonyl)amino]cyclobutyl}acetate (879 mg) as an oil.

Production Example 10

To a mixture of 2-octyldecyl {(1r,3r)-3-[(tert-butoxycarbonyl)amino]cyclobutyl}acetate (877 mg) in DCM (8 mL), TFA (1.5 mL) was added at room temperature, and then the mixture was stirred for 6 hours. After the reaction mixture was added to a mixture of chloroform and saturated sodium hydrogen carbonate aqueous solution, the organic layer was separated, and the aqueous layer was subjected to extraction with chloroform. The combined organic layer was dried over anhydrous sodium sulfate and then concentrated under reduced pressure to obtain 2-octyldecyl [(1r,3r)-3-aminocyclobutyl]acetate (661 mg) as an oil.

Production Example 11

To a mixture of 2-octyldecyl [(1r,3r)-3-aminocyclobutyl]acetate (660 mg) in MeCN (4 mL), 2-nonylundecyl 8-bromooctanoate (430 mg), CPME (4 mL), DIPEA (731 μL) and KI (30 mg) were added at room temperature, and then the mixture was stirred in an oil bath at 80°C for 48 hours. After the reaction mixture was added to a mixture of chloroform and saturated sodium hydrogen carbonate aqueous solution, the organic layer was separated, and the aqueous layer was subjected to extraction with chloroform. The combined organic layer was dried over anhydrous sodium sulfate and then concentrated under reduced pressure. After the obtained residue was purified by amino silica gel column chromatography (hexane / ethyl acetate), the crudely purified product was purified by silica gel column chromatography (chloroform / methanol) to obtain 2-nonylundecyl 8-{[(1r,3r)-3-{2-[(2-octyldecyl)oxy]-2-oxoethyl} cyclobutyl]amino } octanoate (452 mg) as an oil.

Example 4

Under nitrogen atmosphere, to a mixture of triphosgene (42 mg) in DCM (1 mL), a mixture of 2-nonylundecyl 8-{[(1r,3r)-3-{2-[(2-octyldecyl)oxy]-2-oxoethyl}cyclobutyl]amino}octanoate (100 mg) and DIPEA (26 μL) in DCM (1 mL) was added dropwise under water cooling, and then the mixture was stirred at room temperature for 30 minutes. A mixture of 1-methyl-1,4-diazepane (106 μL) and DIPEA (214 μL) in DCM (1 mL) was added dropwise to the reaction mixture under water cooling, and then the mixture was stirred at room temperature overnight. After chloroform and saturated sodium hydrogen carbonate aqueous solution were added to the reaction mixture, the organic layer was separated, and the aqueous layer was subjected to extraction with chloroform. The combined organic layer was dried over anhydrous sodium sulfate and then concentrated under reduced pressure. After the obtained residue was purified by amino silica gel column chromatography (hexane / ethyl acetate), the crudely purified product was purified by silica gel column chromatography (chloroform / methanol) to obtain 2-nonylundecyl 8-{(4-methyl-1,4-diazepane-1-carbonyl)[(1r,3r)-3-{2-[(2-octyldecyl) oxy]-2-oxoethyl}cyclobutyl]amino}octanoate (106 mg) as an oil.

Production Example 12

To a mixture of 9-bromononanoic acid (50 g) and 2-octyldodecan-1-ol (63 g) in DMF (500 mL), DMAP (38.6 g) and EDCI·HCl (60.6 g) were added under ice cooling, and the mixture was stirred at room temperature for 22 hours. The reaction mixture was added to ice water, and ethyl acetate was added. The organic layer was separated. The organic layer was washed with saturated brine, and after the aqueous layer was subjected to extraction with ethyl acetate, the combined organic layer was dried over anhydrous magnesium sulfate and concentrated under reduced pressure. The obtained residue was purified by silica gel column chromatography (hexane / ethyl acetate) to obtain 2-octyldodecyl 9-chlorononanoate (63.7 g) as an oil.

Production Example 13

A mixture of 4-methoxybenzylamine (200 mg), 2-octyldodecyl 9-chlorononanoate (1.7 g), potassium carbonate

(705 mg) and KI (48 mg) in DMAc (4 mL) was stirred in an oil bath at 125°C for 66 hours and then allowed to cool to room temperature. The reaction mixture was added to a mixture of ethyl acetate and water, and the organic layer was separated. The organic layer was washed with water, dried over anhydrous sodium sulfate and then concentrated under reduced pressure. The obtained residue was purified by silica gel column chromatography (chloroform / methanol). To a mixture of the obtained crudely purified product and acetic acid (6 mL), $Pd(OH)_2/C$ (120 mg) was added under nitrogen atmosphere at room temperature. After the inside of the system was substituted with hydrogen gas, the mixture was stirred in the state in which a balloon filled with hydrogen was attached at room temperature for 13 hours. After substitution with nitrogen, the precipitate of the reaction mixture was removed by filtration. After the filtrate was added to a mixture of ethyl acetate and water, potassium carbonate was added to adjust the pH to 10 or more. The organic layer was separated, and the organic layer was washed with water, dried over anhydrous sodium sulfate and then concentrated under reduced pressure. The obtained residue was purified by silica gel column chromatography (chloroform / methanol) to obtain bis(2-octyldodecyl) 9,9'-azanediyldi(nonanoate) (760 mg) as an oil.

Example 5

To a mixture of bis(2-octyldodecyl) 9,9'-azanediyldi(nonanoate) (250 mg) in CPME (2.5 mL), CDI (123 mg) was added at room temperature, and the mixture was stirred at the same temperature for 18 hours and then left still for three days at room temperature. To the reaction mixture, 1-methyl-1,4-diazepane (0.25 mL) was added, and the mixture was stirred at room temperature for 30 minutes and then stirred in an oil bath at 45°C for an hour. At the same temperature, 1-methyl-1,4-diazepane (1 mL) was added, and the mixture was stirred for 30 minutes and then stirred in an oil bath at 65°C for two hours. At the same temperature, TFA (45 μL) was added, and the mixture was stirred at the same temperature for an hour and then stirred in an oil bath at 95°C for 12.5 hours. At the same temperature, TFA (45 μL) was added, and the mixture was stirred at the same temperature for two hours and then stirred in an oil bath at 125°C for 20 hours. After 1-methyl-1,4-diazepane (1 mL) was added, the mixture was stirred in an oil bath at 145°C for 2.5 hours. The reaction mixture was allowed to cool to room temperature, and the reaction mixture was added to a mixture of ethyl acetate and water. The organic layer was separated, and the organic layer was washed with water, dried over anhydrous sodium sulfate and then concentrated under reduced pressure. The obtained residue was purified by silica gel column chromatography (chloroform / methanol), and the obtained crudely purified product was purified by amino silica gel column chromatography (heptane / ethyl acetate) to obtain bis(2-octyldodecyl) 9,9'-[(4-methyl-1,4-diazepane-1-carbonyl)azanediyl]di(nonanoate) (56 mg) as an oil.

Production Example 31

To a mixture of 4-nitrophenyl chloroformate (490 mg) and THF (6 mL), a mixture of 2-(dimethylamino)-1-(4-hydroxypiperidin-1-yl)ethan-1-one (452.4 mg) and THF (3 mL) was added dropwise in a water bath, and then the mixture was stirred at room temperature for an hour. Hexane (4.5 mL) was added to the reaction mixture, and after stirring at room temperature for 15 minutes, the solid was collected by filtration, washed with a mixed solvent of hexane / THF (1/2) and then dried under reduced pressure to obtain 1-(N,N-dimethylglycyl)piperidin-4-yl 4-nitrophenyl carbonate hydrochloride (337.2 mg) as a solid.

Production Example 32

A mixture of 2-hexyldecyl 7-chloroheptanoate (300 mg), tetradecyl glycinate hydrochloride (475 mg), KI (33 mg), MeCN (1.5 mL), CPME (1.5 mL) and DIPEA (0.669 mL) was stirred under irradiation with microwaves at 140°C for four hours. The reaction mixture was allowed to cool to room temperature, and chloroform was added. Then, a mixture of saturated sodium hydrogen carbonate aqueous solution / water (1/1) was added, and the organic layer was separated. After the aqueous layer was subjected to extraction with chloroform, the combined organic layer was dried over anhydrous sodium sulfate and then concentrated under reduced pressure. The obtained residue was purified by silica gel column chromatography (hexane / ethyl acetate) to obtain 2-hexyldecyl 7-{[2-oxo-2-(tetradecyloxy)ethyl]amino}heptanoate (156 mg) as an oil.

Example 14

A mixture of 2-hexyldecyl 7-{[2-oxo-2-(tetradecyloxy)ethyl]amino}heptanoate (154 mg), chloroform (3 mL), 1-(*N,N*-dimethylglycyl)piperidin-4-yl 4-nitrophenyl carbonate hydrochloride (144 mg) and $Et_3N$ (0.138 mL) was stirred under irradiation with microwaves at 140°C for an hour. After 1-(*N,N*-dimethylglycyl)piperidin-4-yl 4-nitrophenyl carbonate hydrochloride (95 mg) was added to the reaction mixture at room temperature, the mixture was stirred under irradiation with microwaves at 140°C for 30 minutes. After 1-(*N,N*-dimethylglycyl)piperidin-4-yl 4-nitrophenyl carbonate hydrochlor-

ide (96 mg) and $Et_3N$ (0.138 mL) were added to the reaction mixture at room temperature, the mixture was stirred under irradiation with microwaves at 140°C for 30 minutes. The reaction mixture was allowed to cool to room temperature and then purified by silica gel column chromatography (chloroform / methanol), and then the obtained crudely purified product was purified by amino silica gel column chromatography (hexane / ethyl acetate) to obtain 2-hexyldecyl 7-{({[1-(N,N-dimethylglycyl)piperidin-4-yl]oxy}carbonyl)[2-oxo-2-(tetradecyloxy)ethyl]amino}heptanoate (28.9 mg) as an oil.

Production Example 33

To [1-(tert-butoxycarbonyl)piperidin-4-yl]acetic acid (88 mg), HATU (138 mg), DCM (1 mL), DIPEA (124 μL) and DMAP (5 mg) were added at room temperature, and the mixture was stirred for an hour. A mixture of 2-hexyldecyl 7-{[2-oxo-2-(tetradecyloxy)ethyl]amino}heptanoate (150 mg) and DCM (1 mL) was added to the reaction mixture at room temperature, and the mixture was stirred overnight. A mixture of saturated sodium hydrogen carbonate aqueous solution / water (1/1) was added to the reaction mixture, and the organic layer was separated. After the aqueous layer was subjected to extraction with chloroform, the combined organic layer was dried over anhydrous sodium sulfate and concentrated under reduced pressure. The obtained residue was purified by silica gel column chromatography (hexane / ethyl acetate) to obtain tert-butyl 4-[2-({7-[(2-hexyldecyl)oxy]-7-oxoheptyl} [2-oxo-2-(tetradecyloxy)ethyl]amino)-2-oxoethyl]piperidine-1-carboxylate (195.2 mg) as an oil.

Production Example 34

To a mixture of tert-butyl 4-[2-({7-[(2-hexyldecyl)oxy]-7-oxoheptyl} [2-oxo-2-(tetradecyloxy)ethyl]amino)-2-oxoethyl]piperidine-1-carboxylate (193 mg) and 1,4-dioxane (2 mL), a 4 M hydrogen chloride 1,4-dioxane solution (2 mL) was added at room temperature, and the mixture was stirred overnight. After chloroform was added to the reaction mixture, a mixture of saturated sodium hydrogen carbonate aqueous solution / water (1/1) was added, and the organic layer was separated. After the aqueous layer was subjected to extraction with chloroform, the combined organic layer was dried over anhydrous sodium sulfate and concentrated under reduced pressure. The obtained residue was purified by silica gel column chromatography (chloroform / methanol) to obtain 2-hexyldecyl 7-{[2-oxo-2-(tetradecyloxy)ethyl][(piperidin-4-yl)acetyl]amino}heptanoate (166.7 mg) as an oil.

Example 15

A mixture of 2-hexyldecyl 7-{[2-oxo-2-(tetradecyloxy)ethyl][(piperidin-4-yl)acetyl]amino}heptanoate (164 mg), N,N-dimethylglycine (35 mg), DCM (2 mL), HATU (134 mg), DMAP (3 mg) and DIPEA (113 μL) was stirred at room temperature overnight. The reaction mixture was purified by amino silica gel column chromatography (hexane / ethyl acetate), and then the crudely purified product was purified by silica gel column chromatography (chloroform / methanol) to obtain 2-hexyldecyl 7-({[1-(N,N-dimethylglycyl)piperidin-4-yl]acetyl} [2-oxo-2-(tetradecyloxy)ethyl]amino)heptanoate (129.1 mg) as an oil.

Example 29 and Production Example 74

A solution of 2-dodecyltetradecan-1-ol (190 mg) in DCM (1.5 mL), HATU (204 mg), DIPEA (0.19 mL) and DMAP (5 mg) were added to a solution of 7,7'-[({[1-(N,N-dimethylglycyl)piperidin-4-yl]oxy}carbonyl)azanediyl]di(heptanoic acid) (100 mg) in DMF (1.5 mL) at room temperature, and the mixture was stirred at room temperature for two hours and in an oil bath at 50°C for 2 hours. Saturated sodium hydrogen carbonate aqueous solution was added to the reaction mixture, and the mixture was stirred at room temperature for 10 minutes. After diluting with chloroform, extraction was conducted using a phase separator, and the organic layer was dried over anhydrous sodium sulfate and then concentrated under reduced pressure. The obtained residue was purified by silica gel column chromatography (chloroform / methanol) and then purified by amino silica gel column chromatography (hexane / ethyl acetate) to obtain bis(2-dodecyltetradecyl) 7,7'-[({[1-(N,N-dimethylglycyl)piperidin-4-yl]oxy}carbonyl)azanediyl]di(heptanoate) (Example 29: 103 mg) as an oil and 7-[({[1-(N,N-dimethylglycyl)piperidin-4-yl]oxy}carbonyl){7-[(2-dodecyltetradecyl)oxy]-7-oxoheptyl}amino]heptanoic acid (Production Example 74: 73 mg) as a solid.

Production Example 82

A solution of bis(2-undecyltridecyl) 7,7'-azanediyldi(heptanoate) (850 mg) and DIPEA (170 μL) in DCM (15 mL) was added dropwise to a solution of triphosgene (420 mg) in DCM (15 mL) in a water bath (internal temperature of 30°C or lower). After the reaction mixture was stirred at room temperature for 30 minutes, a solution of tert-butyl 2,7-diazaspiro[3.5]nonane-2-carboxylate (1.42 g) and DIPEA (1.5 mL) in DCM (15 mL) was added dropwise at room temperature, and the

mixture was stirred for 2 hours. DCM and saturated sodium hydrogen carbonate aqueous solution were added to the reaction mixture, and the organic layer was separated. The aqueous layer was subjected to extraction with chloroform, and the combined organic layer was dried over anhydrous sodium sulfate and concentrated under reduced pressure. The obtained residue was purified by silica gel column chromatography (chloroform / methanol). A mixture of the purified product (650 mg), DCM (12 mL) and TFA (2 mL) was stirred at room temperature for an hour. After DCM and water were added to the reaction mixture, potassium carbonate was added to make the aqueous layer alkaline (pH 10 or more). The organic layer was separated, washed with water and concentrated under reduced pressure. The obtained residue was purified by silica gel column chromatography (chloroform / methanol), and the obtained crudely purified product was purified by silica gel column chromatography (chloroform / methanol) to obtain bis(2-undecyltridecyl) 7,7'-[(2,7-diazaspiro[3.5]nonane-7-carbonyl)azanediyl]di(heptanoate) (202 mg) as an oil.

Production Example 87

A mixture of 2-undecyltridecyl 7-{[(4-methoxyphenyl)methyl]amino}heptanoate (177 mg), 2-octyldecyl 8-bromooctanoate (140 mg), potassium carbonate (145 mg), KI (5 mg) and DMAc (3 mL) was stirred in an oil bath at 125°C for 13 hours. After cooling to room temperature, the reaction mixture was added to a mixture of ethyl acetate and water, and the organic layer was separated. The organic layer was washed with water, then dried over anhydrous sodium sulfate and concentrated under reduced pressure. The obtained residue was purified by silica gel column chromatography (chloroform / methanol). Under nitrogen atmosphere, the purified product (510 mg), acetic acid (2.5 mL) and $Pd(OH)_2/C$ (51 mg) were added at room temperature, the interior of the reaction system was purged with hydrogen, and the mixture was stirred under hydrogen atmosphere in an oil bath at 50°C for 1 hour. After the interior of the reaction system was purged with nitrogen, the reaction mixture was cooled to room temperature, and the insoluble matter was removed by filtration. The filtrate was added to a mixture of ethyl acetate and water. After potassium carbonate was added to make the aqueous layer alkaline (pH 10 or more), the organic layer was separated. The organic layer was washed with water, then dried over anhydrous sodium sulfate and concentrated under reduced pressure. The obtained residue was purified by silica gel column chromatography (chloroform / methanol) to obtain 2-octyldecyl 8-({7-oxo-7-[(2-undecyltridecyl)oxy]heptyl}amino)octanoate (140 mg) as an oil.

Production Example 88

A mixture of 4-methoxybenzylamine (1.5 g), 2-undecyltridecyl 7-bromoheptanoate (3 g), KI (91 mg), potassium carbonate (1.53 g) and DMAc (60 mL) was stirred in an oil bath at 135°C for 16 hours and then cooled to room temperature. Ethyl acetate and water were added to the reaction mixture, and the organic layer was separated. The organic layer was washed with water, dried over anhydrous sodium sulfate and then concentrated under reduced pressure. The obtained residue was purified by silica gel column chromatography (chloroform / methanol) to obtain 2-undecyltridecyl 7-{[(4-methoxyphenyl)methyl]amino}heptanoate (1.6 g) as an oil.

Production Example 94

To a mixture of 2-nonylundecyl 8-{[(1r,3r)-3-{2-[(2-octyldecyl)oxy]-2-oxoethyl}cyclobutyl]amino}octanoate (200 mg) and DCM (3 mL), CDI (61 mg) was added at room temperature, and the reaction mixture was stirred at room temperature overnight. CDI (63 mg) was added to the reaction mixture at room temperature, and the reaction mixture was stirred at room temperature for 2 hours. CDI (66 mg) was added to the reaction mixture at room temperature, and the reaction mixture was stirred at room temperature for 3 hours. The reaction mixture was purified by silica gel chromatography (hexane / ethyl acetate) to obtain 2-nonylundecyl 8-{(1*H*-imidazole-1-carbonyl)[(1*r*,3*r*)-3-{2-[(2-octyldecyl)oxy]-2-oxoethyl}cyclobutyl]amino}octanoate (222.9 mg) as an oil.

Example 48

MeCN (1 mL), DCM (1 mL) and methyl iodide (153 μL) were added to 2-nonylundecyl 8-{(1H-imidazole-1-carbonyl)[(1r,3r)-3-{2-[(2-octyldecyl)oxy]-2-oxoethyl}cyclobutyl]amino}octanoate (219 mg) at room temperature, and the mixture was stirred overnight. Methyl iodide (150 μL) was added to the reaction mixture at room temperature, and the mixture was stirred in an oil bath at 50°C for 9 hours. The reaction mixture was cooled to room temperature and concentrated under reduced pressure. After DMF (1 mL), 2-(dimethylamino)-1-(4-hydroxypiperidin-1-yl)ethan-1-one (51 mg) and THF (1 mL) were added to the obtained residue under nitrogen atmosphere at room temperature, NaH (11 mg, 55% in oil) was added, and the mixture was stirred at room temperature overnight. After 1 M hydrochloric acid (0.5 mL) was added to the reaction mixture, ethyl acetate and saturated sodium hydrogen carbonate aqueous solution were added, and the organic layer was separated. The aqueous layer was subjected to extraction with ethyl acetate, and then the combined

organic layer was washed four times with saturated brine / water (1/1), then washed with saturated brine, then dried over anhydrous sodium sulfate and concentrated under reduced pressure. The obtained residue was purified by silica gel column chromatography (chloroform / methanol), and then the obtained crudely purified product was purified by amino silica gel column chromatography (hexane / ethyl acetate) to obtain 2-nonylundecyl 8-{({[1-(N,N-dimethylglycyl)piperidin-4-yl]oxy}carbonyl)[(1r,3r)-3-{2-[(2-octyldecyl)oxy]-2-oxoethyl}cyclobutyl]amino}octanoate (114.2 mg) as an oil.

Production Example 126

To 2-nonylundecyl 8-{(1*H*-imidazole-1-carbonyl)[(1*r*,3*r*)-3-{2-[(2-octyldecyl)oxy]-2-oxoethyl}cyclobutyl]amino} octanoate (3.21 g), chloroform (15 mL), MeCN (15 mL) and methyl iodide (2.28 mL) were added, and the mixture was stirred in an oil bath at 50°C for 16 hours. The reaction mixture was concentrated under reduced pressure to obtain 3-methyl-1-({8-[(2-nonylundecyl)oxy]-8-oxooctyl}[(1*r*,3*r*)-3-{2-[(2-octyldecyl)oxy]-2-oxoethyl}cyclobutyl]carbamoyl)-1*H*-imidazol-3-ium iodide (3.71 g) as a viscous foamy substance.

Example 56

To 3-methyl-1-({8-[(2-nonylundecyl)oxy]-8-oxooctyl}[(1*r*,3*r*)-3-{2-[(2-octyldecyl)oxy]-2-oxoethyl}cyclobutyl]carbamoyl)-1*H*-imidazol-3-ium iodide (150 mg), DCM (3 mL), 1-ethyl-1,4-diazepane (22 mg) and DIPEA (0.07 mL) were added, and the mixture was stirred at room temperature for a weekend. Chloroform and water were added at room temperature, and the organic layer was separated. The organic layer was washed with saturated brine, dried over anhydrous sodium sulfate and then concentrated under reduced pressure. The obtained residue was purified by silica gel column chromatography (chloroform / methanol) and then purified by amino silica gel column chromatography (hexane / ethyl acetate) to obtain 2-nonylundecyl 8-{(4-ethyl-1,4-diazepane-1-carbonyl)[(1*r*,3*r*)-3-{2-[(2-octyldecyl)oxy]-2-oxoethyl}cyclobutyl]amino}octanoate (107 mg) as an oil.

Production Example 109

Under nitrogen atmosphere, a solution of methyl (1r,3r)-3-[(8-ethoxy-8-oxooctyl)amino]cyclobutane-1-carboxylate (1.4 g) and DIPEA (0.97 mL) in DCM (14 mL) was slowly added dropwise to a solution of triphosgene (1.55 g) in DCM (14 mL) under water cooling. After the reaction mixture was stirred at room temperature for 1.5 hours, a solution of 1-methyl-1,4-diazepane (4.1 mL) and DIPEA (8 mL) in DCM (14 mL) was slowly added dropwise under water cooling, and the mixture was stirred at room temperature overnight. The reaction mixture was added to a mixture of chloroform and saturated sodium hydrogen carbonate aqueous solution at room temperature, and then the organic layer was separated. The aqueous layer was subjected to extraction with chloroform, and the combined organic layer was dried over anhydrous sodium sulfate and concentrated under reduced pressure. The obtained residue was purified by amino silica gel column chromatography (hexane / ethyl acetate) to obtain methyl (1r,3r)-3-[(8-ethoxy-8-oxooctyl)(4-methyl-1,4-diazepane-1-carbonyl)amino]cyclobutane-1-carboxylate (1.527 g) as an oil.

Production Example 117

A mixture of methyl (1*r*,4*r*)-4-aminocyclohexane-1-carboxylate monohydrochloride (3.09 g), MeCN (20 mL), CPME (20 mL), ethyl 8-bromooctanoate (2 g), DIPEA (7 mL) and KI (265 mg) was stirred at room temperature for 10 minutes, and then stirred in an oil bath at 80°C for 2 days. The reaction mixture was cooled to room temperature, chloroform, water and saturated sodium hydrogen carbonate aqueous solution were added to the reaction mixture, and the organic layer was separated
. The aqueous layer was subjected to extraction with chloroform, and the combined organic layer obtained was dried over anhydrous sodium sulfate and then filtered, and the filtrate was concentrated under reduced pressure. The obtained residue was purified by silica gel column chromatography (chloroform / methanol) and then purified by amino silica gel column chromatography (hexane / ethyl acetate) to obtain methyl (1r,4r)-4-[(8-ethoxy-8-oxooctyl)amino]cyclohexane-1-carboxylate (929.2 mg) as an oil.

Production Example 118

Under nitrogen atmosphere, a mixture of methyl (1r,4r)-4-[(8-ethoxy-8-oxooctyl)amino]cyclohexane-1-carboxylate (927 mg), DIPEA (0.6 mL) and DCM (10 mL) was slowly added dropwise to a mixture of triphosgene (0.94 g) and DCM (10 mL) under water cooling, and the reaction mixture was then stirred at room temperature for 2.5 hours. To the reaction mixture, a mixture of 1-methyl-1,4-diazepane (2.48 mL), DIPEA (4.8 mL) and DCM (10 mL) was slowly added dropwise under water cooling, and the reaction mixture was stirred at room temperature for 2 days. The reaction mixture was added

to a mixture of chloroform and saturated sodium hydrogen carbonate aqueous solution at room temperature, and the organic layer was separated. The aqueous layer was subjected to extraction with chloroform, and the combined organic layer obtained was dried over anhydrous sodium sulfate and filtered, and the filtrate was then concentrated under reduced pressure. The obtained residue was purified by amino silica gel column chromatography (hexane / ethyl acetate) to obtain methyl (1*r*,4*r*)-4-[(8-ethoxy-8-oxooctyl)(4-methyl-1,4-diazepane-1-carbonyl)amino]cyclohexane-1-carboxylate (1.3115 g) as an oil.

Production Example 119

To a mixture of methyl (1*r*,4*r*)-4-[(8-ethoxy-8-oxooctyl)(4-methyl-1,4-diazepane-1-carbonyl)amino]cyclohexane-1-carboxylate (1.31 g), THF (15 mL) and EtOH (15 mL), 1 M aqueous sodium hydroxide solution (11 mL) was added at room temperature, and the reaction mixture was stirred at room temperature for 3 hours. After 1 M hydrochloric acid (11 mL) was added to the reaction mixture under water cooling, the reaction mixture was stirred at room temperature for 3 hours. The reaction mixture was concentrated under reduced pressure, and a mixture of chloroform / methanol (4/1) was added to the obtained residue to obtain a suspension, which was then dried over anhydrous sodium sulfate and filtered. The obtained filtrate was concentrated under reduced pressure to obtain (1r,4r)-4-[(7-carboxyheptyl)(4-methyl-1,4-diazepane-1-carbonyl)amino]cyclohexane-1-carboxylic acid (1.2216 g) as a foamy solid.

Example 72

To a mixture of (1*r*,4*r*)-4-[(7-carboxyheptyl)(4-methyl-1,4-diazepane-1-carbonyl)amino]cyclohexane-1-carboxylic acid (150 mg), HATU (410 mg), DMAP (9 mg), DMF (0.5 mL) and DIPEA (0.37 mL), a mixture of 2-heptylnonan-1-ol (225 mg) and DCM (1.5 mL) was added at room temperature, and the reaction mixture was then stirred at room temperature for 4 days. The reaction mixture was added to a mixture of saturated sodium hydrogen carbonate aqueous solution and ethyl acetate, and the organic layer was separated. After the aqueous layer was subjected to extraction with ethyl acetate, the combined organic layer obtained was washed with saturated brine / water (1/1), then washed with saturated brine, dried over anhydrous sodium sulfate and then filtered, and the filtrate was concentrated under reduced pressure. The obtained residue was purified by amino silica gel column chromatography (hexane / ethyl acetate) to obtain 2-heptylnonyl (1r,4r)-4-[{8-[(2-heptylnonyl)oxy]-8-oxooctyl}(4-methyl-1,4-diazepane-1-carbonyl)amino]cyclohexane-1-carboxylate (168.3 mg) as an oil.

Production Example 122

Chloroform (6 mL), MeCN (6 mL) and methyl iodide (2 mL) were added to ethyl 8-{(1H-imidazole-1-carbonyl)[(1r,3r)-3-(2-methoxy-2-oxoethyl)cyclobutyl]amino}octanoate (1.13 g) at room temperature, and the mixture was stirred in an oil bath at 50°C for 16 hours. Then, the reaction mixture was concentrated under reduced pressure. The obtained residue was dissolved in DCM (10 mL), and 1-methyl-1,4-diazepane (0.6 mL) and DIPEA (1 mL) were added. The mixture was stirred at room temperature for a weekend. Chloroform and water were added to the reaction mixture, and the organic layer was separated. The organic layer was washed with water and saturated brine, dried over anhydrous sodium sulfate and then concentrated under reduced pressure. The obtained residue was purified by amino silica gel column chromatography (hexane / ethyl acetate) to obtain ethyl 8-{[(1r,3r)-3-(2-methoxy-2-oxoethyl)cyclobutyl](4-methyl-1,4-diazepane-1-carbonyl)amino}octanoate (1.0902 g) as an oil.

Example 79

A mixture of bis(2-octyldecyl) 8,8'-azanediyldi(octanoate) (270 mg), THF (6 mL) and bis(pentafluorophenyl) carbonate (264 mg) was stirred at room temperature for 3 hours. Bis(pentafluorophenyl) carbonate (264 mg) was added to the reaction mixture, which was stirred at room temperature for 3 hours. THF was added to the reaction mixture to dilute to a total volume of 12 mL, this diluted mixture (6 mL) was added dropwise to a mixture of 1-ethyl-1,4-diazepane (1 g), CPME (4 mL) and $K_2CO_3$ (232 mg) at room temperature, and the resulting mixture was stirred in an oil bath at 85°C for 20 hours. In an oil bath at 85°C, 1-ethyl-1,4-diazepane (0.5 g) was added to the reaction mixture, which was further stirred in an oil bath at 85°C for 5 hours and then cooled to room temperature. Heptane and water were added to the reaction mixture, and the organic layer was separated. The organic layer was washed with 90% methanol aqueous solution and concentrated under reduced pressure. The obtained residue was purified by silica gel column chromatography (heptane / ethyl acetate, ethyl acetate / methanol) to obtain bis(2-octyldecyl) 8,8'-[(4-ethyl-1,4-diazepane-1-carbonyl)azanediyl]di(octanoate) (70 mg) as an oil.

Production Example 128

To benzyl 4-({8-[(2-nonylundecyl)oxy]-8-oxooctyl}[(1*r*,3*r*)-3-{2-[(2-octyldecyl)oxy]-2-oxoethyl}cyclobutyl]carbamoyl)-1,4-diazepane-1-carboxylate (1.64 g), 2-propanol (32 mL) was added, $Pd(OH)_2$/C (164 mg) was then added under argon atmosphere, and the mixture was stirred under hydrogen atmosphere at room temperature for 18 hours. The reaction mixture was filtered through celite (registered trademark), and the filtrate was concentrated under reduced pressure to obtain 2-nonylundecyl 8-{(1,4-diazepane-1-carbonyl)[(1*r*,3*r*)-3-{2-[(2-octyldecyl)oxy]-2-oxoethyl}cyclobutyl]amino}octanoate (1.42 g) as an oil.

Example 80

To 2-nonylundecyl 8-{(1,4-diazepane-1-carbonyl)[(1*r*,3*r*)-3-{2-[(2-octyldecyl)oxy]-2-oxoethyl}cyclobutyl]amino}octanoate (120 mg), DCM (3 mL), *N,N* -dimethylglycine (16 mg), HATU (59 mg) and DIPEA (0.033 mL) were added, and the mixture was stirred at room temperature for 18 hours. The reaction mixture was purified by silica gel column chromatography (chloroform / methanol) and then purified by amino silica gel column chromatography (hexane / ethyl acetate) to obtain 2-nonylundecyl 8-{[4-(*N,N*-dimethylglycyl)-1,4-diazepane-1-carbonyl][(1*r*,3*r*)-3-{2-[(2-octyldecyl)oxy]-2-oxoethyl}cyclobutyl]amino}octanoate (119 mg) as an oil.

Example 81

To 2-nonylundecyl 8-{(1,4-diazepane-1-carbonyl)[(1r,3r)-3-{2-[(2-octyldecyl)oxy]-2-oxoethyl}cyclobutyl]amino}octanoate (130 mg), CPME (2.5 mL), MeCN (2.5 mL), 2-iodopropane (0.028 mL) and DIPEA (0.048 mL)were added, the mixture was stirred in an oil bath at 80°C for 6 hours, 2-iodopropane (0.028 mL) and DIPEA (0.048 mL) were then added, and the mixture was stirred in an oil bath at 80°C for 16 hours. After cooling to room temperature, the reaction mixture was purified by silica gel column chromatography (chloroform / methanol) and then purified by amino silica gel column chromatography (hexane / ethyl acetate) to obtain 2-nonylundecyl 8-{[(1*r*,3*r*)-3-12-[(2-octyldecyl)oxy]-2-oxoethyll cyclobutyl] [4-(propan-2-yl)-1,4-diazepane-1-carbonyl]amino}octanoate (116 mg) as an oil.

Production Example 133

To benzyl 8-[{(1r,3r)-3-[2-(benzyloxy)-2-oxoethyl]cyclobutyl} (1*H*-imidazole-1-carbonyl)amino]octanoate (1.24 g), MeCN (14 mL) and methyl iodide (1.4 mL) were added, and the mixture was stirred in an oil bath at 50°C for 18 hours. The reaction mixture was concentrated under reduced pressure to obtain 1-({(1*r*,3*r*)-3-[2-(benzyloxy)-2-oxoethyl]cyclobutyl }[8-(benzyloxy)-8-oxooctyl]carbamoyl)-3-methyl-1*H*-imidazol-3-ium iodide (1.53 g) as an oil.

Production Example 135

To benzyl 8-[{(1*r*,3*r*)-3-[2-(benzyloxy)-2-oxoethyl]cyclobutyl}(4-ethyl-1,4-diazepane-1-carbonyl)amino]octanoate (200 mg), 2-propanol (5 mL) was added, palladium-activated carbon (Pd 10%) (20 mg) was then added under argon atmosphere, and the mixture was stirred under hydrogen atmosphere at room temperature for 18 hours. The reaction mixture was filtered through celite (registered trademark), and the filtrate was concentrated under reduced pressure to obtain 8-{[(1*r*,3*r*)-3-(carboxymethyl)cyclobutyl](4-ethyl-1,4-diazepane-1-carbonyl)amino}octanoic acid (140 mg) as an oil.

Production Example 139

To a mixture of ethyl 9-{[(1*r*,3*r*)-3-(2-ethoxy-2-oxoethyl)cyclobutyl]amino}nonanoate (2.53 g) and DCM (40 mL), CDI (2.41 g) was added, and the mixture was stirred at room temperature for 30 minutes and then stirred in a water bath at 35°C for 2 hours. The reaction solution was concentrated under reduced pressure, and the obtained residue was then purified by silica gel column chromatography (chloroform / methanol) to obtain ethyl 9-{[(1*r*,3*r*)-3-(2-ethoxy-2-oxoethyl)cyclobutyl](1*H*-imidazole-1-carbonyl)amino}nonanoate (3.23 g) as an oil.

Production Example 142

To a mixture of ethyl 9-{[(1*r*,3*r*)-3-(2-ethoxy-2-oxoethyl)cyclobutyl](4-ethyl-1,4-diazepane-1-carbonyl)amino }nonanoate (2.89 g) and EtOH (30 mL), 1 M aqueous sodium hydroxide solution (15 mL) was added at room temperature, and the mixture was stirred in a water bath at 40°C for 4 hours and stirred in a water bath at 50°C for 1 hour. The reaction mixture was allowed to cool, and then neutralized by adding 1 M hydrochloric acid (15 mL). The reaction mixture was

concentrated under reduced pressure, and chloroform / methanol (4/1) (50 mL) was added to the obtained residue, and the mixture was dried over anhydrous sodium sulfate and concentrated under reduced pressure. To the obtained residue, 1,4-dioxane (50 mL) was added, and the mixture was stirred and then concentrated under reduced pressure to obtain 9-{[(1*r*,3*r*)-3-(carboxymethyl)cyclobutyl](4-ethyl-1,4-diazepane-1-carbonyl)amino}nonanoic acid (3.19 g) as a foamy solid.

Production Example 143

A mixture of 2-octyldecyl [(1*r*,3*r*)-3-aminocyclobutyl]acetate (495.5 mg), CPME (2.4 mL), 2-undecyltridecyl 7-bromoheptanoate (349.5 mg), $K_2CO_3$ (177 mg), sodium iodide (12 mg) and MeCN (2.4 mL) was stirred at an external temperature of 80°C for 17 hours, and then allowed to cool to room temperature. To the reaction mixture, heptane and 90% methanol aqueous solution were added, and the heptane layer was then separated. The heptane layer was washed with 90% methanol aqueous solution, and concentrated under reduced pressure. The obtained residue was purified by silica gel column chromatography (chloroform / methanol) to obtain 2-undecyltridecyl 7-{[(1*r*,3*r*)-3-{2-[(2-octyldecyl)oxy]-2-oxoethyl}cyclobutyl]lamino}heptanoate (260 mg) as an oil.

Example 90

A mixture of 2-undecyltridecyl 7-{[(1*r*,3*r*)-3-{2-[(2-octyldecyl)oxy]-2-oxoethyl}cyclobutyl]amino}heptanoate (129.7 mg), CPME (0.5 mL) and bis(pentafluorophenyl) carbonate (181.4 mg) was stirred at room temperature for 1 hour 45 minutes. After adding 1-ethyl-1,4-diazepane (490 mg) and CPME (0.5 mL) at room temperature, the mixture was stirred at an external temperature of 97°C for 17 hours 15 minutes, and allowed to cool to room temperature. Heptane and 90% methanol aqueous solution were added to the reaction mixture, and the heptane layer was then separated. The heptane layer was washed with 90% methanol aqueous solution, and concentrated under reduced pressure. The obtained residue was purified by silica gel column chromatography (heptane / ethyl acetate) to obtain 2-undecyltridecyl 7-{(4-ethyl-1,4-diazepane-1-carbonyl)[(1*r*,3*r*)-3-{2-[(2-octyldecyl)oxy]-2-oxoethyl}cyclobutyl]amino}heptanoate (123.5 mg) as an oil.

Production Example 145

A mixture of 2-octyldecyl [(1*r*,3*r*)-3-aminocyclobutyl]acetate (400 mg), 2-undecyltridecyl 9-bromononanoate (300 mg), $K_2CO_3$ (220 mg) and MeCN (9 mL) was stirred in an oil bath at 80°C for 18 hours, and then allowed to cool to room temperature. Heptane and water were added to the reaction mixture, and the organic layer was separated. The organic layer was washed with 90% methanol aqueous solution, and concentrated under reduced pressure. The obtained residue was purified by silica gel column chromatography (heptane / ethyl acetate) to obtain 2-undecyltridecyl 9-{[(1*r*,3*r*)-3-{2-[(2-octyldecyl)oxy]-2-oxoethyl}cyclobutyl]amino}nonanoate (280 mg) as an oil.

Production Example 150

To a mixture of methyl (1*r*,4*r*)-4-[(8-ethoxy-8-oxooctyl)amino]cyclohexane-1-carboxylate (370 mg) and THF (8 mL), CDI (367 mg) was added at room temperature, and the mixture was stirred in an oil bath at 50°C for 64 hours and then allowed to cool at room temperature. CDI (376 mg) was added to the reaction mixture, which was stirred in an oil bath at 50°C for 24 hours. The reaction mixture was concentrated under reduced pressure, and the obtained residue was then purified by silica gel column chromatography (chloroform / methanol) to obtain methyl (1*r*,4*r*)-4-[(8-ethoxy-8-oxooctyl)(1*H*-imidazole-1-carbonyl)amino]cyclohexane-1-carboxylate (362 mg) as an oil.

[Table 5-1]

| NUM | STR |
|---|---|
| PEx1 | 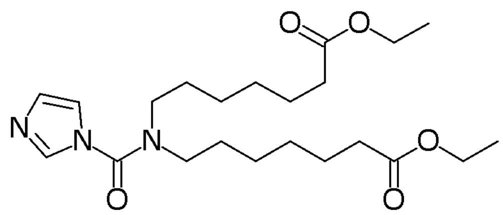 |

(continued)

| NUM | STR |
|---|---|
| PEx2 /I | 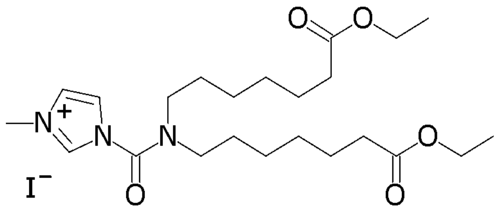 |
| PEx3 | 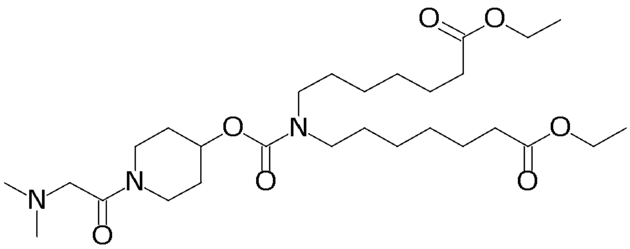 |
| PEx4 /Na | 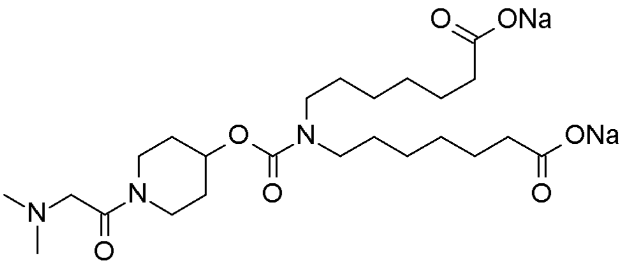 |
| Ex1 | 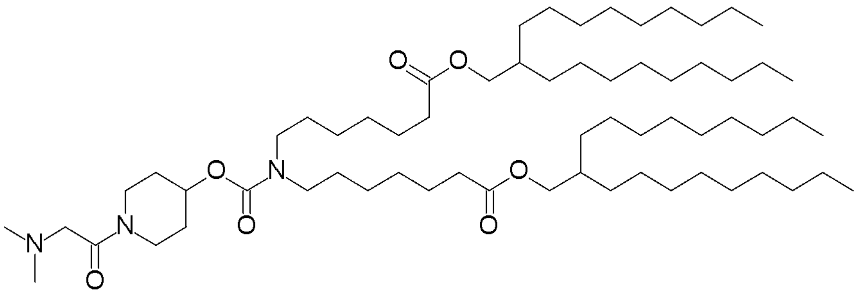 |
| PEx5 | 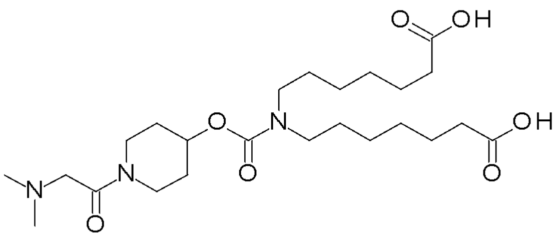 |

[Table 5-2]

| | |
|---|---|
| Ex2 | 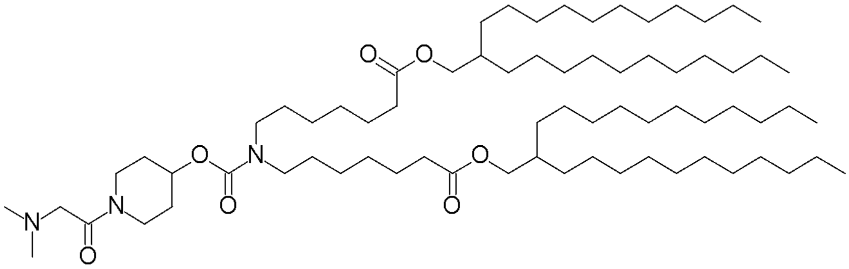 |

(continued)

| | |
|---|---|
| PEx6 | 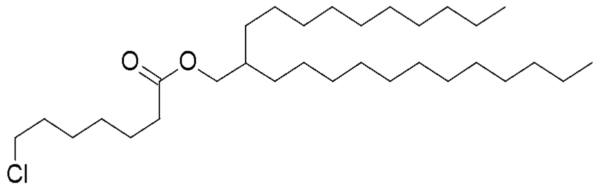 |
| PEx7 | 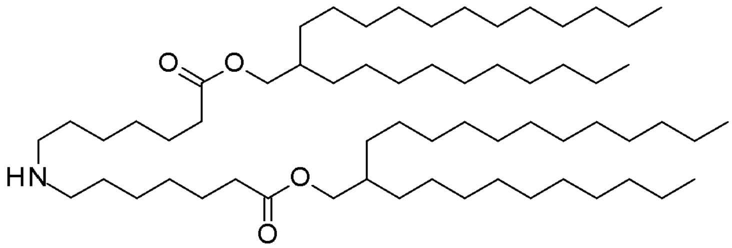 |
| Ex3 | 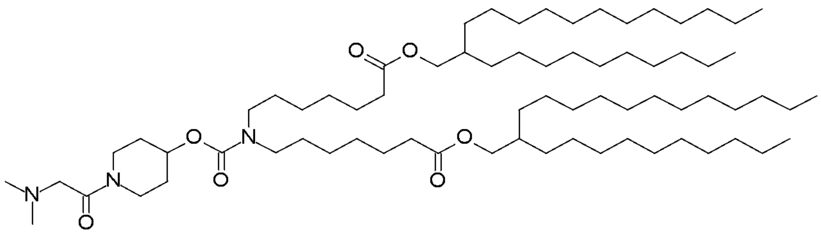 |
| PEx8 | 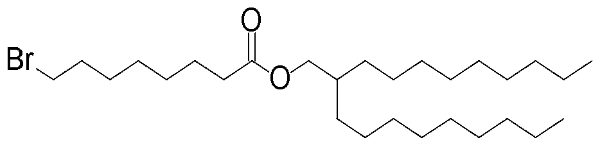 |
| PEx9 | 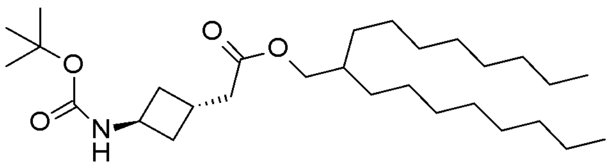 |
| PEx10 | 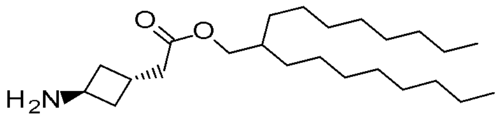 |

[Table 5-3]

| | |
|---|---|
| PEx11 | 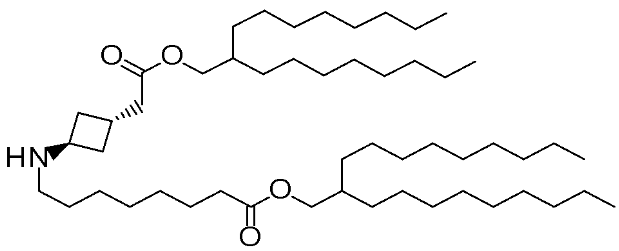 |

(continued)

| | |
|---|---|
| Ex4 | 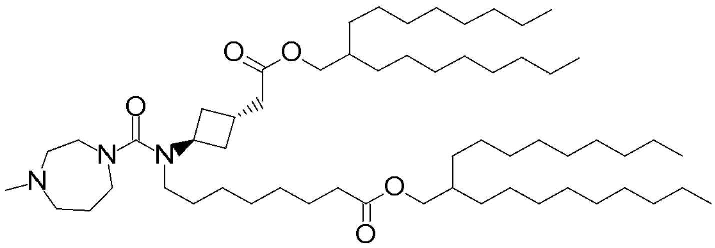 |
| PEx12 | 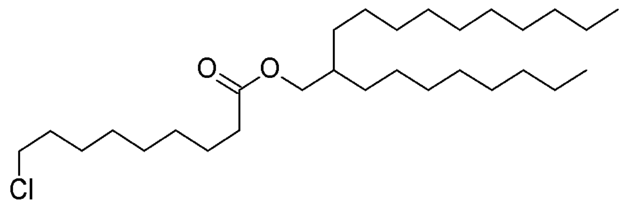 |
| PEx13 | 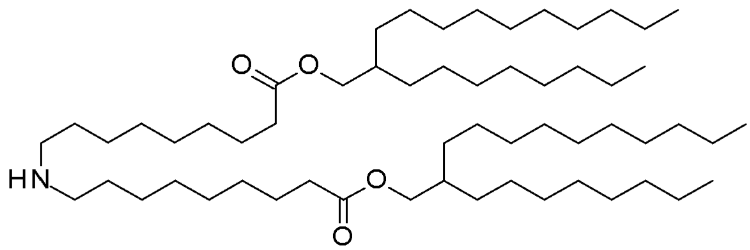 |
| Ex5 | 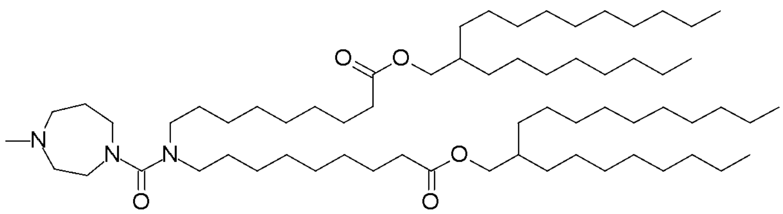 |
| PEx14 | 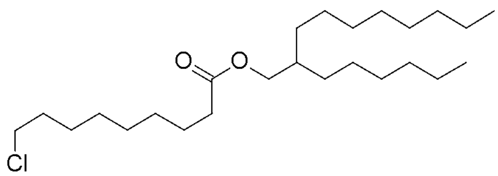 |
| PEx15 | 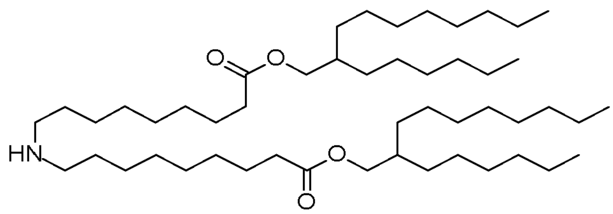 |

[Table 5-4]

| | |
|---|---|
| Ex6 | 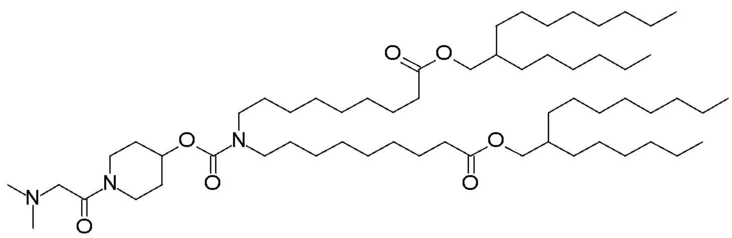 |

(continued)

| | |
|---|---|
| Ex7 | 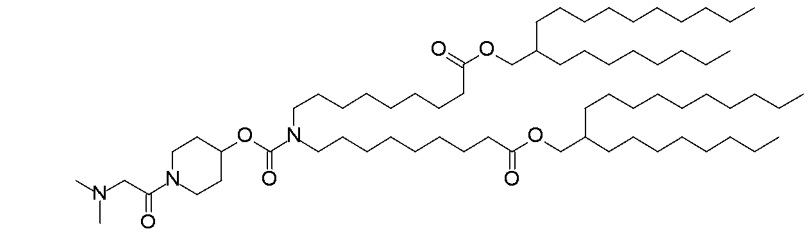 |
| PEx18 | 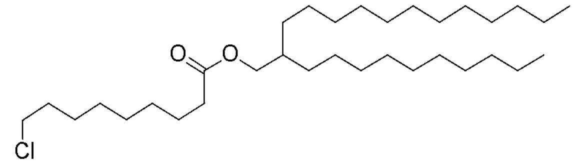 |
| PEx19 | 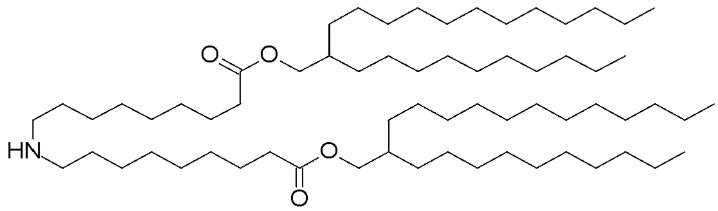 |
| Ex8 | 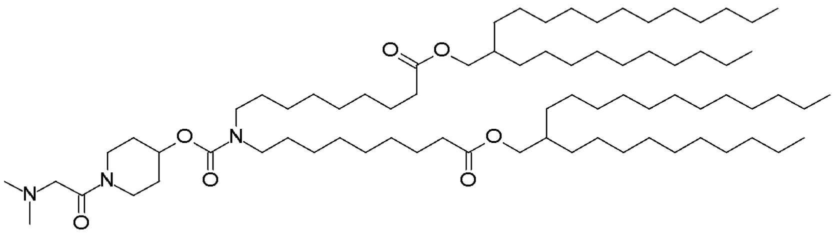 |
| PEx20 | 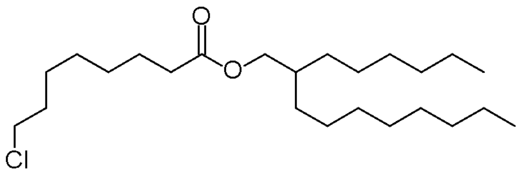 |
| PEx21 | 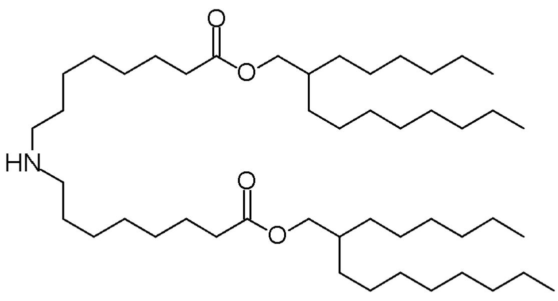 |

[Table 5-5]

| | |
|---|---|
| Ex9 | 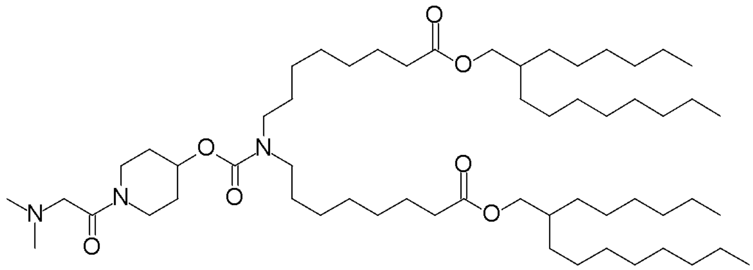 |
| PEx22 | 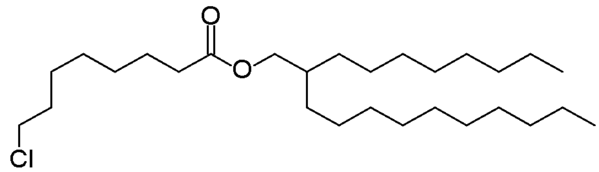 |
| PEx23 | 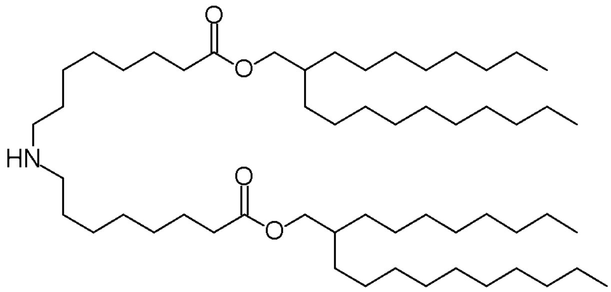 |
| Ex10 | 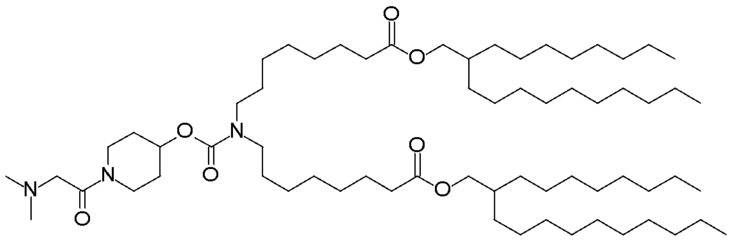 |
| PEx24 | 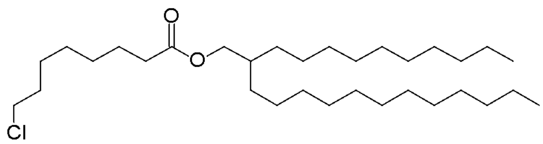 |
| PEx25 | 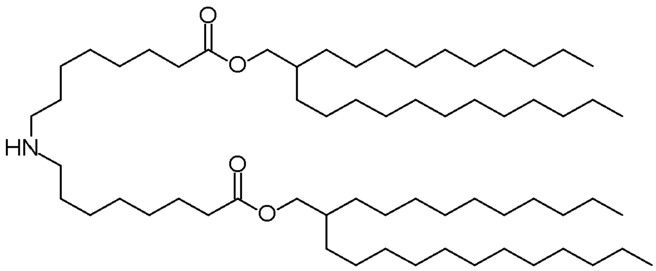 |

[Table 5-6]

| | |
|---|---|
| Ex11 | 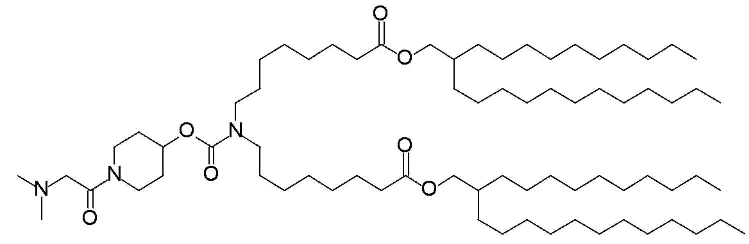 |
| PEx26 | 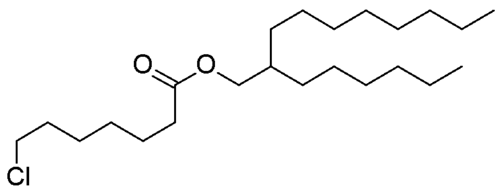 |
| PEx27 | 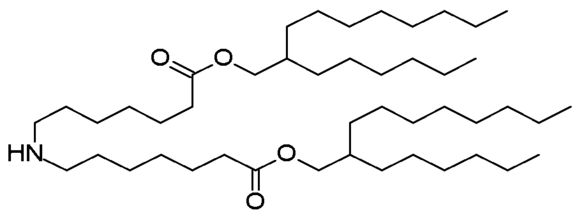 |
| Ex12 | 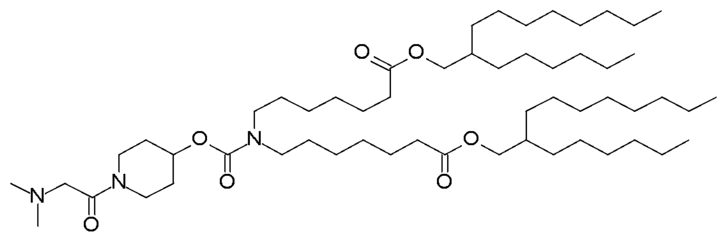 |
| PEx28 | 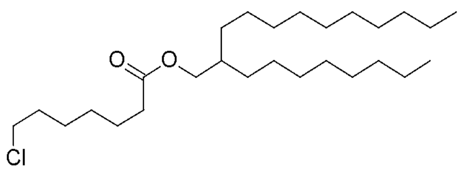 |
| PEx29 | 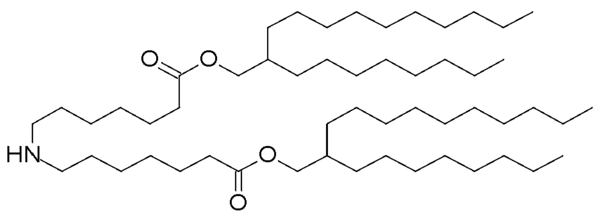 |
| Ex13 | 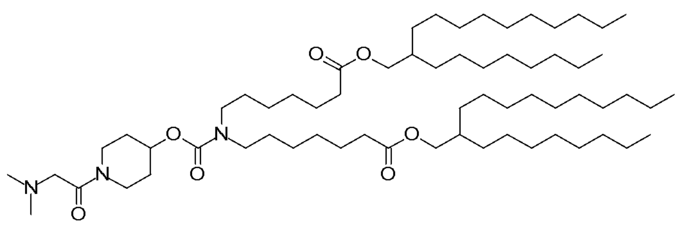 |

[Table 5-7]

| | |
|---|---|
| PEx31/HCl | 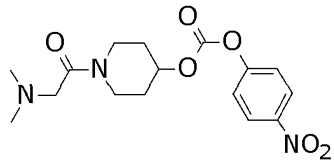 |
| PEx32 | 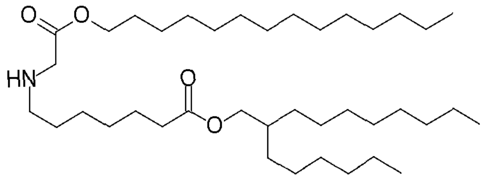 |
| Ex14 | 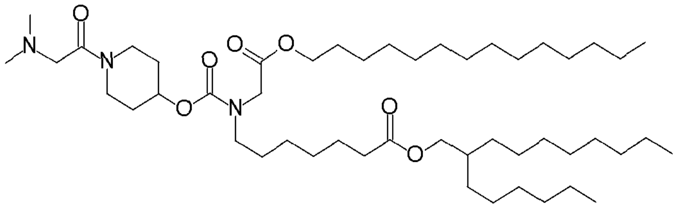 |
| PEx33 | 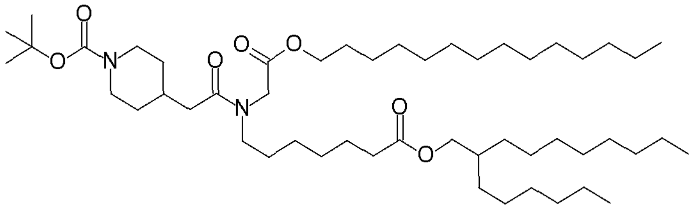 |
| PEx34 | 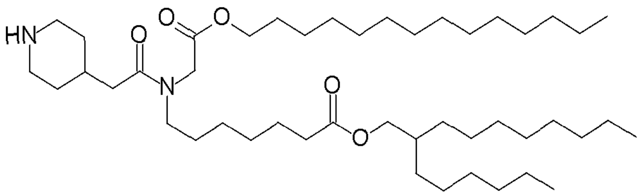 |
| Ex15 | 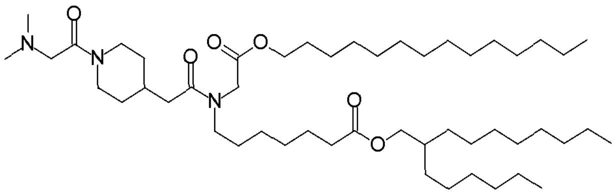 |
| PEx35 | 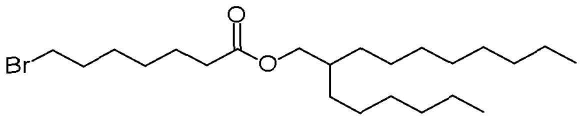 |
| PEx36 | 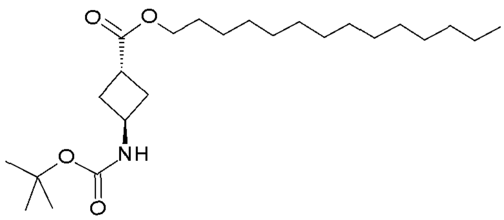 |

[Table 5-8]

| | |
|---|---|
| PEx37/HCl | 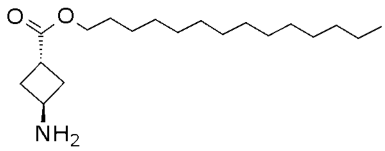 |
| PEx38 | 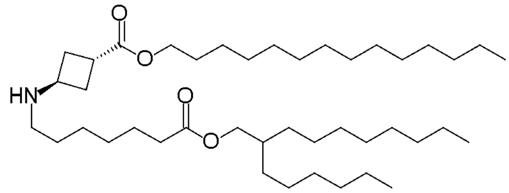 |
| Ex16 | 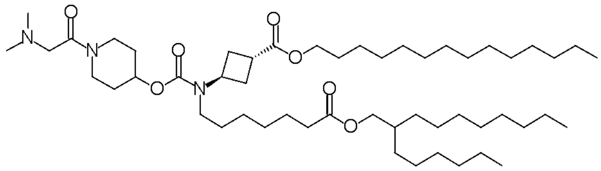 |
| PEx39 | 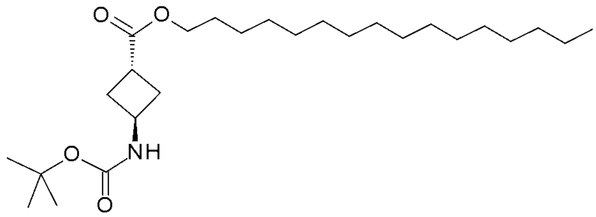 |
| PEx40/HCl | 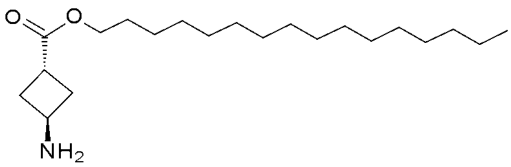 |
| PEx41 | 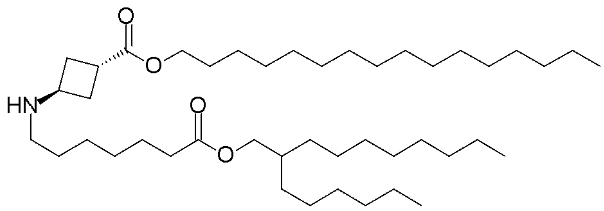 |
| Ex17 | 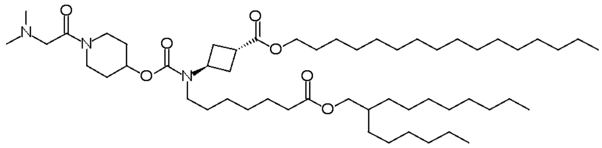 |
| PEx42 | 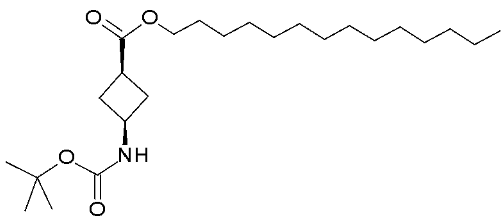 |

[Table 5-9]

| | |
|---|---|
| PEx43/HCl | 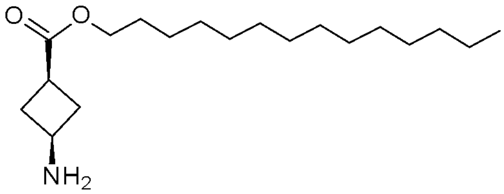 |
| PEx44 | 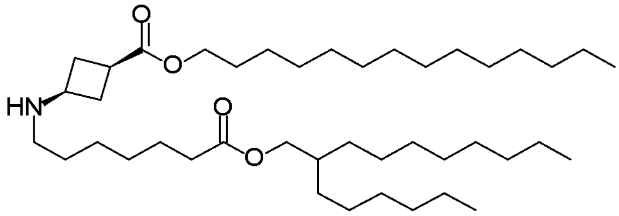 |
| Ex18 | 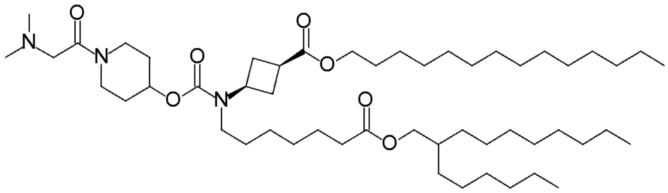 |
| PEx45 | 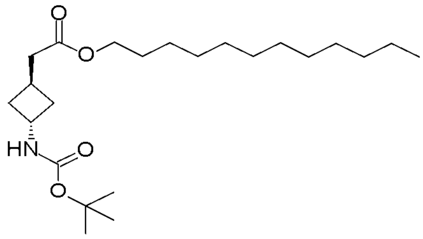 |
| PEx46/HCl | 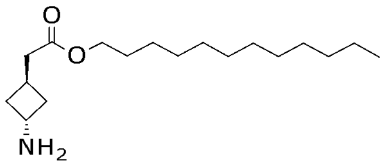 |
| PEx47 | 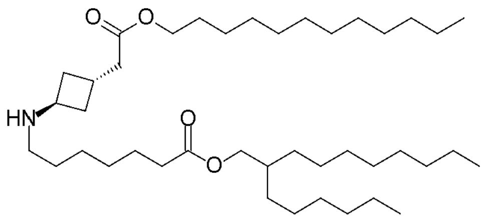 |
| Ex19 | 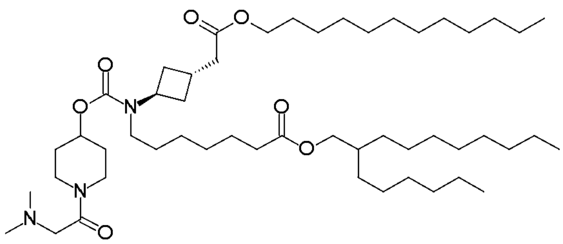 |

[Table 5-10]

| | |
|---|---|
| PEx48 | 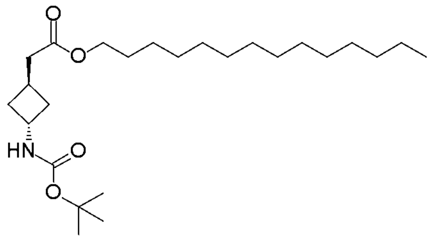 |
| PEx49/HCl | 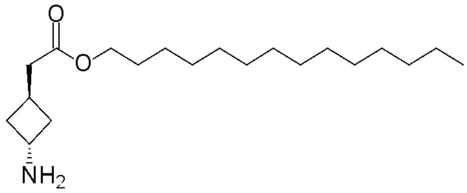 |
| PEx50 | 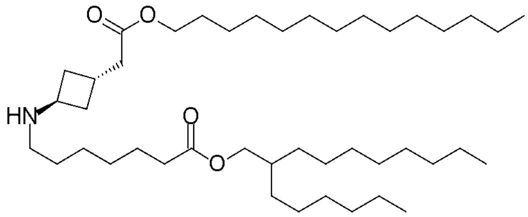 |
| Ex20 | 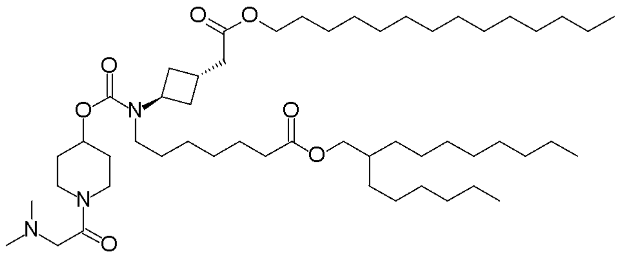 |
| PEx51 | 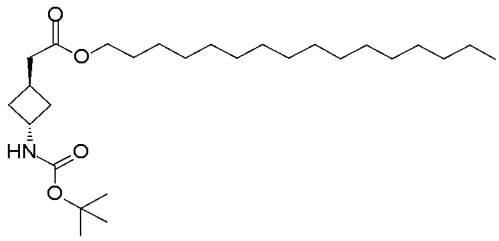 |
| PEx52 /HCl | 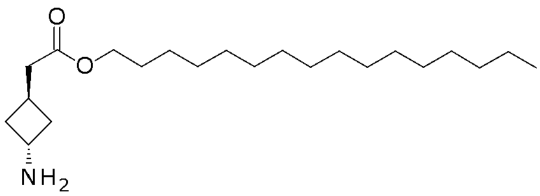 |
| PEx53 | 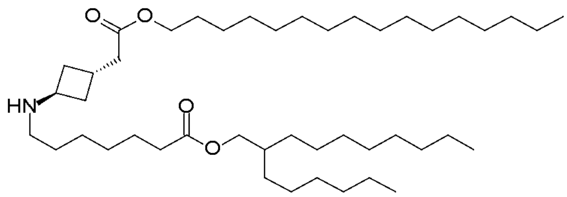 |

[Table 5-11]

| | |
|---|---|
| Ex21 | 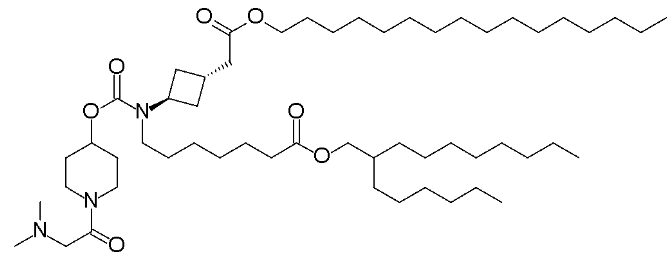 |
| PEx54 | 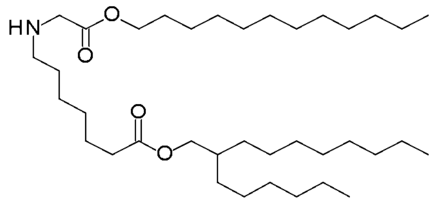 |
| PEx55 | 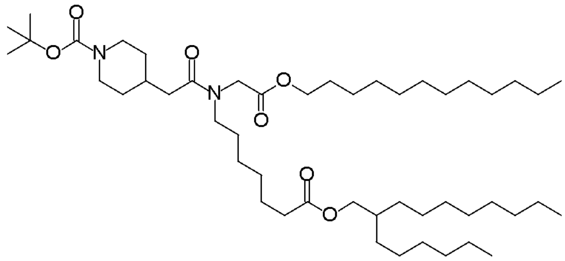 |
| PEx56/HCl | 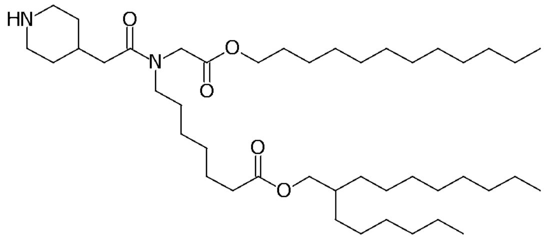 |
| Ex22 | 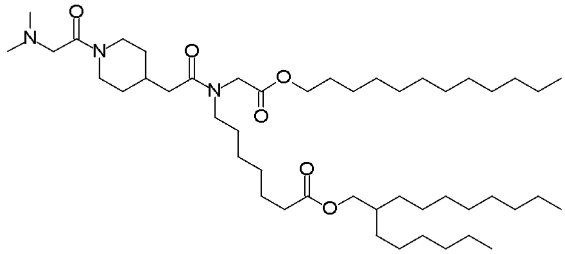 |
| PEx57 | 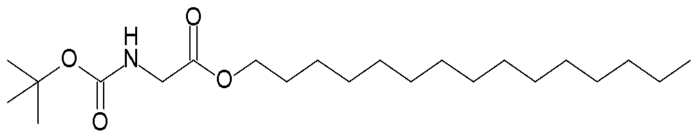 |
| PEx58/HCl | 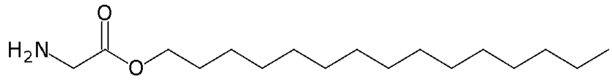 |

[Table 5-12]

| | |
|---|---|
| PEx59 | 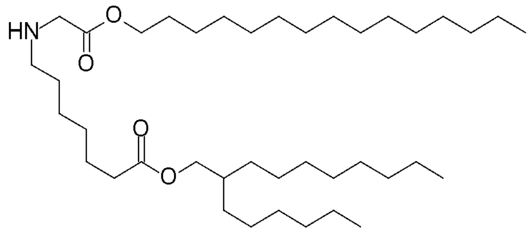 |
| PEx60 | 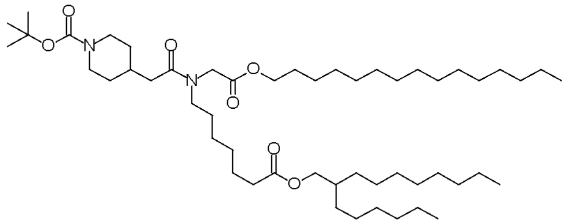 |
| PEx61/HCl | 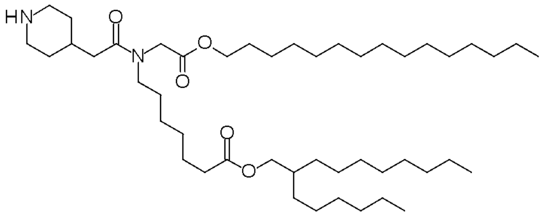 |
| Ex23 | 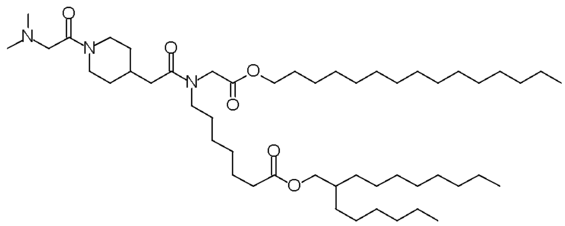 |
| PEx62/HCl | 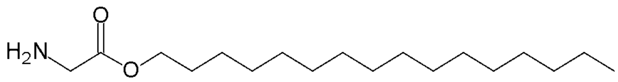 |
| PEx63 | 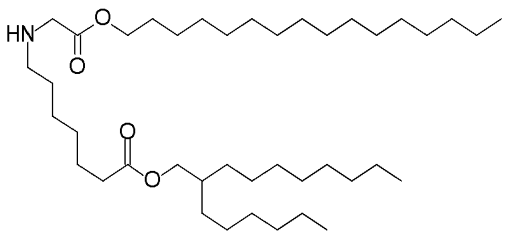 |
| PEx64 | 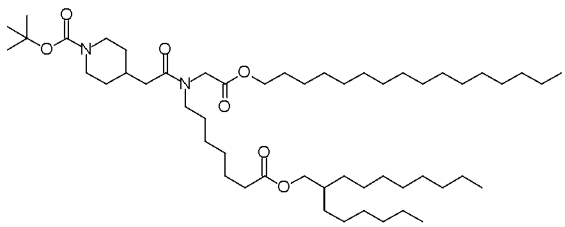 |

[Table 5-13]

| | |
|---|---|
| PEx65/HCl | 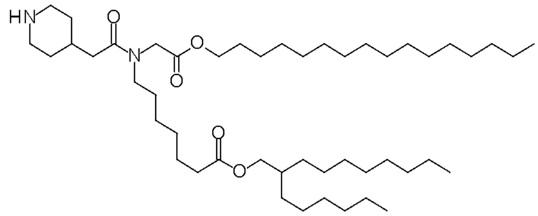 |
| Ex24 | 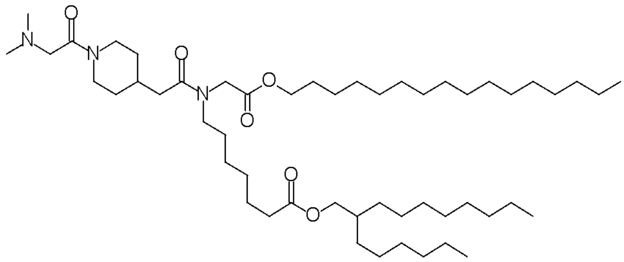 |
| PEx66 | 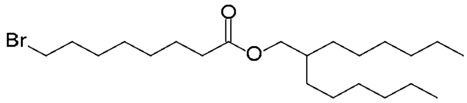 |
| PEx67 | 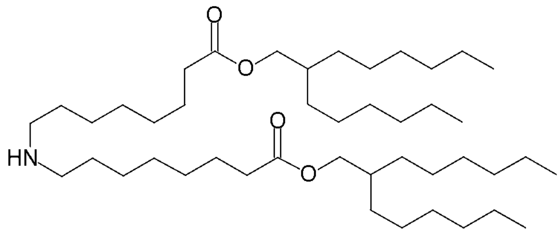 |
| Ex25 | 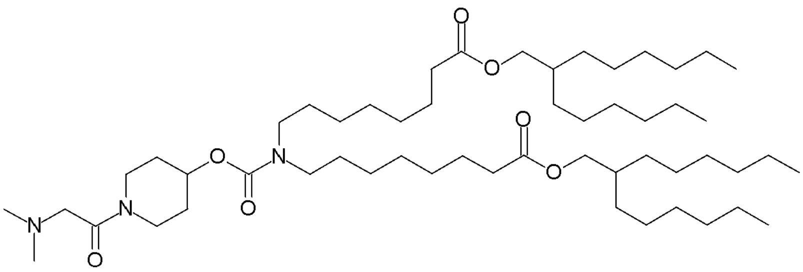 |
| PEx68 | 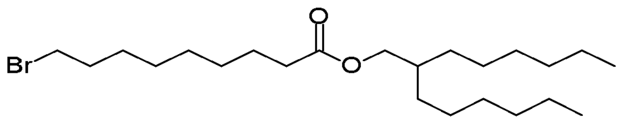 |
| PEx69 | 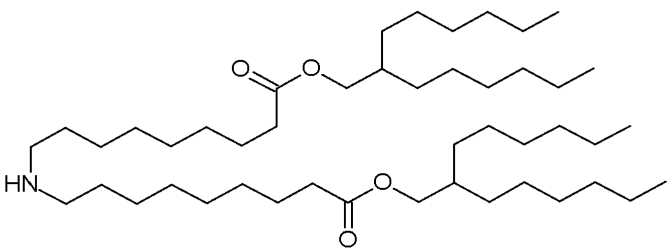 |

[Table 5-14]

| | |
|---|---|
| Ex26 | 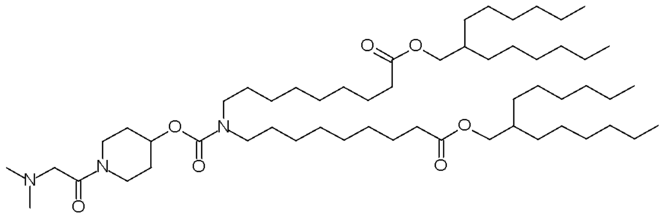 |
| PEx70 | 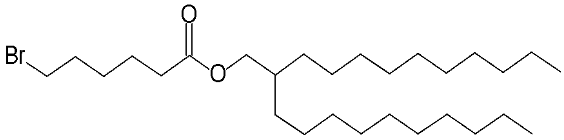 |
| PEx71 | 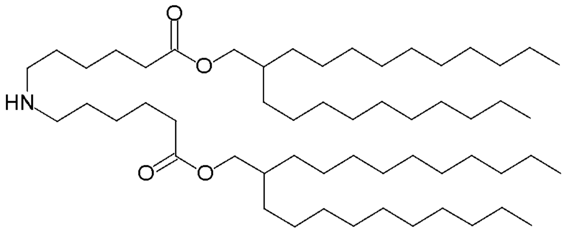 |
| Ex27 | 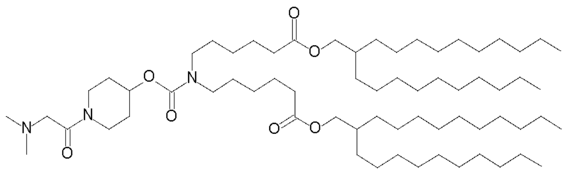 |
| PEx72 | 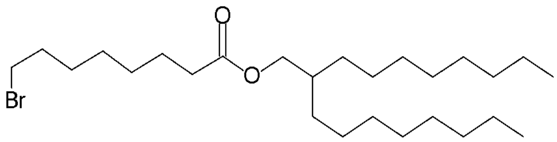 |
| PEx73 | 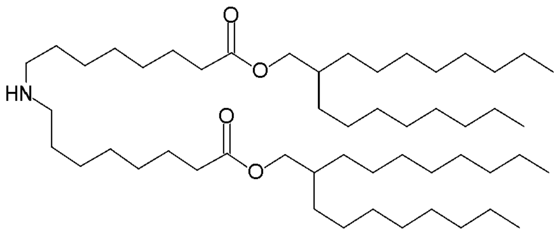 |
| Ex28 | 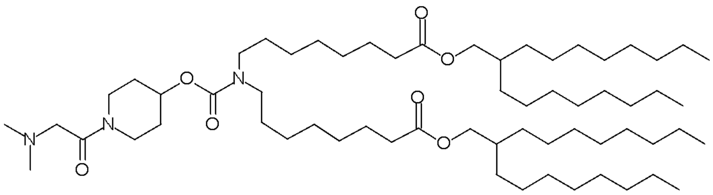 |
| Ex29 | 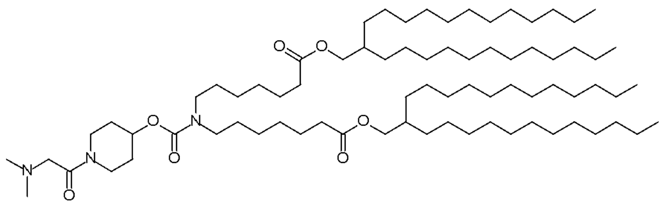 |

[Table 5-15]

| | |
|---|---|
| PEx74 | 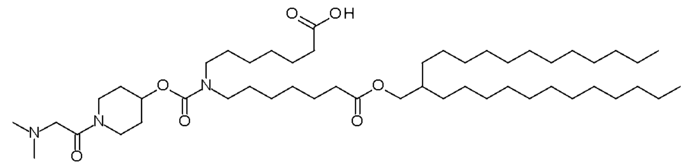 |
| Ex30 | 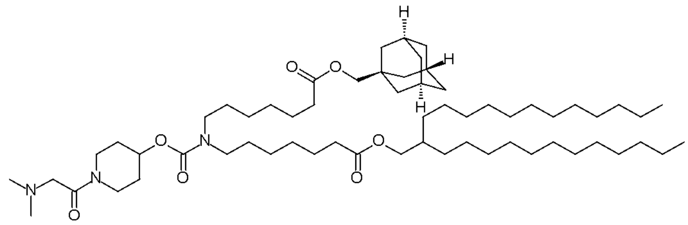 |
| Ex31 | 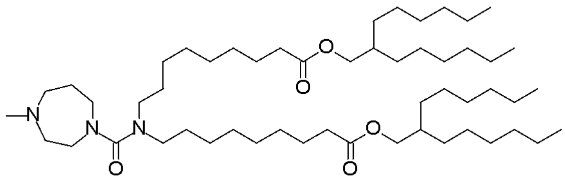 |
| Ex32 | 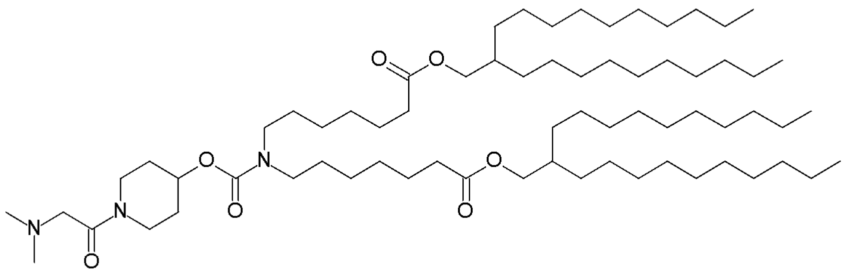 |
| Ex33 | 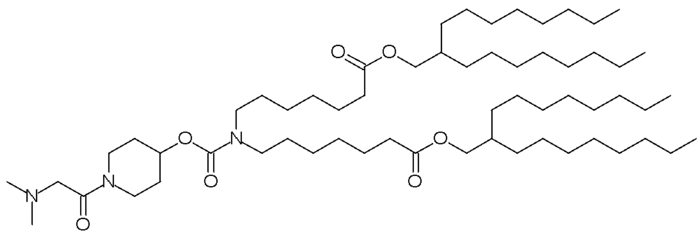 |
| Ex34 | 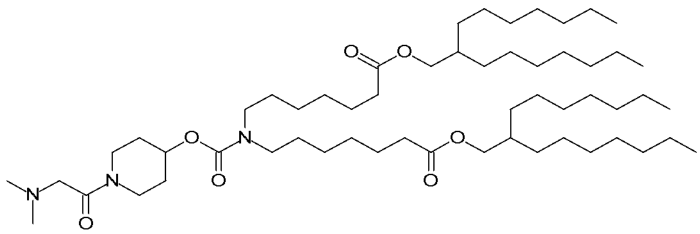 |
| PEx75 | 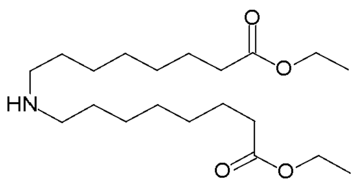 |

[Table 5-16]

| | |
|---|---|
| PEx76 | 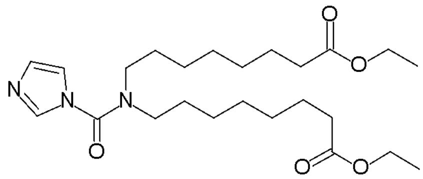 |
| PEx77/I | 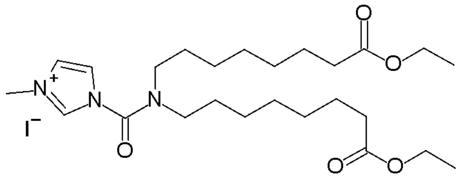 |
| PEx78 | 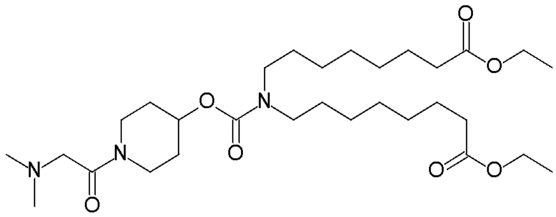 |
| PEx79/Na | 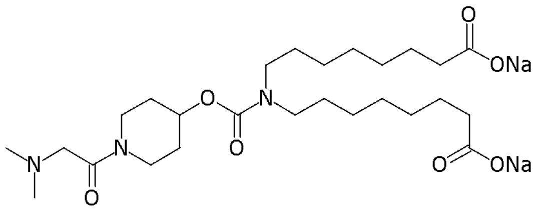 |
| Ex35 | 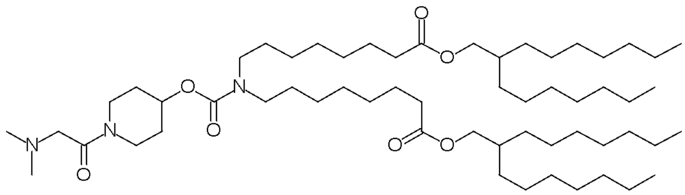 |
| Ex36 | 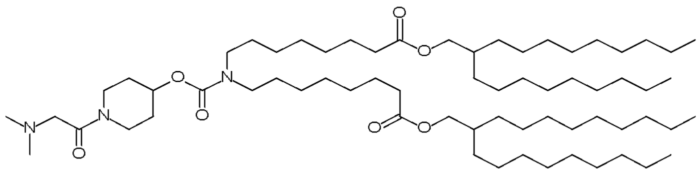 |
| Ex37 | 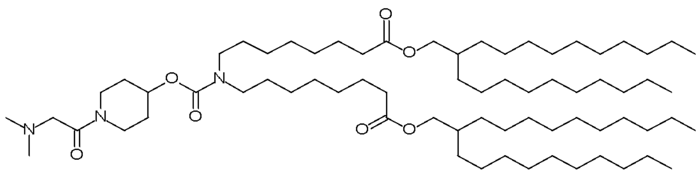 |
| Ex38 | 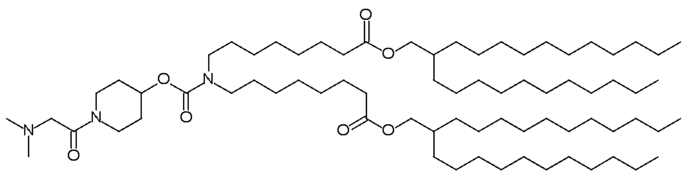 |

[Table 5-17]

| | |
|---|---|
| PEx80 | 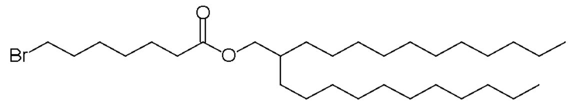 |
| PEx81 | 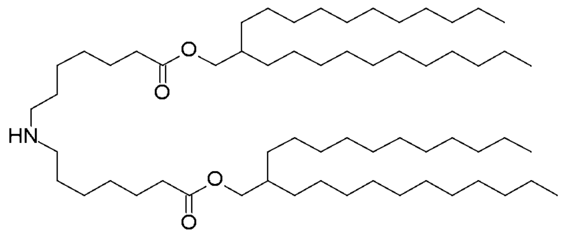 |
| PEx82 | 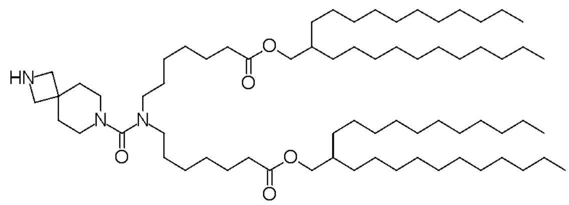 |
| Ex39 | 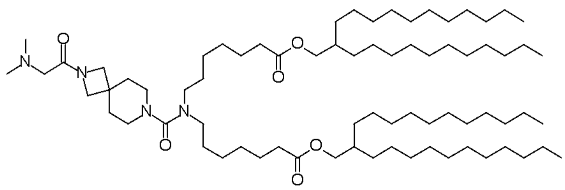 |
| Ex40 | 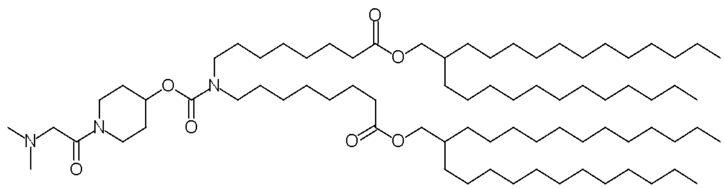 |
| Ex41 | 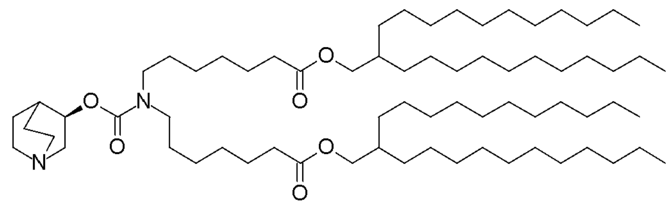 |
| Ex42 | 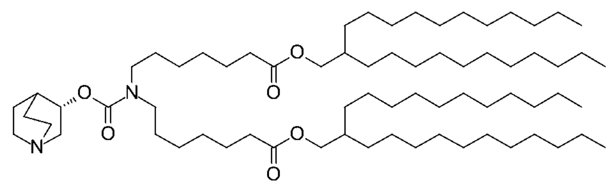 |
| PEx83 | 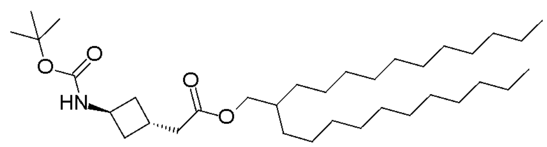 |
| PEx85 | 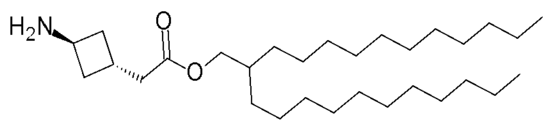 |

[Table 5-18]

| | |
|---|---|
| PEx86 | 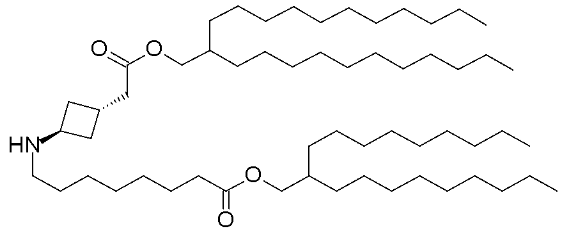 |
| Ex43 | 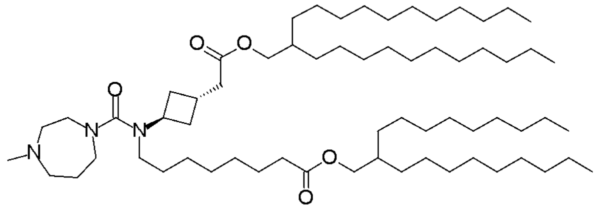 |
| PEx87 | 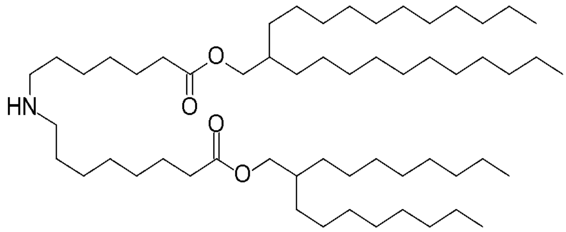 |
| Ex44 | 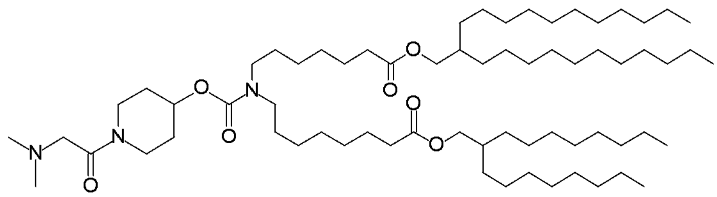 |
| PEx88 | 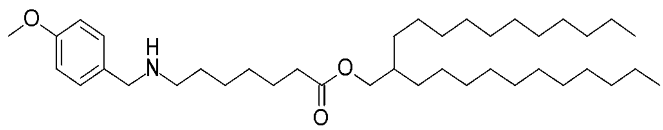 |
| PEx89 | 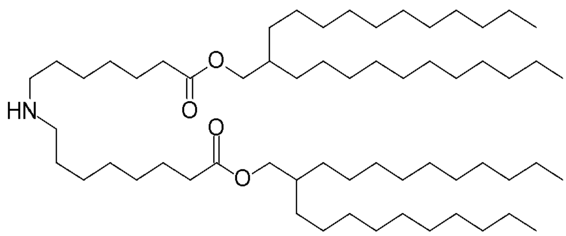 |
| Ex45 | 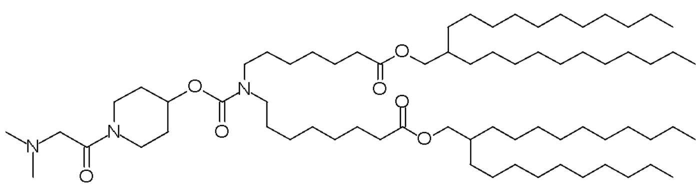 |

[Table 5-19]

| | |
|---|---|
| PEx90 | 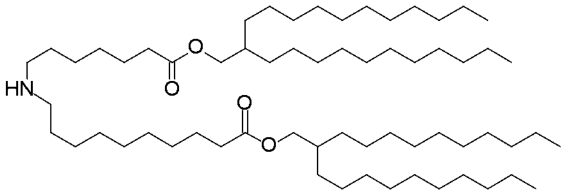 |
| Ex46 | 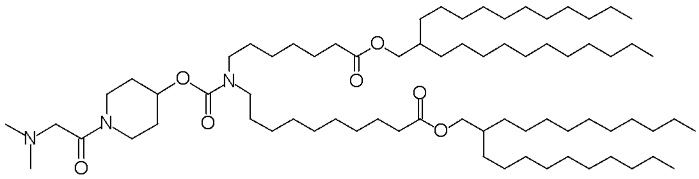 |
| Ex47 | 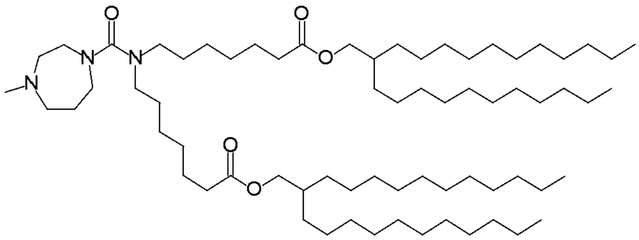 |
| PEx94 | 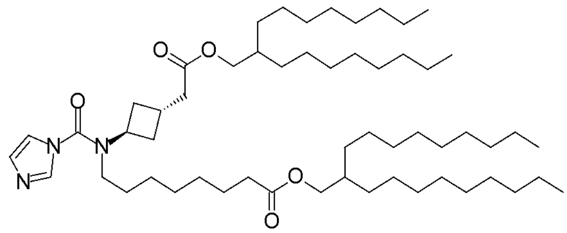 |
| Ex48 | 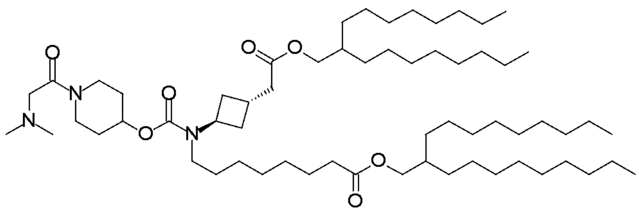 |
| PEx99 | 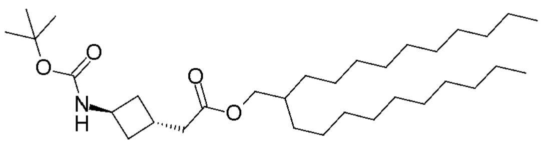 |
| PEx 100 | 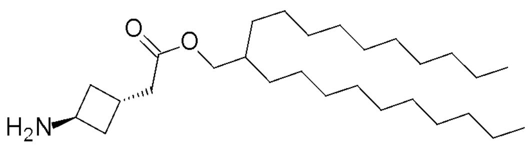 |

[Table 5-20]

| | |
|---|---|
| PEx101 | 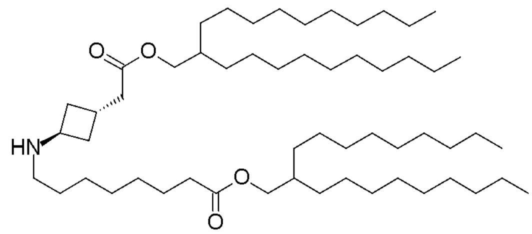 |
| PEx102 | 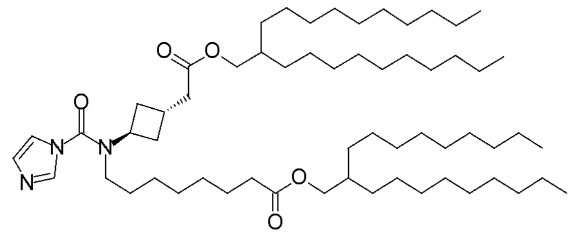 |
| Ex51 | 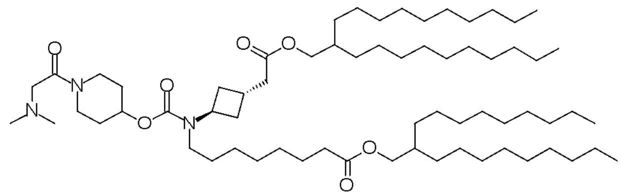 |
| PEx103 | 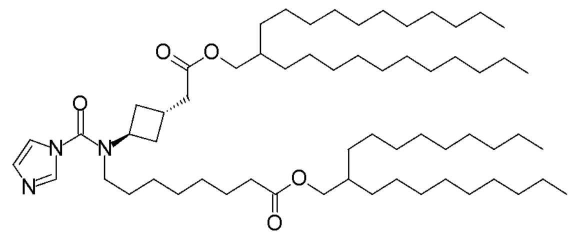 |
| Ex52 | 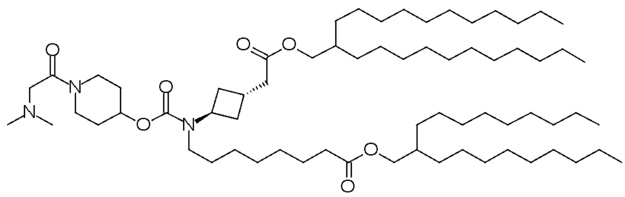 |
| Ex56 | 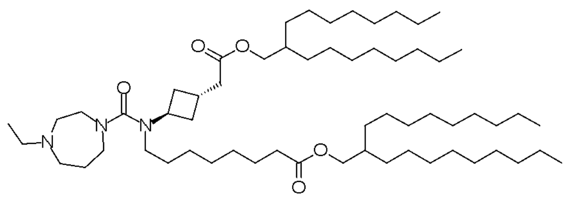 |
| Ex57 | 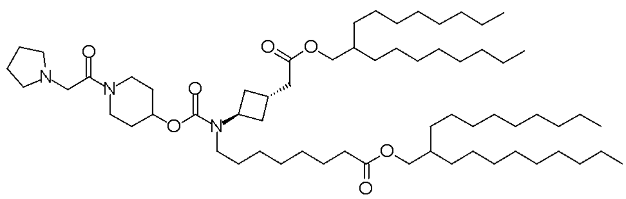 |

[Table 5-21]

| | |
|---|---|
| Ex58 | 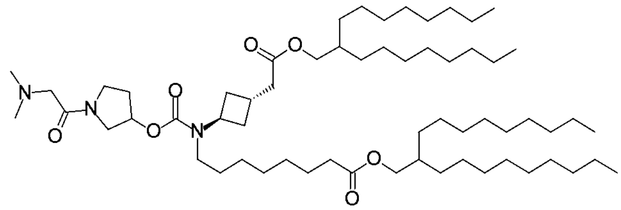 |
| PEx108 | 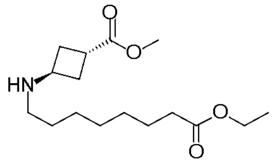 |
| PEx109 | 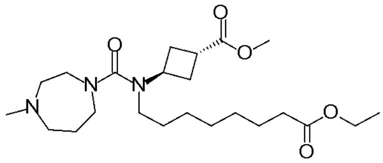 |
| PEx110 | 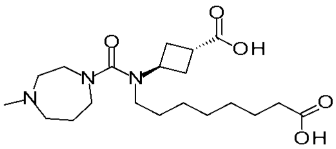 |
| Ex59 | 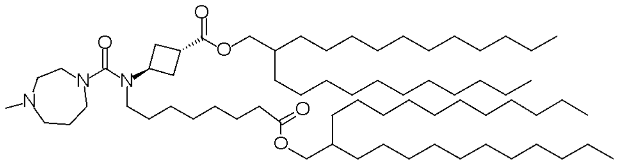 |
| Ex60 | 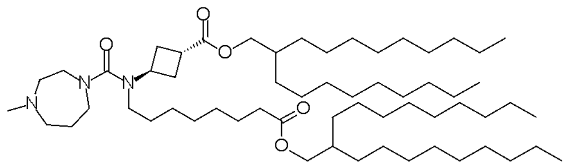 |
| PEx111 | 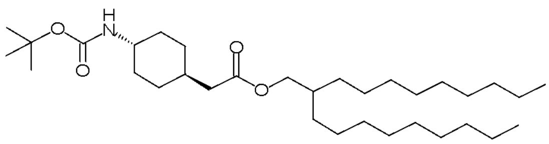 |
| PEx112 | 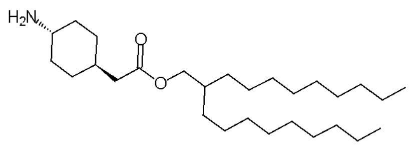 |
| PEx113 | 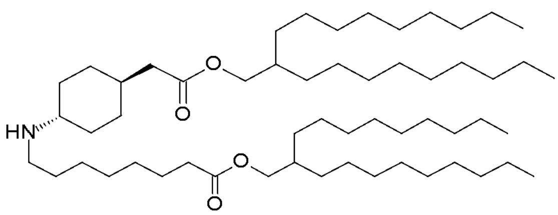 |

[Table 5-22]

| | |
|---|---|
| Ex63 | 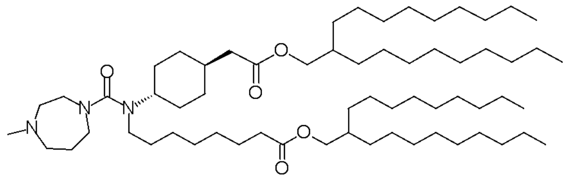 |
| PEx114 | 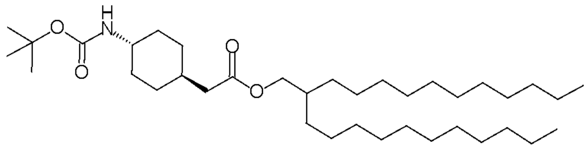 |
| PEx115 | 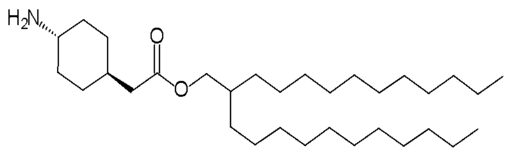 |
| PEx116 | 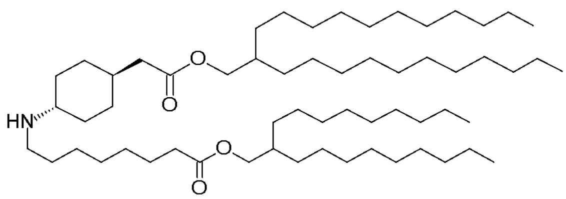 |
| Ex64 | 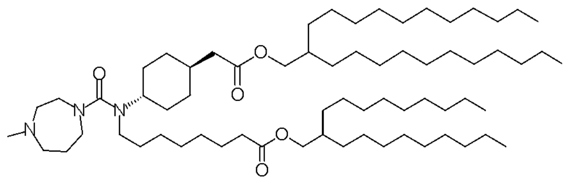 |
| Ex68 | 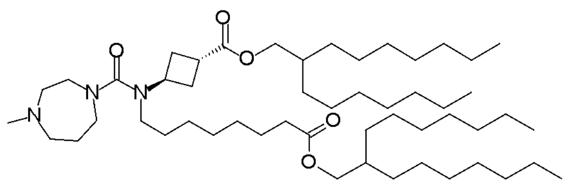 |
| PEx117 | 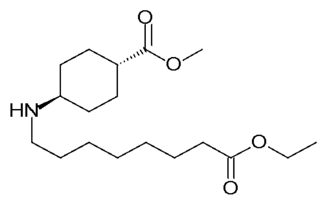 |
| PEx118 | 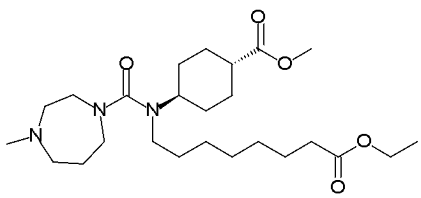 |

[Table 5-23]

| | |
|---|---|
| PEx119 | 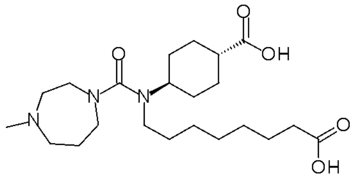 |
| Ex70 | 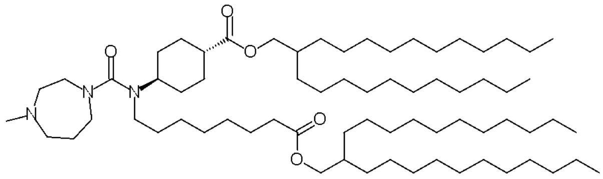 |
| Ex71 | 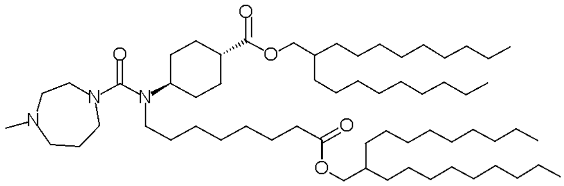 |
| Ex72 | 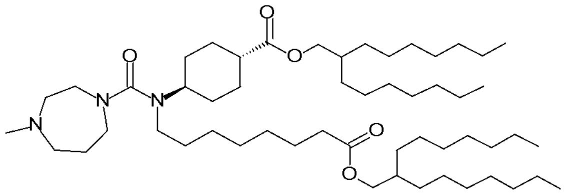 |
| PEx120 | 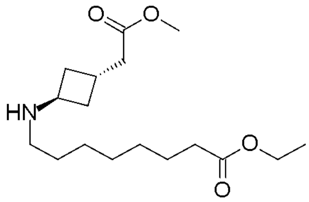 |
| PEx121 | 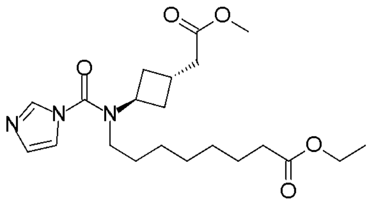 |
| PEx122 | 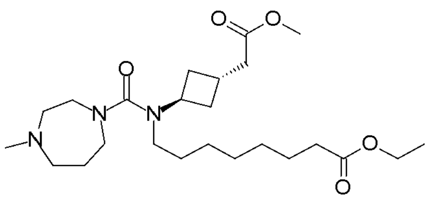 |
| PEx123 | 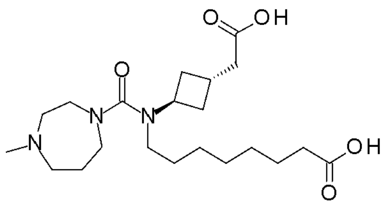 |

[Table 5-24]

| | |
|---|---|
| Ex73 | 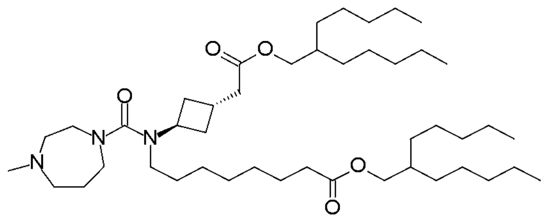 |
| Ex74 | 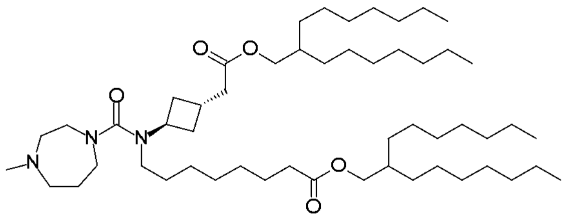 |
| Ex75 | 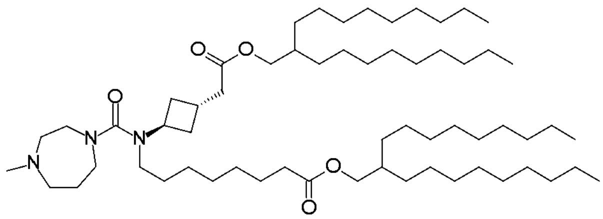 |
| Ex76 | 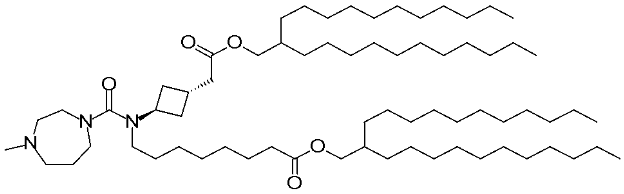 |
| PEx 124 | 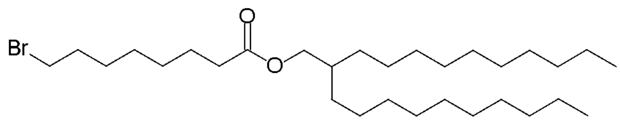 |
| PEx125 | 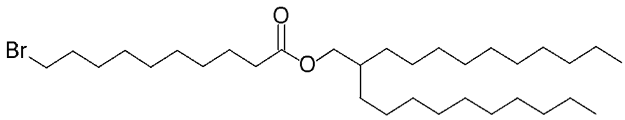 |
| Ex78 | 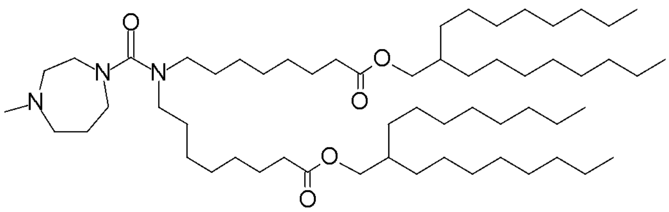 |
| Ex79 | 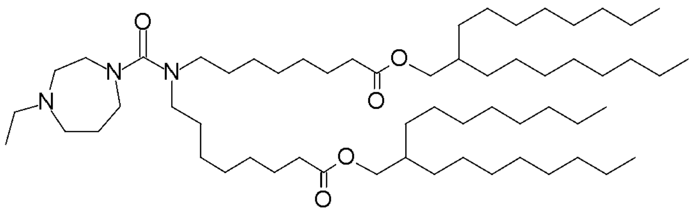 |

[Table 5-25]

| | |
|---|---|
| PEx126/I | 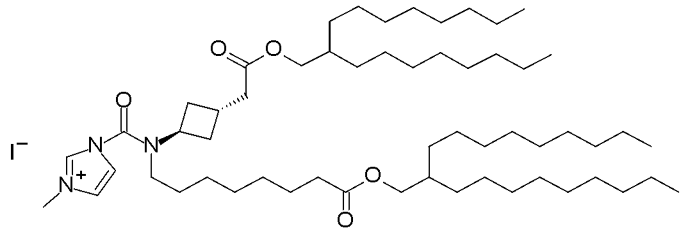 |
| PEx127 | 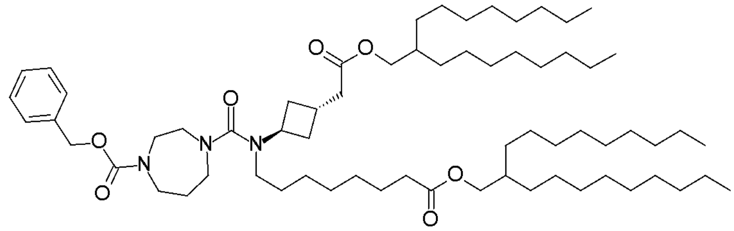 |
| PEx128 | 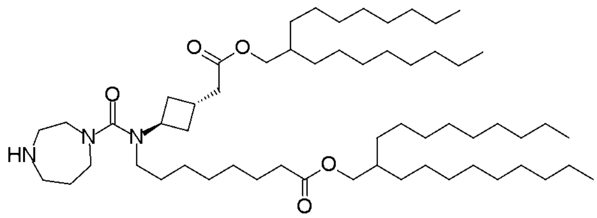 |
| Ex80 | 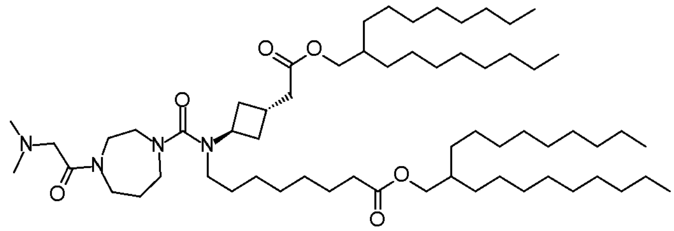 |
| Ex81 | 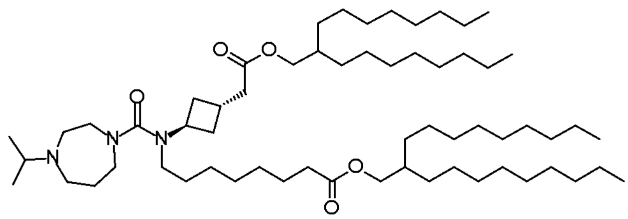 |
| Ex82 | 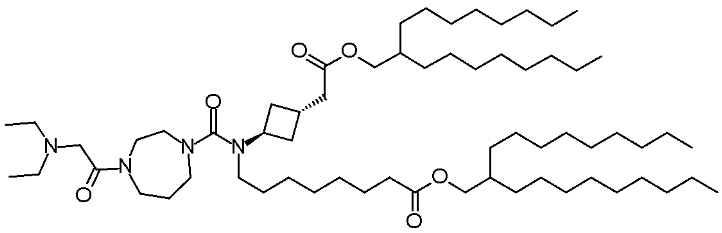 |
| Ex83 | 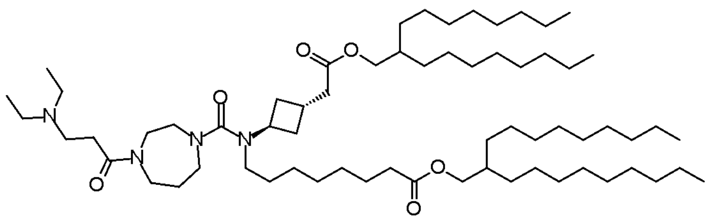 |

[Table 5-26]

| | |
|---|---|
| PEx129 | 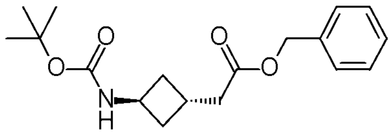 |
| PEx130 | 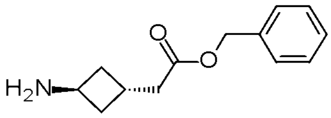 |
| PEx131 | 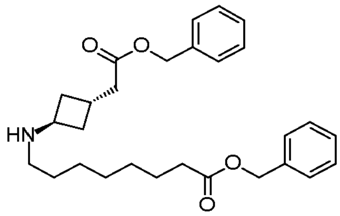 |
| PEx132 | 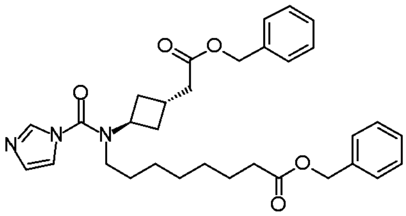 |
| PEx133/I | 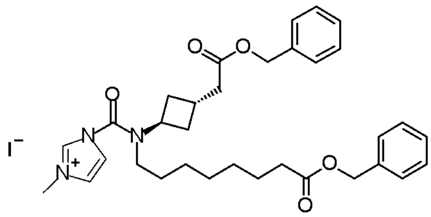 |
| PEx134 | 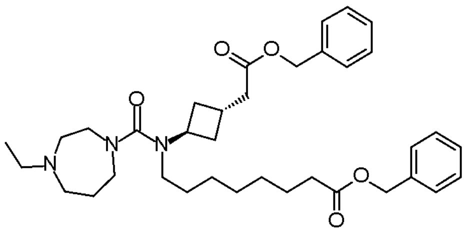 |
| PEx135 | 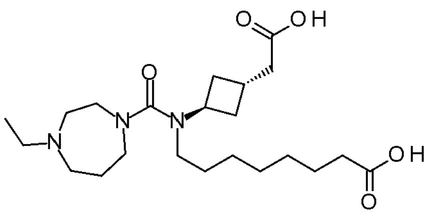 |
| Ex84 | 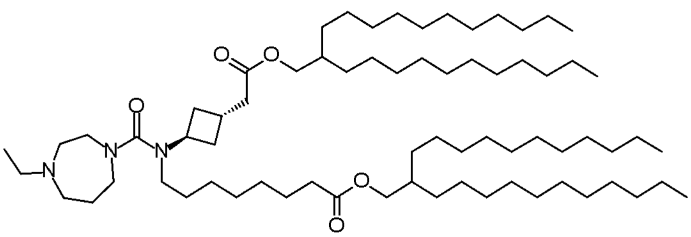 |

[Table 5-27]

| | |
|---|---|
| Ex85 | 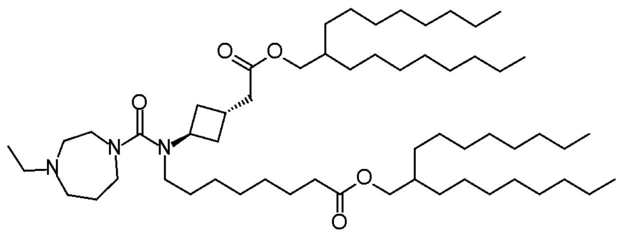 |
| PEx136 | 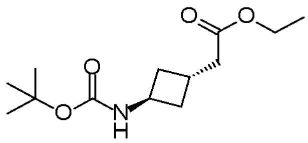 |
| PEx137 | 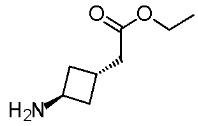 |
| PEx138 | 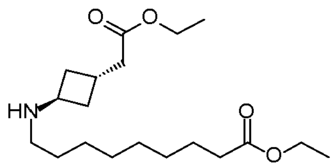 |
| PEx139 | 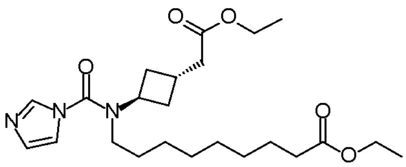 |
| PEx140/I | 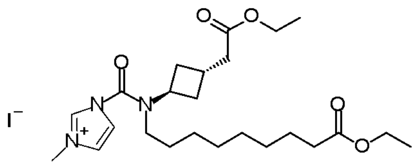 |
| PEx141 | 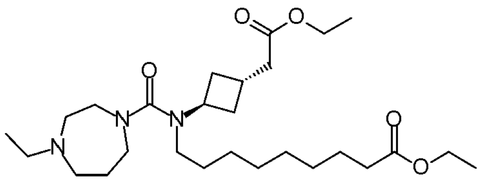 |
| PEx142 | 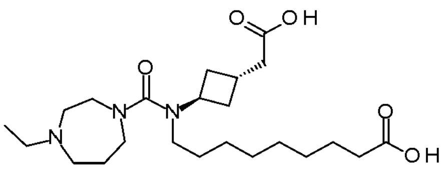 |
| Ex86 | 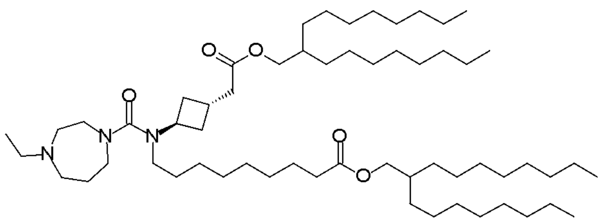 |

[Table 5-28]

| | |
|---|---|
| Ex87 | 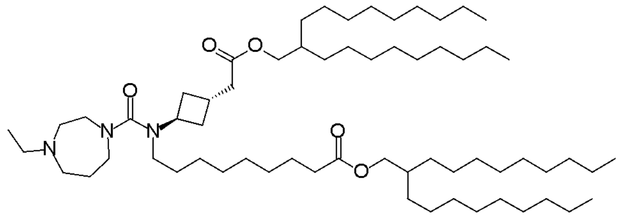 |
| Ex88 | 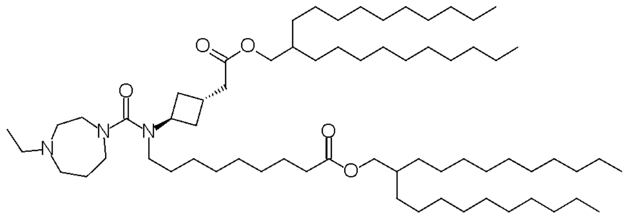 |
| Ex89 | 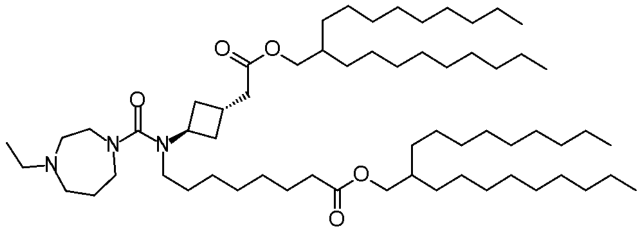 |
| PEx143 | 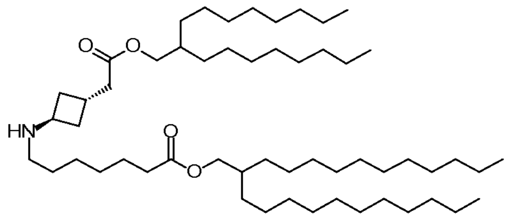 |
| Ex90 | 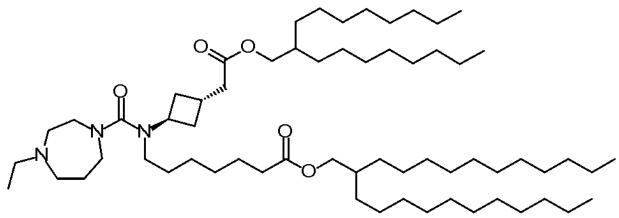 |
| PEx144 | 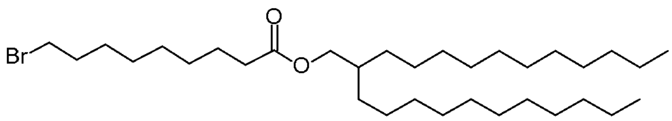 |
| PEx145 | 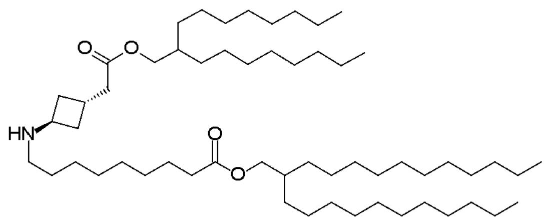 |

[Table 5-29]

| | |
|---|---|
| Ex91 | 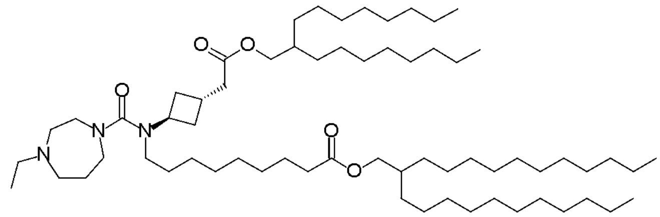 |
| PEx146 | 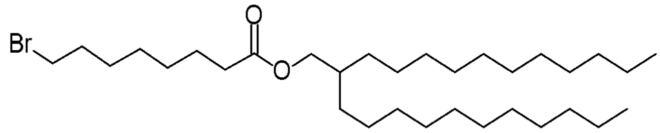 |
| PEx147 | 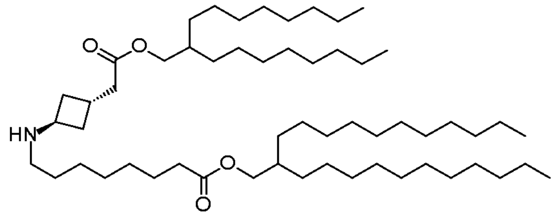 |
| Ex92 | 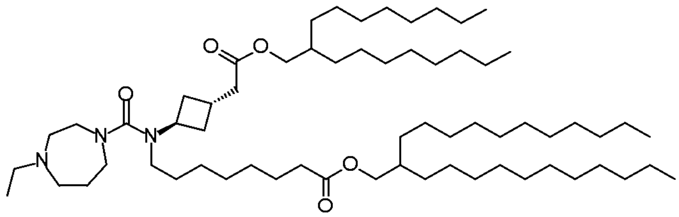 |
| PEx148 | 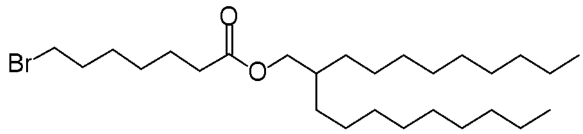 |
| PEx149 | 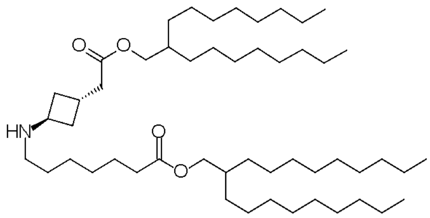 |
| Ex93 | 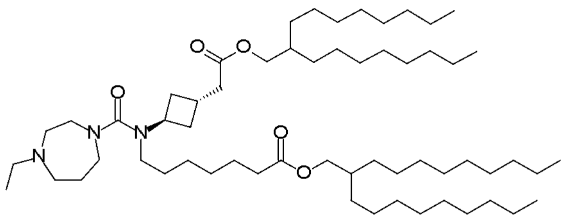 |

[Table 5-30]

| | |
|---|---|
| Ex94 | 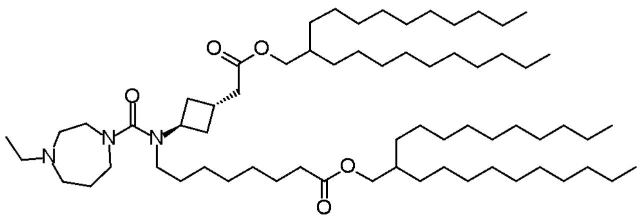 |
| PEx150 | 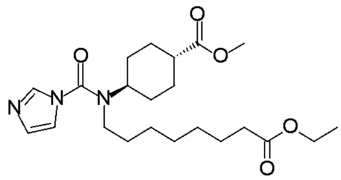 |
| PEx151/I | 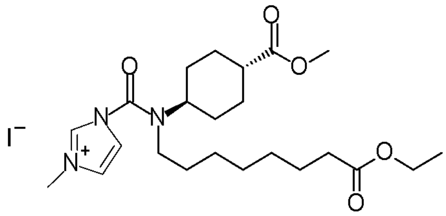 |
| PEx152 | 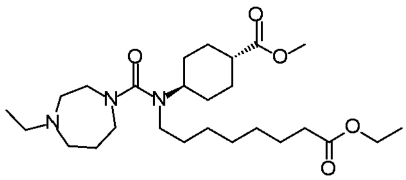 |
| PEx153 | 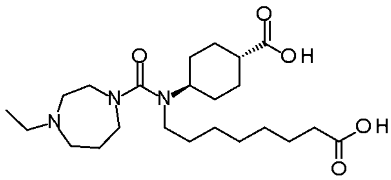 |
| Ex95 | 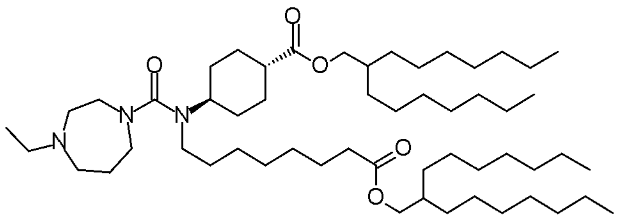 |
| Ex96 | 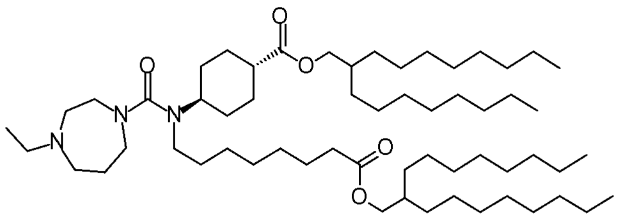 |

[Table 5-31]

| | |
|---|---|
| Ex97 | 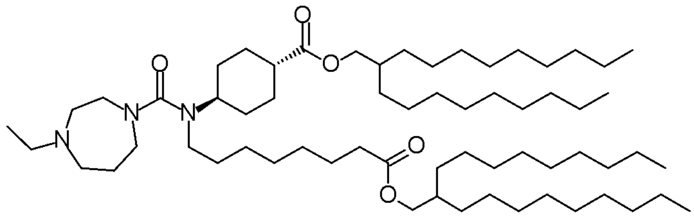 |

[Table 6-1]

| NUM | REF | DAT |
|---|---|---|
| PEx1 | PEx1 | ESI+:424.5 |
| PEx2/I | PEx2 | ESI+:438.5 |
| PEx3 | PEx3 | ESI+:542.7 |
| PEx4/Na | PEx4 | ESI+:486.5 |
| Ex1 | Ex1 | ESI+:1047.3<br>NMR ($CD_3OD$): 0.86-0.93 (m, 12H), 1.23-1.41 (m, 72H), 1.52-1.73 (m, 12H), 1.85-1.99 (m, 2H), 2.28-2.35 (m, 10H), 3.21-3.27 (m, 6H), 3.47-3.54 (m, 2H), 3.69-3.80 (m, 2H), 3.99 (d, *J*=5.50 Hz, 4H), 4.85-4.91 (m, 1H) |
| PEx5 | PEx5 | ESI+:486.5 |
| Ex2 | Ex2 | ESI+:1159.2<br>NMR ($CD_3OD$): 0.83-0.94 (m, 12H), 1.23-1.45 (m, 88H), 1.51-1.74 (m, 12H), 1.85-1.99 (m, 2H), 2.29-2.35 (m, 10H), 3.21-3.28 (m, 6H), 3.47-3.54 (m, 2H), 3.69-3.80 (m, 2H), 3.99 (d, *J*=5.66 Hz, 4H), 4.85-4.93 (m, 1H) |
| PEx6 | PEx6 | ESI+:523.5 |
| PEx7 | PEx7 | ESI+:947.2 |
| Ex3 | Ex3 | ESI+:1159.4<br>NMR ($CDCl_3$): 0.78-0.93 (m, 12H), 1.20-1.40 (m, 88H), 1.46-1.71 (m, 12H), 1.83-1.98 (m, 2H), 2.23-2.40 (m, 10H), 3.11-3.27 (m, 6H), 3.39-3.51 (m, 2H), 3.70-3.87 (m, 2H), 3.96 (d, *J*=5.81 Hz, 4H), 4.85-4.96 (m, 1H) |
| PEx8 | PEx8 | CI+: 503.1, 505.3 |
| PEx9 | PEx9 | ESI+: 504.5 |
| PEx10 | PEx10 | ESI+: 382.5 |
| PEx11 | PEx11 | ESI+: 805.0 |
| Ex4 | Ex4 | ESI+: 945.2<br>NMR ($CD_3OD$): 0.88-0.93 (m, 12H), 1.24-1.36 (m, 66H), 1.44 (quin, *J*=7.19 Hz, 2H), 1.58-1.66 (m, 4H), 1.90-1.96 (m, 2H), 1.97-2.04 (m, 2H), 2.17-2.25 (m, 2H), 2.31 (t, *J*=7.27 Hz, 2H), 2.35 (s, 3H), 2.47-2.57 (m, 3H), 2.58-2.62 (m, 2H), 2.69-2.73 (m, 2H), 3.12 (t, *J*=7.19 Hz, 2H), 3.42-3.48 (m, 4H), 3.99 (dd, *J*=3.21 Hz, 5.66 Hz, 4H), 4.05-4.13 (m, 1H) |
| PEx12 | PEx12 | ESI+:495.5 |
| PEx13 | PEx13 | ESI+:891.3 |
| Ex5 | Ex5 | ESI+: 1031.5<br>NMR ($CDCl_3$): 0.84-0.93 (m, 12H), 1.19-1.36 (m, 80H), 1.44-1.67 (m, 12H), 1.85-2.01 (m, 2 H), 2.29 (t, *J*=7.50 Hz, 4 H), 2.34-2.41 (m, 2 H), 2.53-2.77 (m, 3 H), 3.00-3.09 (m, 4 H), 3.41 (t, *J*=6.12 Hz, 2 H), 3.43-3.50 (m, 2 H), 3.96 (d, *J*=5.81 Hz, 4 H) |
| PEx14 | PEx6 | ESI+:439.4 |

[Table 6-2]

| | | |
|---|---|---|
| PEx15 | PEx7 | ESI+:779.1 |
| Ex6 | Ex3 | ESI+:990.8 |
| Ex7 | Ex3 | ESI+:1103.3 |
| PEx18 | PEx6 | ESI+:551.5 |
| PEx19 | PEx7 | ESI+:1003.3 |
| Ex8 | Ex3 | ESI+:1215.4 |
| PEx20 | PEx6 | ESI+:425.4 |
| PEx21 | PEx7 | ESI+:751.1 |
| Ex9 | Ex3 | ESI+:963.3 |
| PEx22 | PEx6 | ESI+:481.4 |
| PEx23 | PEx7 | ESI+:863.2 |
| Ex10 | Ex3 | ESI+:1075.0 |
| PEx24 | PEx6 | ESI+:537.5 |
| PEx25 | PEx7 | ESI+:975.3 |
| Ex11 | Ex3 | ESI+:1187.1 |
| PEx26 | PEx6 | ESI+:411.3 |
| PEx27 | PEx7 | ESI+:723.0 |
| Ex12 | Ex3 | ESI+:935.0 |
| PEx28 | PEx6 | ESI+:467.4 |
| PEx29 | PEx7 | ESI+:834.7 |
| Ex13 | Ex3 | ESI+:1047.1 |
| PEx31/HCl | PEx31 | ESI+:352.4 |
| PEx32 | PEx32 | ESI+:625.0 |
| Ex14 | Ex14 | ESI+:837.1 |
| PEx33 | PEx33 | ESI+:872.1 |
| PEx34 | PEx34 | ESI+:750.1 |
| Ex15 | Ex15 | ESI+:835.1 |
| PEx35 | PEx8 | ESI+:455.6 |
| PEx36 | PEx9 | ESI+:434.5 |
| PEx37/HCl | PEx34 | ESI+:312.5 |
| PEx38 | PEx32 | ESI+:665.0 |
| Ex16 | Ex14 | ESI+:877.2 |
| PEx39 | PEx9 | ESI+:462.5 |
| PEx40/HCl | PEx34 | ESI+:340.6 |
| PEx41 | PEx32 | ESI+:693.1 |
| Ex17 | Ex14 | ESI+:905.3 |
| PEx42 | PEx9 | ESI+:434.5 |
| PEx43/HCl | PEx34 | ESI+:312.5 |
| PEx44 | PEx32 | ESI+:665.1 |
| Ex18 | Ex14 | ESI+:877.2 |

(continued)

| | | |
|---|---|---|
| PEx45 | PEx9 | ESI+:420.5 |
| PEx46/HCl | PEx34 | ESI+:298.5 |

[Table 6-3]

| | | |
|---|---|---|
| PEx47 | PEx32 | ESI+:651.0 |
| Ex19 | Ex14 | ESI+:863.2 |
| PEx48 | PEx9 | ESI+:448.5 |
| PEx49/HCl | PEx34 | ESI+:326.5 |
| PEx50 | PEx32 | ESI+:679.1 |
| Ex20 | Ex14 | ESI+:891.2 |
| PEx51 | PEx9 | ESI+:476.5 |
| PEx52/HCl | PEx34 | ESI+:354.6 |
| PEx53 | PEx32 | ESI+:707.1 |
| Ex21 | Ex14 | ESI+:919.3 |
| PEx54 | PEx32 | ESI+:597.0 |
| PEx55 | PEx33 | ESI+:822.2 |
| PEx56/HCl | PEx34 | ESI+:722.1 |
| Ex22 | Ex15 | ESI+:807.0 |
| PEx57 | PEx9 | ESI+:408.4 |
| PEx58/HCl | PEx34 | ESI+:286.3 |
| PEx59 | PEx32 | ESI+:639.0 |
| PEx60 | PEx33 | ESI+:864.2 |
| PEx61/HCl | PEx34 | ESI+:764.1 |
| Ex23 | Ex15 | ESI+:849.1 |
| PEx62/HCl | PEx34 | ESI+:300.5 |
| PEx63 | PEx32 | ESI+:653.0 |
| PEx64 | PEx33 | ESI+:878.2 |
| PEx65/HCl | PEx34 | ESI+:778.2 |
| Ex24 | Ex15 | ESI+:863.2 |
| PEx66 | PEx8 | ESI+:443.4 |
| PEx67 | PEx7 | ESI+:695.1 |
| Ex25 | Ex14 | ESI+:907.2 |
| PEx68 | PEx8 | ESI+:457.4 |
| PEx69 | PEx7 | ESI+:723.1 |
| Ex26 | Ex14 | ESI+:935.3 |
| PEx70 | PEx8 | ESI+:527.7 |
| PEx71 | PEx7 | ESI+:863.3 |
| Ex27 | Ex3 | ESI+:1075.3 |
| PEx72 | PEx8 | ESI+:497.3 |
| PEx73 | PEx7 | ESI+:807.3 |

(continued)

| | | |
|---|---|---|
| Ex28 | Ex3 | ESI+:1019.2 |
| Ex29 | Ex29, PEx74 | ESI+:1215.6 |
| PEx74 | Ex29, PEx74 | ESI+:851.1 |
| Ex30 | Ex2 | ESI+:999.2 |

[Table 6-4]

| | | |
|---|---|---|
| Ex31 | Ex4 | ESI+:863.2 |
| Ex32 | Ex2 | ESI+:1103.0 |
| Ex33 | Ex1 | ESI+:991.3 |
| Ex34 | Ex1 | ESI+:935.0 |
| PEx75 | PEx7 | ESI+:358.4 |
| PEx76 | PEx1 | ESI+:452.5 |
| PEx77/I | PEx2 | ESI+:466.5 |
| PEx78 | PEx3 | ESI+:570.5 |
| PEx79/Na | PEx4 | ESI+:514.5 |
| Ex35 | Ex1 | ESI+:963.2 |
| Ex36 | Ex1 | ESI+:1075.0 |
| Ex37 | Ex1 | ESI+:1131.0 |
| Ex38 | Ex1 | ESI+:1187.1 |
| PEx80 | PEx8 | ESI+:567.3 |
| PEx81 | PEx7 | ESI+:947.4 |
| PEx82 | PEx82 | ESI+:1099.0 |
| Ex39 | Ex15 | ESI+:1184.2 |
| Ex40 | Ex1 | ESI+:1244.1 |
| Ex41 | Ex3 | ESI+:1100.2 |
| Ex42 | Ex3 | ESI+:1100.1 |
| PEx83 | PEx9 | ESI+:588.8 |
| PEx85 | PEx10 | ESI+:466.7 |
| PEx86 | PEx11 | ESI+:889.0 |
| Ex43 | Ex4 | ESI+:1029.3 |
| PEx87 | PEx87 | ESI+:877.3 |
| Ex44 | Ex3 | ESI+:1089.2 |
| PEx88 | PEx88 | ESI+:602.9 |
| PEx89 | PEx87 | ESI+:932.8 |
| Ex45 | Ex3 | ESI+:1145.2 |
| PEx90 | PEx87 | ESI+:961.4 |
| Ex46 | Ex3 | ESI+:1173.1 |
| Ex47 | Ex4 | ESI+:1086.8 |
| PEx94 | PEx94 | ESI+:898.8 |
| Ex48 | Ex48 | ESI+:1017.0 |

(continued)

| | | |
|---|---|---|
| PEx99 | PEx9 | ESI+:560.6 |
| PEx 100 | PEx34 | ESI+:438.6 |
| PEx101 | PEx11 | ESI+:861.0 |
| PEx102 | PEx1 | ESI+:954.9 |
| Ex51 | Ex48 | ESI+:1072.9 |
| PEx103 | PEx1 | ESI+:983.0 |
| Ex52 | Ex48 | ESI+:1101.1 |

[Table 6-5]

| | | |
|---|---|---|
| Ex56 | Ex56 | ESI+:958.9<br>NMR ($CDCl_3$): 0.85-0.92 (m, 12H), 1.07 (t, *J*=7.19 Hz, 3H), 1.18-1.69 (m, 72H), 1.86-2.03 (m, 4H), 2.15-2.24 (m, 2H), 2.25-2.32 (m, 2H), 2.46-2.57 (m, 5H), 2.58-2.65 (m, 2H), 2.68-2.74 (m, 2H), 3.00-3.06 (m, 2H), 3.38-3.48 (m, 4H), 3.93-4.00 (m, 4H), 4.03-4.12 (m, 1H) |
| Ex57 | Ex48 | ESI+:1043.0 |
| Ex58 | Ex48 | ESI+:1003.0 |
| PEx108 | PEx11 | ESI+:300.3 |
| PEx109 | PEx109 | ESI+:440.5 |
| PEx110 | PEx5 | ESI+:398.4 |
| Ex59 | Ex2 | ESI+:1093.1 |
| Ex60 | Ex2 | ESI+:958.9 |
| PEx111 | PEx9 | ESI+:560.7 |
| PEx112 | PEx10 | ESI+:438.5 |
| PEx113 | PEx11 | ESI+:860.9 |
| Ex63 | Ex4 | ESI+:1000.9 |
| PEx114 | PEx9 | ESI+:616.7 |
| PEx115 | PEx10 | ESI+:494.6 |
| PEx116 | PEx11 | ESI+:917.1 |
| Ex64 | Ex4 | ESI+:1056.9 |
| Ex68 | Ex2 | ESI+:846.8 |
| PEx117 | PEx117 | ESI+:328.3 |
| PEx118 | PEx118 | ESI+:468.4 |
| PEx119 | PEx119 | ESI+:426.3 |
| Ex70 | Ex2 | ESI+:1099.0 |
| Ex71 | Ex2 | ESI+:986.7 |
| Ex72 | Ex72 | ESI+:874.8<br>NMR ($CD_3OD$): 0.88-0.93 (m, 12H), 1.24-1.37 (m, 54H), 1.38-1.46 (m, 2H), 1.46-1.54 (m, 2H), 1.56-1.67 (m, 6H), 1.75-1.82 (m, 2H), 1.91-1.98 (m, 2H), 2.01-2.08 (m ,2H), 2.23-2.34 (m, 3H), 2.35 (s, 3H), 2.57-2.63 (m, 2H), 2.69-2.75 (m, 2H), 3.03 (t, *J*=7.04 Hz, 2H), 3.22-3.29 (m, 1H), 3.43-3.49 (m, 4H), 3.96-4.01 (m, 4H) |
| PEx120 | PEx11 | ESI+:314.3 |
| PEx121 | PEx1 | ESI+:408.4 |
| PEx122 | PEx122 | ESI+:454.3 |

(continued)

| | | |
|---|---|---|
| PEx123 | PEx5 | ESI+:412.4 |
| Ex73 | Ex2 | ESI+:748.7 |
| Ex74 | Ex2 | ESI+:860.9 |
| Ex75 | Ex2 | ESI+:972.7 |
| Ex76 | Ex2 | ESI+:1084.8 |

[Table 6-6]

| | | |
|---|---|---|
| PEx124 | PEx8 | ESI+:555.2 |
| PEx125 | PEx8 | ESI+:583.6 |
| Ex78 | Ex79 | ESI+:947.2 |
| Ex79 | Ex79 | ESI+:961.1 |
| PEx126/I | PEx126 | ESI+:913.0 |
| PEx127 | Ex56 | ESI+:1087.2 |
| PEx128 | PEx128 | ESI+:931.0 |
| Ex80 | Ex80 | ESI+:1016.0 |
| Ex81 | Ex81 | ESI+:973.1 |
| Ex82 | Ex80 | ESI+:1044.2 |
| Ex83 | Ex80 | ESI+:1058.0 |
| PEx129 | PEx9 | ESI+:342.2 |
| PEx130 | PEx10 | ESI+:220.2 |
| PEx131 | PEx11 | ESI+:452.5 |
| PEx132 | PEx1 | ESI+:546.5 |
| PEx133/I | PEx133 | ESI+:560.5 |
| PEx134 | Ex56 | ESI+:606.5 |
| PEx135 | PEx135 | ESI+:426.4 |
| Ex84 | Ex72 | ESI+:1099.2 |
| Ex85 | Ex72 | ESI+:931.0 |
| PEx136 | PEx9 | ESI+:280.3 |
| PEx137 | PEx10 | ESI+:158.1 |
| PEx138 | PEx11 | ESI+:342.4 |
| PEx139 | PEx139 | ESI+:436.4 |
| PEx140/I | PEx133 | ESI+:450.4 |
| PEx141 | Ex56 | ESI+:496.5 |
| PEx142 | PEx142 | ESI+:440.4 |
| Ex86 | Ex72 | ESI+:945.1 |
| Ex87 | Ex72 | ESI+:1001.2 |
| Ex88 | Ex72 | ESI+:1057.3 |
| Ex89 | Ex72 | ESI+:987.1 |
| PEx143 | PEx143 | ESI+:847.2 |
| Ex90 | Ex90 | ESI+:1001.2 |

(continued)

| | | |
|---|---|---|
| PEx144 | PEx8 | ESI+:595.3, 597.2 |
| PEx145 | PEx145 | ESI+:875.0 |
| Ex91 | Ex90 | ESI+:1029.2 |
| PEx146 | PEx8 | ESI+:581.3, 583.2 |
| PEx147 | PEx145 | ESI+:861.0 |
| Ex92 | Ex90 | ESI+:1015.1 |
| PEx148 | PEx8 | ESI+:513.2 |
| PEx149 | PEx143 | ESI+:791.1 |
| Ex93 | Ex90 | ESI+:945.3 |

[Table 6-7]

| | | |
|---|---|---|
| Ex94 | Ex72 | ESI+:1043.3 |
| PEx150 | PEx150 | ESI+:422.4 |
| PEx151/l | PEx126 | ESI+:436.4 |
| PEx152 | Ex56 | ESI+:482.6 |
| PEx153 | PEx119 | ESI+:440.4 |
| Ex95 | Ex72 | ESI+:889.0 |
| Ex96 | Ex72 | ESI+:945.0 |
| Ex97 | Ex72 | ESI+:1001.1 |

(Raw Materials of Nucleic Acid Lipid Nanoparticles)

In the Examples below, DSPC used was manufactured by NOF corporation (product name: COATSOME (registered trademark) MC-8080); DOPE used was manufactured by NOF corporation (product name: COATSOME (registered trademark) ME-8181); DOPC used was manufactured by NOF corporation (product name: COATSOME (registered trademark) MC-8181); DPPC used was manufactured by NOF corporation (product name: COATSOME (registered trademark) MC-6060); DHSM used was manufactured by Nippon Fine Chemical Co., Ltd.; SOPC used was manufactured by Avanti Polar Lipids (catalog No.: 850467P); DoPhPE used was manufactured by Avanti Polar Lipids (catalog No.: 999985P); cholesterol used was manufactured by Nippon Fine Chemical Co., Ltd. (product name: Cholesterol HP); β-sitosterol used was manufactured by Sigma-Aldrich Co. LLC (catalog No.: S1270); DMG-PEG2000 used was manufactured by NOF corporation (SUNBRIGHT (registered trademark) GM-020); the ceramide C8 PEG2000 Ceramide used was manufactured by Avanti Polar Lipids (catalog No.: 880170P); polyethylene glycol monostearate used was manufactured by FUJIFILM Wako Pure Chemical Corporation (catalog No.: 320-32585); and mRNA (Fluc mRNA, ND1 mRNA, eGFP mRNA) used were manufactured by TriLink BioTechnologies.

(Preparation of Nucleic Acid Lipid Nanoparticles)

For each of Ex1-L1 to Ex1-L36, a cationic lipid, phospholipid, sterol and PEGylated lipid were dissolved in ethanol at a lipid composition ratio and an N/P ratio shown in the table below, and an oil phase was obtained. FLuc mRNA was diluted with 10 mM citrate buffer (pH 4) to be 85 μg/mL, and thus an aqueous phase was obtained. The oil phase and the aqueous phase were mixed at a volume ratio of oil phase:aqueous phase=1:3 with a microfluidic device (NanoAssemblr (registered trademark), manufactured by Precision NanoSystems), and the mixture solution was diluted two-fold with phosphate-buffered saline (PBS). Thus, a dispersion of nucleic acid lipid nanoparticles was obtained. The dispersion was dialyzed with PBS to remove ethanol and concentrated through ultrafiltration to adjust to any concentration. Thus, nucleic acid lipid nanoparticles of Example lipids were obtained. Ex1-L1 to Ex1-L36 shown in NUM in the table below indicate Ex1-L1-Fluc mRNA to Ex1-L36-Fluc mRNA respectively.

[Table 7]

| NUM | Lipid composition (mol%) Ex1 \| DSPC \| Cholesterol \| DMG-PEG2000 | N/P ratio |
|---|---|---|
| Ex1-L1 | 50 \| 10 \| 38.5 \| 1.5 | 6 |
| Ex1-L1* | 50 \| 10 \| 38.5 \| 1.5 | 6 |
| Ex1-L2 | 40 \| 6 \| 52.5 \| 1.5 | 6 |
| Ex1-L3 | 40 \| 12 \| 46.5 \| 1.5 | 6 |
| Ex1-L4 | 40 \| 18 \| 40.5 \| 1.5 | 6 |
| Ex1-L5 | 42.5 \| 6 \| 50 \| 1.5 | 6 |
| Ex1-L6 | 42.5 \| 11.5 \| 44.5 \| 1.5 | 6 |
| Ex1-L7 | 42.5 \| 17.5 \| 38.5 \| 1.5 | 6 |
| Ex1-L8 | 45 \| 5.5 \| 48 \| 1.5 | 6 |
| Ex1-L9 | 45 \| 9 \| 44.5 \| 1.5 | 6 |
| Ex1-L10 | 45 \| 11 \| 42.5 \| 1.5 | 6 |
| Ex1-L11 | 45 \| 13 \| 40.5 \| 1.5 | 6 |
| Ex1-L12 | 45 \| 16.5 \| 37 \| 1.5 | 6 |
| Ex1-L13 | 47.5 \| 5.5 \| 45.5 \| 1.5 | 6 |
| Ex1-L14 | 47.5 \| 10.5 \| 40.5 \| 1.5 | 6 |
| Ex1-L15 | 47.5 \| 16 \| 35 \| 1.5 | 6 |
| Ex1-L16 | 50 \| 0 \| 48.5 \| 1.5 | 6 |
| Ex1-L17 | 50 \| 5 \| 43.5 \| 1.5 | 6 |
| Ex1-L18 | 50 \| 15 \| 33.5 \| 1.5 | 6 |
| Ex1-L19 | 60 \| 4 \| 34.5 \| 1.5 | 6 |
| Ex1-L20 | 60 \| 8 \| 30.5 \| 1.5 | 6 |
| Ex1-L21 | 60 \| 12 \| 26.5 \| 1.5 | 6 |
| Ex1-L22 | 45 \| 11 \| 43 \| 1 | 6 |
| Ex1-L23 | 45 \| 11 \| 42 \| 2 | 6 |
| Ex1-L24 | 45 \| 11 \| 41.5 \| 2.5 | 6 |
| Ex1-L25 | 60 \| 8 \| 29.5 \| 2.5 | 6 |
| Ex1-L26 | 50 \| 10 \| 38.5 \| 1.5 | 3 |
| Ex1-L27 | 50 \| 10 \| 38.5 \| 1.5 | 9 |

[Table 8]

| NUM | Lipid composition (mol%) 50 \| 10 \| 38.5 \| 1.5 | N/P ratio |
|---|---|---|
| Ex1-L28 | Ex1 \| DOPE \| Cholesterol \| DMG-PEG2000 | 6 |
| Ex1-L29 | Ex1 \| DOPC \| Cholesterol \| DMG-PEG2000 | 6 |
| Ex1-L30 | Ex1 \| DPPC \| Cholesterol \| DMG-PEG2000 | 6 |
| Ex1-L31 | Ex1 \| DHSM \| Cholesterol \| DMG-PEG2000 | 6 |
| Ex1-L32 | Ex1 \| SOPC \| Cholesterol \| DMG-PEG2000 | 6 |
| Ex1-L33 | Ex1 \| DoPhPE \| Cholesterol \| DMG-PEG2000 | 6 |
| Ex1-L34 | Ex1 \| DSPC \| β-sitosterol \| DMG-PEG2000 | 6 |

(continued)

| NUM | Lipid composition (mol%)<br>50 \| 10 \| 38.5 \| 1.5 | N/P ratio |
|---|---|---|
| Ex1-L35 | Ex1 \| DSPC \| Cholesterol \| C8 PEG2000 Ceramide | 6 |
| Ex1-L36 | Ex1 \| DSPC \| Cholesterol \| Polyethylene Glycol Monostearate | 6 |

As the fluorescence intensity of Ex1-L1-Fluc mRNA subjected to pharmacological evaluation in Test Example 1 was assumed as a reference, 1, the proportions of components with a fluorescence intensity of 0.01 or more and those with a fluorescence intensity of 0.1 or more were calculated. The results showed that: the composition ratios of Ex1 were 40.0 to 60.0 mol% and 40.0 to 60.0 mol% based on the total amount of the lipid nanoparticles; the composition ratios of phospholipid were 0 to 18.0 mol% and 0 to 18.0 mol% based on the total amount of the lipid nanoparticles; the composition ratios of sterol were 26.5 to 52.5 mol% and 26.5 to 52.5 mol% based on the total amount of the lipid nanoparticles; and the composition ratios of PEGylated lipid were 1.0 to 2.5 mol% and 1.0 to 2.0 mol% based on the total amount of the lipid nanoparticles.

(Raw Materials of ND1 Nucleic Acid Lipid Nanoparticles)

As NeuroD1 mRNA encoding human NeuroD1 protein, mRNA which had a base sequence (SEQ ID NO: 5) in that all the uridines were substituted with N1-methylpseudouridines in a base sequence (SEQ ID NO: 4) containing a 5' UTR derived from human $\alpha$-globin gene, a 3' UTR derived from human $\alpha$-globin gene, CDS of human NeuroD1 gene (SEQ ID NO: 3) and a poly(A) chain of 79 nucleotides and in which the 5' cap structure was Cap-1 (called "ND1-mRNA" below) was produced (outsourced to TriLink BioTechnologies). In this regard, in the base sequences of SEQ ID NOs: 4 and 5, the nucleotides at positions 1 to 3 correspond to a 5' terminal sequence suitable for the capping method using CleanCap (registered trademark) Reagent AG (TriLink BioTechnologies), positions 4 to 43 correspond to the 5' UTR, positions 44 to 1114 correspond to CDS of human NeuroD1 gene (SEQ ID NO: 3), positions 1115 to 1120 correspond to two successive stop codons, positions 1121 to 1231 correspond to the 3' UTR, and positions 1232 to 1310 correspond to the poly(A) chain. As the control, eGFP mRNA (TriLink BioTechnologies) was used.

(Preparation of ND1 Nucleic Acid Lipid Nanoparticles)

The ND1 nucleic acid lipid nanoparticles were prepared specifically by the following method. Example compound Ex1, DSPC, the cholesterol and DMG-PEG2000 were dissolved in ethanol at N/P ratio=6, and an oil phase was obtained. Using 10 mM citrate buffer (pH 4) containing the mRNA as an aqueous phase, the oil phase was added in such a manner that the volume ratio of the aqueous phase and the oil phase became oil phase:aqueous phase=1:3. After mixing with microfluidic device (NanoAssemblr (registered trademark), manufactured by Precision NanoSystems), the mixture solution was diluted two-fold with PBS, and thus, a dispersion of ND1 nucleic acid lipid nanoparticles was obtained. Ethanol was removed through dialysis of the dispersion. Subsequently, the dispersion was concentrated through ultrafiltration to obtain ND1 nucleic acid lipid nanoparticles adjusted to a concentration.

The lipid nanoparticles which were prepared as nucleic acid lipid nanoparticles using 10 mM citrate buffer (pH 4) and which contained (Ex1, DSPC, cholesterol, DMG-PEG2000 (mole ratio of 50/10/38.5/1.5) as components are called Ex1-L0-ND1 mRNA. Similarly, the lipid nanoparticles encapsulating eGFP mRNA and having the lipid composition of Ex1-L0 are called Ex1-L0-eGFP mRNA.

(Measurement of Particle Size)

The particle sizes of the nucleic acid lipid nanoparticles were measured using the nucleic acid lipid nanoparticle dispersions using a particle size analyzer (Zetasizer (registered trademark) Nano ZSP or Zetasizer (registered trademark) Nano S, manufactured by Malvern Panalytical).

(Evaluation of Encapsulation Efficiency of mRNA)

Regarding the nucleic acid lipid nanoparticles, the encapsulation efficiencies of mRNA in the nucleic acid lipid nanoparticle dispersions which were diluted to an mRNA concentration of around 400 to 3500 ng/mL were measured. Specifically, the nucleic acid lipid nanoparticles obtained in accordance with above were diluted with TE buffer (10 mM Tris/1 mM EDTA, pH 7.5 to 8.0), and the mRNA concentration (A) measured using Quant-iT RiboGreen RNA Reagent (manufactured by Thermo Fisher Scientific) was regarded as the mRNA existing in the external liquid of the nucleic acid

lipid nanoparticles. Moreover, the mRNA concentration (B) measured after diluting the nucleic acid lipid nanoparticles with 2% Triton X-100 was regarded as the total mRNA concentration in the composition. Subsequently, the encapsulation efficiency of mRNA was calculated by the following equation (F1).

$$\text{Encapsulation efficiency (\%)} = 100 - (A/B) \times 100 \ldots (F1)$$

[Table 9]

| NUM | Particle Size (nm) | Encapsulation efficiency (%) |
|---|---|---|
| Ex1-L0 | 101.2 | 99.3 |
| Ex2-L0 | 108.5 | 99.4 |
| Ex3-L0 | 104.0 | 99.4 |
| Ex4-L0 | 91.5 | 98.7 |
| Ex5-L0 | 111.0 | 99.1 |
| Ex6-L0 | 105.7 | 99.1 |
| Ex7-L0 | 110.8 | 99.4 |
| Ex8-L0 | 110.7 | 99.4 |
| Ex9-L0 | 90.6 | 99.3 |
| Fx10-L0 | 108.8 | 99.2 |
| Fx11-L0 | 118.0 | 99.1 |
| Ex12-L0 | 90.6 | 99.3 |
| Ex13-L0 | 108.8 | 99.3 |
| Ex14-L0 | 122.5 | 98.9 |
| Ex15-L0 | 228.6 | 96.4 |
| Ex16-L0 | 132.7 | 98.9 |
| Ex17-L0 | 135.7 | 98.9 |
| Ex18-L0 | 117.5 | 99.6 |
| Ex19-L0 | 142.1 | 99.5 |
| Ex20-L0 | 106.7 | 99.8 |
| Ex21-L0 | 97.6 | 99.8 |
| Ex22-L0 | 223.7 | 98.1 |
| Ex23-L0 | 205.1 | 98.8 |
| Ex24-L0 | 185.4 | 98.8 |
| Ex25-L0 | 102.7 | 98.8 |
| Ex26-L0 | 101.4 | 99.4 |
| Ex27-L0 | 109.5 | 99.5 |
| Ex28-L0 | 105.8 | 99.3 |
| Ex29-L0 | 120.3 | 99.6 |
| Ex30-L0 | 94.2 | 99.0 |
| Ex31-L0 | 105.1 | 98.5 |
| Ex32-L0 | 110.4 | 99.4 |
| Ex33-L0 | 90.9 | 99.2 |
| Ex34-L0 | 114.7 | 99.2 |

(continued)

| NUM | Particle Size (nm) | Encapsulation efficiency (%) |
|---|---|---|
| Ex35-L0 | 86.4 | 96.3 |
| Ex36-L0 | 116.5 | 99.4 |
| Ex37-L0 | 109.8 | 99.3 |
| Ex38-L0 | 112.3 | 99.2 |
| Ex39-L0 | 98.9 | 99.7 |
| Ex40-L0 | 120.4 | 99.2 |
| Ex41-L0 | 84.9 | 99.0 |
| Ex42-L0 | 87.2 | 98.9 |
| Ex43-L0 | 107.6 | 99.0 |
| Ex44-L0 | 89.8 | 98.8 |
| Ex45-L0 | 98.1 | 98.5 |
| Ex46-L0 | 90.1 | 99.2 |
| Ex47-L0 | 114.1 | 98.7 |
| Ex48-L0 | 91.1 | 99.0 |
| Ex51-L0 | 90.4 | 98.8 |
| Ex52-L0 | 105.9 | 98.9 |
| Ex56-L0 | 83.8 | 97.1 |
| Ex57-L0 | 88.9 | 98.2 |
| Ex58-L0 | 91.6 | 98.2 |
| Ex59-L0 | 97.9 | 98.4 |
| Ex60-L0 | 93.4 | 98.3 |
| Ex63-L0 | 90.1 | 97.6 |
| Ex64-L0 | 99.3 | 97.6 |
| Ex68-L0 | 82.7 | 97.6 |
| Ex70-L0 | 106.5 | 98.7 |
| Ex71-L0 | 93.8 | 97.9 |
| Ex72-L0 | 80.4 | 97.6 |
| Ex73-L0 | 98.1 | 96.3 |
| Ex74-L0 | 81.0 | 97.4 |
| Ex75-L0 | 91.4 | 98.4 |
| Ex76-L0 | 105.0 | 98.6 |
| Ex78-L0 | 95.3 | 98.6 |
| Ex79-L0 | 88.9 | 97.9 |
| Ex80-L0 | 110.8 | 99.1 |
| Ex81-L0 | 88.5 | 97.7 |
| Ex82-L0 | 105.3 | 97.9 |
| Ex83-L0 | 151.0 | 99.5 |
| Ex84-L0 | 93.4 | 98.7 |
| Ex85-L0 | 97.5 | 98.3 |

(continued)

| NUM | Particle Size (nm) | Encapsulation efficiency (%) |
|---|---|---|
| Ex86-L0 | 93.5 | 98.6 |
| Ex87-L0 | 91.8 | 98.6 |
| Ex88-L0 | 98.4 | 98.6 |
| Ex89-L0 | 100.9 | 98.4 |
| Ex90-L0 | 101.7 | 98.6 |
| Ex91-L0 | 97.8 | 98.5 |
| Ex92-L0 | 97.6 | 98.7 |
| Ex93-L0 | 96.4 | 97.6 |
| Ex94-L0 | 100.8 | 98.4 |
| Ex95-L0 | 82.4 | 97.7 |
| Ex96-L0 | 80.6 | 97.9 |
| Ex97-L0 | 85.3 | 98.1 |

[Table 10]

| NUM | Particle Size (nm) | Encapsulation efficiency (%) |
|---|---|---|
| Ex1-L1 | 98.4 | 98.9 |
| Ex1-L1* | 96.5 | 98.8 |
| Ex1-L2 | 93.4 | 98.9 |
| Ex1-L3 | 79.1 | 99.0 |
| Ex1-L4 | 70.4 | 98.6 |
| Ex1-L5 | 94.6 | 99.1 |
| Fx1-L6 | 80.5 | 99.1 |
| Ex1-L7 | 73.4 | 98.9 |
| Ex1-L8 | 105.0 | 98.8 |
| Ex1-L9 | 98.2 | 98.9 |
| Ex1-L10 | 89.8 | 98.5 |
| Ex1-L11 | 81.8 | 98.8 |
| Ex1-L12 | 77.7 | 98.5 |
| Ex1-L13 | 114.3 | 99.1 |
| Ex1-L14 | 93.2 | 98.9 |
| Ex1-L15 | 76.6 | 99.0 |
| Ex1-L16 | 127.2 | 99.0 |
| Ex1-L17 | 111.9 | 98.9 |
| Ex1-L18 | 83.5 | 98.7 |
| Ex1-L19 | 137.0 | 98.7 |
| Ex1-L20 | 120.3 | 98.5 |
| Ex1-L21 | 109.8 | 98.4 |
| Ex1-L22 | 107.7 | 99.0 |
| Ex1-L23 | 71.2 | 98.6 |

(continued)

| NUM | Particle Size (nm) | Encapsulation efficiency (%) |
|---|---|---|
| Ex1-L24 | 67.1 | 98.6 |
| Ex1-L25 | 92.2 | 97.5 |
| Ex1-L26 | 116.6 | 98.6 |
| Ex1-L27 | 89.4 | 98.9 |
| Ex1-L28 | 113.8 | 98.7 |
| Ex1-L29 | 98.9 | 98.4 |
| Ex1-L30 | 88.5 | 98.7 |
| Ex1-L31 | 105.5 | 98.7 |
| Ex1-L32 | 93.6 | 98.8 |
| Ex1-L33 | 127.0 | 98.4 |
| Ex1-L34 | 98.4 | 98.6 |
| Ex1-L35 | 95.3 | 98.0 |
| Ex1-L36 | 146.6 | 98.7 |

[Industrial Applicability]

The compound of the formula (I) or a salt thereof can form lipid nanoparticles and can encapsulate a nucleic acid to be delivered to an astrocyte. Using such a compound or a salt thereof, lipid nanoparticles containing a nucleic acid and a pharmaceutical containing lipid nanoparticles can be produced. Moreover, use of mRNA or the like as a nucleic acid is expected to be useful in the prevention and/or the treatment of an astrocyte-related disease.

[Sequence Listing Free Text]

SEQ ID NO: 1: Human NeuroD1 gene (CDS)
SEQ ID NO: 2: Human NeuroD1 protein
SEQ ID NO: 3: Human NeuroD1 gene (CDS)
SEQ ID NO: 4: Human NeuroD1 mRNA sequence
SEQ ID NO: 5: Human NeuroD1 mRNA sequence containing modified nucleotides

Sequence Listing

**Sequence Listing**

| | | |
|---|---|---|
| 1 | **Sequence Listing Information** | |
| 1-1 | File Name | A24001.xml |
| 1-2 | DTD Version | V1_3 |
| 1-3 | Software Name | WIPO Sequence |
| 1-4 | Software Version | 2.3.0 |
| 1-5 | Production Date | 2024-01-29 |
| 1-6 | Original free text language code | |
| 1-7 | Non English free text language code | |
| 2 | **General Information** | |
| 2-1 | Current application: IP Office | |
| 2-2 | Current application: Application number | |
| 2-3 | Current application: Filing date | |
| 2-4 | Current application: Applicant file reference | A24001 |
| 2-5 | Earliest priority application: IP Office | JP |
| 2-6 | Earliest priority application: Application number | 2023-013548 |
| 2-7 | Earliest priority application: Filing date | 2023-01-31 |
| 2-8en | Applicant name | Astellas Pharma Inc. |
| 2-8 | Applicant name: Name Latin | |
| 2-9 | Inventor name | |
| 2-9 | Inventor name: Name Latin | |
| 2-10en | Invention title | Carbamoyl lipid or urea lipid with cyclic amine group, and lipid nanoparticle and pharmaceutical composition containing the same |
| 2-10ja | | 環状アミンを有するカルバモイル脂質又はウレア脂質、それを含む脂 質ナノ粒子、及び医薬組成物 |
| Invention title | | |
| 2-11 | Sequence Total Quantity | 5 |
| **3-1** | **Sequences** | |
| 3-1-1 | Sequence Number [ID] | 1 |
| 3-1-2 | Molecule Type | DNA |
| 3-1-3 | Length | 1071 |
| | Features | **source** 1..1071 |
| 3-1-4-1 | Location/Qualifiers | mol_type= other DNA<br>organism= Homo sapiens |
| | NonEnglishQualifier Value | |

(continued)

| | | |
|---|---|---|
| **3-1** | **Sequences** | |
| | Features | **misc_feature** 1..1071 |
| 3-1-4-2 | Location/Qualifiers | note= Human NeuroD1 Gene (CDS) |
| | NonEnglishQualifier Value | |
| | **Residues** | |
| 3-1-5 | atgaccaaat cgtacagcga gagtgggctg atgggcgagc ctcagcccca aggtcctcca 60<br>agctggacag acgagtgtct cagttctcag gacgaggagc acgaggcaga caagaaggag 120<br>gacgacctcg aaaccatgaa cgcagaggag gactcactga ggaacggggg agaggaggag 180<br>gacgaagatg aggacctgga agaggaggaa gaagaggaag aggaggatga cgatcaaaag 240<br>cccaagagac gcggccccaa aaagaagaag atgactaagg ctcgcctgga gcgttttaaa 300<br>ttgagacgca tgaaggctaa cgcccgggag cggaaccgca tgcacggact gaacgcggcg 360<br>ctagacaacc tgcgcaaggt ggtgccttgc tattctaaga cgcagaagct gtccaaaatc 420<br>gagactctgc gcttggccaa gaactacatc tgggctctgt cggagatcct gcgctcaggc 480<br>aaaagcccag acctggtctc cttcgttcag acgctttgca agggcttatc ccaacccacc 540<br>accaacctgg ttgcgggctg cctgcaactc aatcctcgga cttttctgcc tgagcagaac 600<br>caggacatgc ccccccacct gccgacggcc agcgcttcct tccctgtaca cccctactcc 660<br>taccagtcgc ctgggctgcc cagtccgcct tacggtacca tggacagctc ccatgtcttc 720<br>cacgttaagc ctccgccgca cgcctacagc gcagcgctgg agcccttctt tgaaagccct 780<br>ctgactgatt gcaccagccc ttcctttgat ggacccctca gcccgccgct cagcatcaat 840<br>ggcaacttct ctttcaaaca cgaaccgtcc gccgagtttg agaaaaatta tgcctttacc 900<br>atgcactatc ctgcagcgac actggcaggg gcccaaagcc acggatcaat cttctcaggc 960<br>accgctgccc ctcgctgcga gatccccata gacaatatta tgtccttcga tagccattca 1020<br>catcatgagc gagtcatgag tgcccagctc aatgccatat ttcatgatta g 1071 | |
| **3-2** | **Sequences** | |
| 3-2-1 | Sequence Number [ID] | 2 |
| 3-2-2 | Molecule Type | AA |
| 3-2-3 | Length | 356 |
| | | source 1..356 |
| | Features | mol_type= protein |
| 3-2-4-1 | Location/Qualifiers | note= Human NeuroD1 Protein |
| | | organism= Homo sapiens |
| | NonEnglishQualifier Value | |
| | **Residues** | |
| 3-2-5 | MTKSYSESGL MGEPQPQGPP SWTDECLSSQ DEEHEADKKE DDLETMNAEE DSLRNGGEEE 60<br>DEDEDLEEEE EEEEEDDDQK PKRRGPKKKK MTKARLERFK LRRMKANARE RNRMHGLNAA 120<br>LDNLRKVVPC YSKTQKLSKI ETLRLAKNYI WALSEILRSG KSPDLVSFVQ TLCKGLSQPT 180<br>TNLVAGCLQL NPRTFLPEQN QDMPPHLPTA SASFPVHPYS YQSPGLPSPP YGTMDSSHVF 240<br>HVKPPPHAYS AALEPFFESP LTDCTSPSFD GPLSPPLSIN GNFSFKHEPS AEFEKNYAFT 300<br>MHYPAATLAG AQSHGSIFSG TAAPRCEIPI DNIMSFDSHS HHERVMSAQL NAIFHD 356 | |
| **3-3** | **Sequences** | |

(continued)

| | | |
|---|---|---|
| **3-2** | **Sequences** | |
| 3-3-1 | Sequence Number [ID] | 3 |
| 3-3-2 | Molecule Type | DNA |
| 3-3-3 | Length | 1071 |
| 3-3-4-1 | Features<br>Location/Qualifiers | **source** 1..1071<br>mol_type= other DNA<br>note= Human NeuroD1 Gene (CDS)<br>organism= Homo sapiens |
| | NonEnglishQualifier Value | |
| 3-3-5 | **Residues**<br>atgaccaaat cgtacagcga gagtgggctg atgggcgagc ctcagcccca aggtcctcca 60<br>agctggacag acgagtgtct cagttctcag gacgaggagc acgaggcaga caagaaggag 120<br>gacgacctcg aaaccatgaa cgcagaggag gactcactga ggaacggggg agaggaggag 180<br>gacgaagatg aggacctgga agaggaggaa gaagaggaag aggaggatga cgatcaaaag 240<br>cccaagagac gcggccccaa aaagaagaag atgactaagg ctcgcctgga gcgttttaaa 300<br>ttgagacgca tgaaggctaa cgcccgggag cggaaccgca tgcacggact gaacgcggcg 360<br>ctagacaacc tgcgcaaggt ggtgccttgc tattctaaga cgcagaagct gtccaaaatc 420<br>gagactctgc gcttggccaa gaactacatc tgggctctgt cggagatcct gcgctcaggc 480<br>aaaagcccag acctggtctc cttcgttcag acgctttgca agggcttatc ccaacccacc 540<br>accaacctgg ttgcgggctg cctgcaactc aatcctcgga cttttctgcc tgagcagaac 600<br>caggacatgc ccccccacct gccgacggcc agcgcttcct tccctgtaca cccctactcc 660<br>taccagtcgc ctgggctgcc cagtccgcct tacggtacca tggacagctc ccatgtcttc 720<br>cacgttaagc ctccgccgca cgcctacagc gcagcgctgg agcccttctt tgaaagccct 780<br>ctgactgatt gcaccagccc ttcctttgat ggacccctca gcccgccgct cagcatcaat 840<br>ggcaacttct ctttcaaaca cgaaccgtcc gccgagtttg agaaaaatta tgcctttacc 900<br>atgcactatc ctgcagcgac actggcaggg gcccaaagcc acggatcaat cttctcaggc 960<br>accgctgccc ctcgctgcga gatccccata gacaatatta tgtccttcga tagccattca 1020<br>catcatgagc gagtcatgag tgcccagctc aatgccatat ttcatgatta a 1071 | |
| **3-4** | **Sequences** | |
| 3-4-1 | Sequence Number [ID] | 4 |
| 3-4-2 | Molecule Type | RNA |
| 3-4-3 | Length | 1310 |
| 3-4-4-1 | Features<br>Location/Qualifiers | **source** 1..1310<br>mol_type= mRNA<br>note= Human NeuroD1 mRNA sequence<br>organism= Homo sapiens |
| | NonEnglishQualifier Value | |
| 3-4-5 | **Residues** | |

(continued)

| | | |
|---|---|---|
| **3-2** | **Sequences** | |
| | aggactcttc tggtccccac agactcagag agaacccgcc accatgacca aatcgtacag 60<br>cgagagtggg ctgatgggcg agcctcagcc ccaaggtcct ccaagctgga cagacgagtg 120<br>tctcagttct caggacgagg agcacgaggc agacaagaag gaggacgacc tcgaaaccat 180<br>gaacgcagag gaggactcac tgaggaacgg gggagaggag gaggacgaag atgaggacct 240<br>ggaagaggag gaagaagagg aagaggagga tgacgatcaa aagcccaaga gacgcggccc 300<br>caaaaagaag aagatgacta aggctcgcct ggagcgtttt aaattgagac gcatgaaggc 360<br>taacgcccgg gagcggaacc gcatgcacgg actgaacgcg gcgctagaca acctgcgcaa 420<br>ggtggtgcct tgctattcta agacgcagaa gctgtccaaa atcgagactc tgcgcttggc 480<br>caagaactac atctgggctc tgtcggagat cctgcgctca ggcaaaagcc cagacctggt 540<br>ctccttcgtt cagacgcttt gcaagggctt atcccaaccc accaccaacc tggttgcggg 600<br>ctgcctgcaa ctcaatcctc ggacttttct gcctgagcag aaccaggaca tgccccccca 660<br>cctgccgacg gccagcgctt ccttccctgt acaccccctac tcctaccagt cgcctgggct 720<br>gcccagtccg ccttacggta ccatggacag ctcccatgtc ttccacgtta agcctccgcc 780<br>gcacgcctac agcgcagcgc tggagccctt ctttgaaagc cctctgactg attgcaccag 840<br>cccttccttt gatggacccc tcagcccgcc gctcagcatc aatggcaact tctctttcaa 900<br>acacgaaccg tccgccgagt ttgagaaaaa ttatgccttt accatgcact atcctgcagc 960<br><br>gacactggca ggggcccaaa gccacggatc aatcttctca ggcaccgctg cccctcgctg 1020<br>cgagatcccc atagacaata ttatgtcctt cgatagccat tcacatcatg agcgagtcat 1080<br>gagtgcccag ctcaatgcca tatttcatga ttaatgatag gctggagcct cggtggccat 1140<br>gcttcttgcc ccttgggcct ccccccagcc cctcctcccc ttcctgcacc cgtacccccg 1200<br>tggtctttga ataaagtctg agtgggcggc aaaaaaaaaa aaaaaaaaaa aaaaaaaaaa 1260<br>aaaaaaaaaa aaaaaaaaaa aaaaaaaaaa aaaaaaaaaa aaaaaaaaaa 1310 | |
| **3-5** | **Sequences** | |
| 3-5-1 | Sequence Number [ID] | 5 |
| 3-5-2 | Molecule Type | RNA |
| 3-5-3 | Length | 1310 |
| 3-5-4-1 | Features<br>Location/Qualifiers<br>NonEnglishQualifier Value | **source** 1..1310<br>mol_type= other RNA<br>note= Human NeuroD1 mRNA containing modified nucleotides organism= synthetic construct |
| 3-5-4-2 | Features<br>Location/Qualifiers<br>NonEnglishQualifier Value | **modified_base** 6..1223<br>mod_base= m1f<br>note= all uridine bases are modified into N1-methyl pseudouridine(m1f) |
| | Residues | |

(continued)

| 3-5 | Sequences |
|---|---|
| 3-5-5 | aggactcttc tggtccccac agactcagag agaacccgcc accatgacca aatcgtacag 60<br>cgagagtggg ctgatgggcg agcctcagcc ccaaggtcct ccaagctgga cagacgagtg 120<br>tctcagttct caggacgagg agcacgaggc agacaagaag gaggacgacc tcgaaaccat 180<br>gaacgcagag gaggactcac tgaggaacgg gggagaggag gaggacgaag atgaggacct 240<br>ggaagaggag gaagaagagg aagaggagga tgacgatcaa aagcccaaga gacgcggccc 300<br>caaaaagaag aagatgacta aggctcgcct ggagcgtttt aaattgagac gcatgaaggc 360<br>taacgcccgg gagcggaacc gcatgcacgg actgaacgcg gcgctagaca acctgcgcaa 420<br>ggtggtgcct tgctattcta agacgcagaa gctgtccaaa atcgagactc tgcgcttggc 480<br>caagaactac atctgggctc tgtcggagat cctgcgctca ggcaaaagcc cagacctggt 540<br>ctccttcgtt cagacgcttt gcaagggctt atcccaaccc accaccaacc tggttgcggg 600<br>ctgcctgcaa ctcaatcctc ggacttttct gcctgagcag aaccaggaca tgcccccca 660<br>cctgccgacg gccagcgctt ccttccctgt acacccctac tcctaccagt cgcctgggct 720<br>gcccagtccg ccttacggta ccatggacag ctcccatgtc ttccacgtta agcctccgcc 780<br>gcacgcctac agcgcagcgc tggagccctt ctttgaaagc cctctgactg attgcaccag 840<br>cccttccttt gatggacccc tcagcccgcc gctcagcatc aatggcaact tctctttcaa 900<br>acacgaaccg tccgccgagt ttgagaaaaa ttatgccttt accatgcact atcctgcagc 960<br>gacactggca ggggcccaaa gccacggatc aatcttctca ggcaccgctg cccctcgctg 1020<br>cgagatcccc atagacaata ttatgtcctt cgatagccat tcacatcatg agcgagtcat 1080<br>gagtgcccag ctcaatgcca tatttcatga ttaatgatag gctggagcct cggtggccat 1140<br>gcttcttgcc ccttgggcct ccccccagcc cctcctcccc ttcctgcacc cgtacccccg 1200<br>tggtctttga ataaagtctg agtgggcggc aaaaaaaaaa aaaaaaaaaa aaaaaaaaaa 1260<br>aaaaaaaaaa aaaaaaaaaa aaaaaaaaaa aaaaaaaaaa aaaaaaaaaa 1310 |

**Claims**

1. A compound of a formula (I) or a salt thereof:

[Chem. 1]

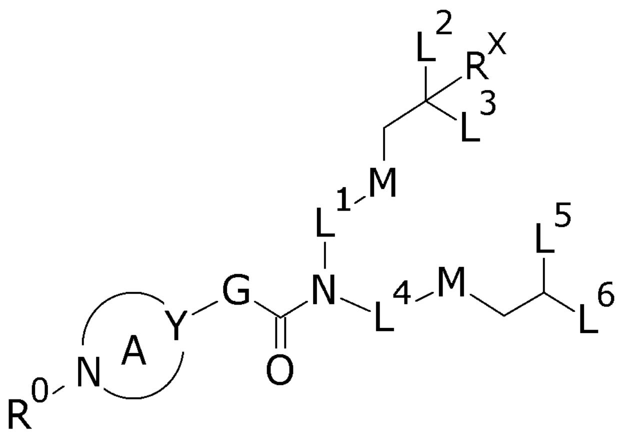

(I)

(in the formula,

$L^1$ is a $C_{5-10}$ alkylene, $CH_2$ or $-C_{3-6}$ cycloalkanediyl-$(CH_2)_p$-,
wherein p is 0 or 1,
$L^2$ is H or a $C_{5-15}$ alkyl,
wherein $L^2$ is H when $L^1$ is $CH_2$,
$L^3$ is a $C_{5-15}$ alkyl,
$R^X$ is H or adamantyl formed together with $L^2$, $L^3$ and the carbon atom to which they are bonded,
$L^4$ is a $C_{5-10}$ alkylene,
$L^5$ and $L^6$ are the same or different and are a $C_{5-15}$ alkyl,
M's are each independently C(=O)O or OC(=O),
the ring A is a group selected from the group consisting of

i) a 5- to 7-membered ring group composed of three to five carbon atoms, one N and one Y,

wherein one of the hydrocarbon chains between N and Y is $CH_2CH_2$, and the other is $(CH_2)_n$, wherein n is an integer of 1, 2 or 3,

ii) a diazaspirononane ring group and
iii) a quinuclidine ring group,

$R^0$ is $(C_{1-6}$ alkyl$)_2$N-$C_{1-6}$ alkylene-C(=O)-, (cyclic amino)-$C_{1-6}$ alkylene-C(=O)- or a $C_{1-6}$ alkyl,
wherein $R^0$ does not exist when the ring A is a quinuclidine ring group, and
the combination of G and Y (G, Y) is (O, CH), ($CH_2$, CH) or (a bond, N),
wherein Y is N when the ring A is a diazaspirononane ring group, and Y is CH when the ring A is a quinuclidine ring group.)

2. The compound or the salt thereof according to claim 1, wherein the combination of G and Y (G, Y) is (O, CH) or ($CH_2$, CH).

3. The compound or the salt thereof according to claim 2, wherein the combination of G and Y (G, Y) is (O, CH).

4. The compound or the salt thereof according to claim 3, wherein M is C(=O)O.

5. The compound or the salt thereof according to claim 4, wherein the ring A is a 6-membered ring composed of four carbon atoms, one N and one Y, one of the hydrocarbon chains between N and Y is $CH_2CH_2$, the other is $(CH_2)_n$, and n

is 2.

6. The compound or the salt thereof according to claim 5, wherein $L^1$ is a $C_{6-9}$ alkylene, $L^2$ is a $C_{6-14}$ alkyl, $L^3$ is a $C_{6-14}$ alkyl, $L^4$ is a $C_{6-9}$ alkylene, $L^5$ is a $C_{6-14}$ alkyl, and $L^6$ is a $C_{6-14}$ alkyl.

7. The compound or the salt thereof according to claim 6, wherein $R^0$ is $(CH_3)_2N-CH_2-C(=O)-$.

8. The compound or the salt thereof according to claim 1, wherein the combination of G and Y (G, Y) is (a bond, N).

9. The compound or the salt thereof according to claim 8, wherein M is C(=O)O.

10. The compound or the salt thereof according to claim 9, wherein the ring A is a 7-membered ring composed of five carbon atoms, one N and one Y, wherein one of the hydrocarbon chains between N and Y is $CH_2CH_2$, the other is $(CH_2)_n$, and n is 3.

11. The compound or the salt thereof according to claim 10, wherein $L^1$ is $-C_{3-6}$ cycloalkanediyl-$(CH_2)_p$-.

12. The compound or the salt thereof according to claim 10, wherein $L^1$ is a $C_{6-9}$ alkylene, $L^2$ is a $C_{6-14}$ alkyl, $L^3$ is a $C_{6-14}$ alkyl, $R^x$ is H, $L^4$ is a $C_{6-9}$ alkylene, $L^3$ is a $C_{6-14}$ alkyl, and $L^6$ is a $C_{6-14}$ alkyl.

13. The compound or the salt thereof according to claim 11 or 12, wherein $R^0$ is $CH_3$ or $C_2H_5$.

14. The compound or the salt thereof according to claim 1, wherein the compound is selected from the group consisting of

bis(2-nonylundecyl) 7,7-[({[1-(N,N-dimethylglycyl)piperidin-4-yl]oxy}carbonyl)azanediyl]di(heptanoate),
bis(2-undecyltridecyl) 7,7-[({[1-(N,N-dimethylglycyl)piperidin-4-yl]oxy}carbonyl)azanediyl]di(heptanoate),
bis(2-decyltetradecyl) 7,7'-[({[1-(N,N-dimethylglycyl)piperidin-4-yl]oxy}carbonyl)azanediyl]di(heptanoate),
2-octyldecyl 8-[({[1-(N,N-dimethylglycyl)piperidin-4-yl]oxy} carbonyl) {7-oxo-7-[(2-undecyltridecyl)oxy]heptyl} amino]octanoate,
2-nonylundecyl 8-{[(1r,3r)-3-{2-[(2-decyldodecyl)oxy]-2-oxoethyl}cyclobutyl]({[1-(N,N-dimethylglycyl)piperidin-4-yl]oxy}carbonyl)amino}octanoate and
2-nonylundecyl 8-{({[1-(N,N-dimethylglycyl)piperidin-4-yl]oxy}carbonyl)[(1r,3r)-3-{2-oxo-2-[(2-undecyltridecyl)oxy]ethyl}cyclobutyl]amino}octanoate.

15. The compound or the salt thereof according to claim 1, wherein the compound is selected from the group consisting of

2-nonylundecyl 8-{(4-methyl-1,4-diazepane-1-carbonyl)[(1r,3r)-3-{2-[(2-octyldecyl)oxy]-2-oxoethyl}cyclobutyl]amino}octanoate,
bis(2-octyldodecyl) 9,9'-[(4-methyl-1,4-diazepane-1-carbonyl)azanediyl]di(nonanoate),
2-nonylundecyl 8-{(4-ethyl-1,4-diazepane-1-carbonyl)[(1r,3r)-3-{2-[(2-octyldecyl)oxy]-2-oxoethyl}cyclobutyl]amino}octanoate,
2-nonylundecyl 8-{(4-methyl-1,4-diazepane-1-carbonyl)[(1r,4r)-4-{2-oxo-2-[(2-undecyltridecyl)oxy]ethyl}cyclohexyl]amino}octanoate and
2-heptylnonyl (1r,4r)-4-[{8-[(2-heptylnonyl)oxy]-8-oxooctyl}(4-methyl-1,4-diazepane-1-carbonyl)amino]cyclohexane-1-carboxylate.

16. Lipid nanoparticles containing the compound or the salt thereof according to claim 1.

17. The Lipid nanoparticles containing the compound or the salt thereof according to claim 1, a neutral lipid and a PEGylated lipid.

18. The lipid nanoparticles according to claim 17, wherein the phospholipid is DSPC, DOPE, DOPC, DPPC, DHSM, SOPC or DoPhPE.

19. The lipid nanoparticles according to claim 17, wherein the sterol is cholesterol or β-sitosterol.

20. The lipid nanoparticles according to claim 17, wherein the PEGylated lipid is DMG-PEG2000, C8 PEG2000 ceramide or polyethylene glycol monostearate.

**21.** The lipid nanoparticles according to claim 17 which encapsulate a nucleic acid.

**22.** The lipid nanoparticles according to claim 21, wherein the nucleic acid is mRNA.

**23.** The lipid nanoparticles according to claim 21 containing 40.0 to 60.0 mol% of the compound or the salt thereof according to claim 1, 37.5 to 59.0 mol% of the neutral lipid and 1.0 to 2.5 mol% of the PEGylated lipid based on the total amount of the lipid nanoparticles.

**24.** The lipid nanoparticles according to claim 22 containing 40.0 to 60.0 mol% of the compound or the salt thereof according to claim 1, 38.0 to 59.0 mol% of the neutral lipid and 1.0 to 2.0 mol% of the PEGylated lipid based on the total amount of the lipid nanoparticles.

**25.** The lipid nanoparticles according to claim 22 which can express a protein in an astrocyte.

**26.** The lipid nanoparticles according to claim 21, wherein the nucleic acid is a nucleic acid which is useful for preventing and/or treating an astrocyte-related disease.

**27.** The Lipid nanoparticles encapsulating a nucleic acid according to claim 22,

wherein the nucleic acid is mRNA encoding NeuroD1 protein, and
the lipid nanoparticles contain a compound of a formula (I) or a salt thereof which is bis(2-nonylundecyl) 7,7'-[({[1-(N,N-dimethylglycyl)piperidin-4-yl]oxy}carbonyl)azanediyl]di(heptanoate) or a salt thereof, neutral lipids which are DSPC and cholesterol and a PEGylated lipid which is DMG-PEG2000.

**28.** The lipid nanoparticles encapsulating mRNA according to claim 22 or 27,
wherein the nucleic acid is mRNA encoding NeuroD1 protein containing a base sequence encoding a protein having the amino acid sequence of SEQ ID NO: 2.

**29.** A pharmaceutical composition containing the lipid nanoparticles according to claim 21 or 27 and one or more pharmaceutically acceptable pharmaceutical additives.

**30.** The pharmaceutical composition according to claim 29 for preventing or treating an astrocyte-related disease.

**31.** Use of the lipid nanoparticles according to claim 21 or 27 for producing a pharmaceutical composition for preventing and/or treating an astrocyte-related disease.

**32.** The lipid nanoparticles according to claim 21 or 27 for use in preventing and/or treating an astrocyte-related disease.

**33.** The use of the lipid nanoparticles according to claim 21 or 27 for preventing and/or treating an astrocyte-related disease.

**34.** A method for preventing and/or treating an astrocyte-related disease including administering an effective amount of the lipid nanoparticles according to claim 21 or 27.

Fig. 1

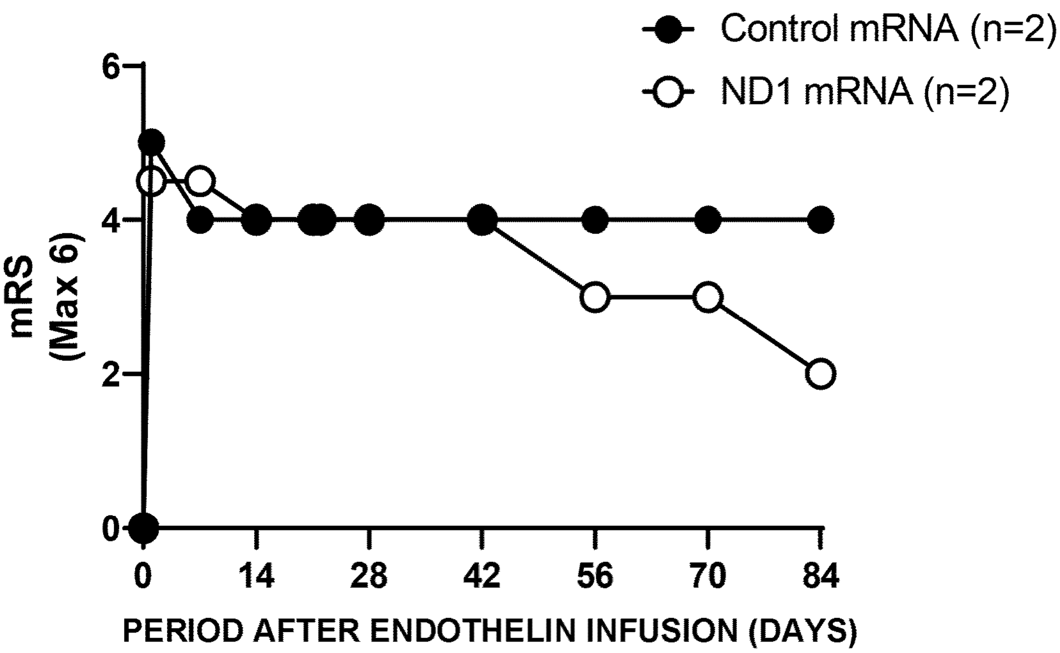
Control mRNA (n=2)
ND1 mRNA (n=2)
mRS
(Max 6)
6
4
2
0
0
14
28
42
56
70
84
PERIOD AFTER ENDOTHELIN INFUSION (DAYS)

## INTERNATIONAL SEARCH REPORT

International application No. **PCT/JP2024/002768**

**A. CLASSIFICATION OF SUBJECT MATTER**

***C07D 211/46***(2006.01)i; ***A61K 9/51***(2006.01)i; ***A61K 9/127***(2006.01)i; ***A61K 31/7088***(2006.01)i; ***A61K 47/22***(2006.01)i; ***A61K 47/44***(2017.01)i; ***A61K 48/00***(2006.01)i; ***A61P 21/00***(2006.01)i; ***A61P 25/00***(2006.01)i; ***A61P 43/00***(2006.01)i; ***C07D 207/12***(2006.01)i; ***C07D 211/34***(2006.01)i; ***C07D 243/08***(2006.01)i; ***C07D 453/00***(2006.01)i; ***C07D 453/02***(2006.01)i; ***C07D 471/10***(2006.01)i; ***C12N 15/12***(2006.01)i; ***C12N 15/88***(2006.01)i

FI: C07D211/46 CSP; A61K9/127; A61K9/51; A61K31/7088; A61K47/22; A61K47/44; A61K48/00; A61P21/00; A61P25/00; A61P43/00 111; C07D207/12; C07D211/34; C07D243/08 506; C07D243/08 507; C07D453/00; C07D453/02; C07D471/10 101; C12N15/12 ZNA; C12N15/88 Z

According to International Patent Classification (IPC) or to both national classification and IPC

**B. FIELDS SEARCHED**

Minimum documentation searched (classification system followed by classification symbols)

C07D211/46; A61K9/51; A61K9/127; A61K31/7088; A61K47/22; A61K47/44; A61K48/00; A61P21/00; A61P25/00; A61P43/00; C07D207/12; C07D211/34; C07D243/08; C07D453/00; C07D453/02; C07D471/10; C12N15/12; C12N15/88

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| Y | WO 2022/173531 A1 (ONCORUS, INC.) 18 August 2022 (2022-08-18) claims, table 1, compound no. CAT7, CAT17, CAT19 to CAT23, CAT25, CAT28, examples | 1-34 |
| Y | JP 2015-523990 A (NITTO DENKO CORPORATION) 20 August 2015 (2015-08-20) claims, definition of X in formula I, examples | 1-34 |
| Y | WO 2021/204175 A1 (SUZHOU ABOGEN BIOSCIENCES CO., LTD.) 14 October 2021 (2021-10-14) claims, table 1, compound 11, examples | 1-34 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **09 April 2024** | **23 April 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**

International application No.
**PCT/JP2024/002768**

**C. DOCUMENTS CONSIDERED TO BE RELEVANT**

| Category* | Citation of document, with indication, where appropriate, of the relevant passages | Relevant to claim No. |
|---|---|---|
| Y | WO 2020/246581 A1 (FUJIFILM CORPORATION) 10 December 2020 (2020-12-10) claims, examples 137, 138, 409, 410, etc. | 1-34 |
| Y | WO 2022/081750 A1 (GEORGE MASON RESEARCH FOUNDATION, INC.) 21 April 2022 (2022-04-21) table 1c, no. 19-24, 26-43 in table 10, etc. | 1-34 |
| Y | WO 2022/168884 A1 (SHIONOGI & CO., LTD.) 11 August 2022 (2022-08-11) claims, compounds I-1 to I-4, I-70, etc. preparation examples, test examples | 1-34 |
| Y | WO 2022/037652 A1 (SUZHOU ABOGEN BIOSCIENCES CO., LTD.) 24 February 2022 (2022-02-24) claims, definition of formula (VII), table 1, compounds 9, 38, 51, 52, 61, 63, examples | 7, 14 |
| Y | JP 2022-187826 A (KABUSHIKI KAISHA TOSHIBA) 20 December 2022 (2022-12-20) claims, compounds in formulae (1-02), (1-06), (1-07), (1-11) | 8-13, 15 |
| Y | WO 2021/108609 A1 (THE PENN STATE RESEARCH FOUNDATION) 03 June 2021 (2021-06-03) claims, paragraphs [00195]-[00199], examples | 25-28, 30-34 |
| Y | JP 2020-514328 A (THE PENN STATE RESEARCH FOUNDATION) 21 May 2020 (2020-05-21) claims, paragraphs [0106]-[0110], examples | 25-28, 30-34 |
| P, X | WO 2024/005124 A1 (ASTELLAS PHARMA INC.) 04 January 2024 (2024-01-04) claims, compound 2, examples | 1-7, 14, 16-34 |

Form PCT/ISA/210 (second sheet) (July 2022)

**INTERNATIONAL SEARCH REPORT**

International application No.
**PCT/JP2024/002768**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
**Information on patent family members**

International application No.
**PCT/JP2024/002768**

| Patent document cited in search report | Publication date (day/month/year) | Patent family member(s) | Publication date (day/month/year) |
|---|---|---|---|
| WO 2022/173531 A1 | 18 August 2022 | WO 2022/150485 A1 | |
| | | EP 4291201 A1 | |
| | | EP 4274591 A1 | |
| | | AU 2022218642 A | |
| | | CA 3207753 A | |
| | | KR 10-2023-0155448 A | |
| | | IL 305064 A | |
| | | BR 112023015937 A | |
| | | TW 202246213 A | |
| | | CA 3204244 A | |
| | | AU 2022206676 A | |
| | | KR 10-2023-0143141 A | |
| | | CN 116916943 A | |
| JP 2015-523990 A | 20 August 2015 | US 2013/0330401 A1 claims, definition of X in formula I, examples | |
| | | US 2016/0074514 A1 | |
| | | US 2020/0085957 A1 | |
| | | WO 2013/185116 A1 | |
| | | EP 2858974 A1 | |
| | | EP 3489220 A1 | |
| | | TW 201402146 A | |
| | | CA 2876148 A | |
| | | AU 2013270685 A | |
| | | KR 10-2015-0021566 A | |
| | | CN 104640841 A | |
| | | RU 2014153658 A | |
| | | BR 112014030714 A | |
| | | DK 2858974 T | |
| | | LT 2858974 T | |
| | | HR P20181855 T | |
| | | ES 2697680 T | |
| | | SI 2858974 T | |
| | | RS 58108 B | |
| | | PL 2858974 T | |
| | | HU E041882 T | |
| | | TR 201816986 T | |
| | | PT 2858974 T | |
| | | CY 1120929 T | |
| | | DK 3489220 T | |
| | | LT 3489220 T | |
| | | RS 62288 B | |
| | | PT 3489220 T | |
| | | PL 3489220 T | |
| | | SI 3489220 T | |
| | | HR P20211424 T | |
| | | ES 2897216 T | |
| | | HU E056014 T | |
| | | CY 1124499 T | |
| WO 2021/204175 A1 | 14 October 2021 | JP 2023-529522 A | |

Form PCT/ISA/210 (patent family annex) (July 2022)

**INTERNATIONAL SEARCH REPORT**
**Information on patent family members**

International application No.
**PCT/JP2024/002768**

| Patent document cited in search report | Publication date (day/month/year) | Patent family member(s) | Publication date (day/month/year) |
|---|---|---|---|
| | | US 2022/0153686 A1 | |
| | | US 2023/0286902 A1 | |
| | | EP 4077272 A1 | |
| | | CN 114206827 A | |
| | | AU 2021254312 A | |
| | | CA 3178455 A | |
| | | BR 112022020203 A | |
| | | IL 297084 A | |
| | | KR 10-2022-0166802 A | |
| | | CN 117003658 A | |
| | | MX 2022012630 A | |
| | | TW 202204309 A | |
| | | AR 121797 A | |
| | | ZA 202210782 B | |
| WO 2020/246581 A1 | 10 December 2020 | US 2022/0096381 A1<br>claims, examples 137, 138, 409, 410, etc. | |
| | | EP 3981435 A1 | |
| | | CN 113924128 A | |
| | | KR 10-2022-0007121 A | |
| | | CA 3143865 A | |
| WO 2022/081750 A1 | 21 April 2022 | JP 2023-546908 A | |
| | | JP 2023-546175 A | |
| | | US 2022/0218622 A1 | |
| | | US 2022/0235377 A1 | |
| | | WO 2022/081752 A1 | |
| | | EP 4228658 A1 | |
| | | EP 4229208 A1 | |
| | | CA 3195093 A | |
| | | AU 2021360494 A | |
| | | IL 301890 A | |
| | | KR 10-2023-0118715 A | |
| | | CN 116917266 A | |
| | | TW 202229228 A | |
| | | TW 202228725 A | |
| | | AR 123782 A | |
| | | AR 123781 A | |
| | | CA 3195123 A | |
| | | AU 2021362206 A | |
| | | BR 112023006710 A | |
| | | CN 116710074 A | |
| WO 2022/168884 A1 | 11 August 2022 | EP 4289814 A1<br>claims, compounds I-1 to I-4, I-70, etc., preparation examples, test examples | |
| WO 2022/037652 A1 | 24 February 2022 | JP 2023-537887 A | |
| | | US 2023/0053437 A1 | |
| | | EP 4168391 A1 | |
| | | CN 114391008 A | |
| | | AU 2021328980 A | |
| | | KR 10-2023-0054672 A | |

Form PCT/ISA/210 (patent family annex) (July 2022)

**INTERNATIONAL SEARCH REPORT**
**Information on patent family members**

International application No.
**PCT/JP2024/002768**

| Patent document cited in search report | Publication date (day/month/year) | Patent family member(s) | Publication date (day/month/year) |
|---|---|---|---|
| | | TW 202214566 A | |
| JP 2022-187826 A | 20 December 2022 | (Family: none) | |
| WO 2021/108609 A1 | 03 June 2021 | JP 2023-502782 A<br>EP 4065227 A1<br>AU 2020391479 A<br>CA 3162891 A<br>CN 115135382 A | |
| JP 2020-514328 A | 21 May 2020 | WO 2018/160712 A1<br>claims, paragraphs [00193]-[00197], examples<br>JP 2024-1178 A<br>US 2020/0054711 A1<br>EP 3589307 A1<br>EP 4039812 A1<br>CN 110366423 A | |
| WO 2024/005124 A1 | 04 January 2024 | (Family: none) | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2022150485 A
- WO 2022136641 A
- WO 2020246581 A
- JP 2023013548 A
- JP 2023107539 A
- WO 2014015261 A


### Non-patent literature cited in the description


- *Frontiers in Cellular Neuroscience*, 2022, vol. 16, 850866
- *Toxicologic Pathology*, 2011, vol. 39 (1), 115-123
- *Neuron*, 2014, vol. 81, 229-248
- *Nature Reviews Molecular Cell Biology*, 2014, vol. 15 (5), 313-326
- *Nucleic Acids Res.*, 2015, vol. 43, W580-W584
- **P. G. M. WUTS** ; **T. W. GREENE**. Greene's Protective Groups in Organic Synthesis. 2006
- **GREENE** ; **WUTS**. Protective Groups in Organic Synthesis. John Wiley & Sons Inc, 1999
- **S. R. SANDLER** ; **W. KARO**. Organic Functional Group Preparations. Academic Press Inc., 1991, vol. 1
- Jikken Kagaku Koza (Courses in Experimental Chemistry). Maruzen, 2005, vol. 14
- **LYNETTE C. FOO.** Purification of Rat and Mouse Astrocytes by Immunopanning. *Cold Spring Harbor Protocols*, 2013, vol. 5, 421-432
- **LYNETTE C.FOO.** Purification of Rat and Mouse Astrocytes by Immunopanning. *Cold Spring Harbor Protocols*, 2013, vol. 5, 421-432